# EUROPEAN PATENT SPECIFICATION

(11) **EP 2 603 607 B1**
(45) Date of publication and mention of the grant of the patent: **06.04.2016**
(21) Application number: 11750010.8
(22) Date of filing: 11.08.2011
(51) Int. Cl.: C12Q 1/68

(54) **GENOTYPING DNA**
DNS GENOTYPISIERUNG
GENOTYPAGE D'ADN

(30) Priority: 11.08.2010 US 372849 P; 12.05.2011 US 201161485376 P
(43) Date of publication of application: 19.06.2013
(73) Proprietor: Celula, Inc., San Diego CA 92121 (US)
(72) Inventor: KRUMMEL, Kurt, A., San Diego, CA 92130 (US); ZHANG, Haichuan, San Diego, CA 92126 (US); KATZ, Andrew, S., La Jolla, CA 92037 (US); BALDOCCHI, Russell, Encinitas, CA 92024 (US); GHATTI, Rama, San Diego, CA 92131 (US); ZHANG, Yi, San Diego, CA 92122 (US); GUNNING, Kerry, Coralville, IA 52241 (US); TU, Eugene, San Diego, CA 92103 (US)
(74) Representative: O'Neill, Michelle
(86) International application number: PCT/US2011/047485
(87) International publication number: WO 2012/021749

(56) References cited:
- WO-A1-2005/118847
- WO-A1-2010/075459
- US-A1- 2007 141 610
- US-A1- 2007 264 642
- US-A1- 2007 292 681
- WIEDMANN M ET AL: "Ligase chain reaction (LCR)--overview and applications", PCR METHODS & APPLICATIONS, COLD SPRING HARBOR LABORATORY PRESS, US, vol. 3, no. 4, 1 February 1994 (1994-02-01), pages S51-S64, XP002226705, ISSN: 1054-9803

## Description

### TECHNICAL FIELD

Provided herein are compositions and methods for genotyping nucleic acids.

### BACKGROUND OF THE INVENTION

Numerous methods for genotyping nucleic acids are known in the art. Schon, U.S. Patent No. 5,866,337; Landegren, U.S. Patent No. 6,235,472; and Willis, U.S. Patent No. 6,858,412 provide probes whose terminal domains hybridize on a target sequence, resulting in open circle probes bound to the target. Under certain conditions, ligation occurs in a double stranded portion of the hybridization complex formed by the probe and the target, resulting in concatenation of the probe and the target. Willis additionally incorporates a cleavage site into the pre-circle probes disclosed therein. Closure of the pre-circle probe is followed by cleavage and amplification to generate amplicons comprising an address site. Amplification in Willis also includes amplification of sequences identical or complementary to the original target.

Shen, U.S. Patent Application Publication No. 2008/0242555 and Oliphant, U.S. Patent Application Publication No. 2010/0015626 describe multiplexing methods for amplifying and/or genotyping a variety of samples simultaneously. In these references, primers are used to generate amplification products that include sequences of the original target, which include genomic DNA.

WO 2005/118847 relates to a method for the detection of target nucleic acid sequences based on ligation of a first and a second probe that are hybridized adjacent on a target nucleic acid sequence, removal of any unligated probes, preferably by exonuclease treatment, amplification of a portion of the second probe that does not contain the target specific section to provide amplicons and detection of said amplicons. US 2007/264642 relates to methods of multiplexing nucleic acid reactions, including amplification, detection and genotyping and relies on the use of precircle probes that are circularized in the presence of the corresponding target nucleic acids, cleaved, and then amplified. US 2007/292681 relates to an organic electroluminescence device which includes: an anode; a cathode; and an organic compound layer; the organic compound layer including at least one layer including a charge-transporting polyester; the charge-transporting polyester including repeating units each containing a structure represented by the following formula (I-1) or (I-2); the thickness being about 20 to 100 nm of the nearest layer to the anode of the at least one layer including the charge-transporting polyester; the cathode including a first layer and a second layer; the first layer being in contact with the organic compound layer and including an alkaline metal oxide, alkaline earth metal oxide, alkaline metal halide or alkaline earth metal halide; the second layer being in contact with the first layer and including an alkaline metal or alkaline earth metal.

### SUMMARY OF THE INVENTION

The methods described herein are useful for the detection of nucleic acid variations. In certain embodiments, portions of a target sequence or its complement are not amplified.

In one aspect, the invention provides a method of identifying a detection nucleotide in a primary target sequence, said primary target sequence comprising a first target domain, a second target domain and said detection nucleotide, said method comprising: (a) providing a first ligation probe comprising: in order, (i) a first address sequence, (ii) a first priming domain, (iii) a first hybridization domain that is complementary to said first target domain and (iv) a first interrogation nucleotide; (b) providing a second ligation probe comprising: in order, (i) a second hybridization domain that is complementary to said second target domain and (ii) a second priming domain, wherein one of said first and second ligation probes comprises a cleavage site; (c) hybridizing said first and second ligation probes to said first and second target domains, respectively, to form a first hybridization complex; (d) subjecting said first hybridization complex to conditions whereby if said interrogation nucleotide is perfectly complementary to said detection nucleotide, ligation occurs to form a first ligated probe; (e) forming a first circularized probe from said first ligated probe; (f) cleaving said first circularized probe at said cleavage site to form a first secondary target sequence comprising, in order, said second priming domain, said first address sequence and said first priming domain; (g) amplifying said first address sequence using said priming domains on said first secondary target sequence to form a first tertiary target sequence; and (h) detecting the presence of said first tertiary target sequence to identify said detection nucleotide, wherein said first and said second ligation probes are different molecules.

In some embodiments, said circularized probe is formed by circularizing said ligated probe.

In some embodiments, the method further comprising (a) providing a third ligation probe comprising: in order, (i) a second address sequence, (ii) said first priming domain, (iii) said first hybridization domain that is complementary to said first target domain and (iv) a second interrogation nucleotide, wherein said second address sequence is different from said first address sequence, said second interrogation nucleotide is different from said first interrogation nucleotide and wherein one of the said second or third ligation probe comprises a cleavage site; (b) hybridizing said second and third ligation probes to said first and second target domains, respectively, to form a second hybridization complex; (c) subjecting said second hybridization complex to conditions whereby if said interrogation nucleotide is perfectly complementary to said detection nucleotide, ligation occurs to form a second ligated probe; (d) forming a second circularized probe from said second ligated probe; (e) cleaving said second circularized probe at said cleavage site to form a second secondary target sequence comprising, in order, said second priming domain, said second address sequence and said first priming domain; (f) amplifying said second address sequence using said priming domains on said second secondary target sequence to form a second tertiary target sequence; and (g) detecting the presence of said second tertiary target sequence to identify said detection nucleotide, wherein said first, second and third ligation probes are different molecules.

In some embodiments, said circularized probe is formed when said ligation occurs.

In some embodiments, said first address sequence has a size different from that of said second address sequence.

In some embodiments, said first address sequence has a nucleotide sequence different from that of said second address sequence.

In some embodiments, the method further comprises contacting said hybridization complex with at least one dNTP and a polymerase prior to said ligation, wherein the 3' terminus of said first ligation probe and the 5' terminus of said second ligation probe are separated by at least one nucleotide in said hybridization complex.

In some embodiments, the 3' terminus of said first ligation probe and the 5' terminus of said second ligation probe are adjacent in said hybridization complex.

In some embodiments, the method further comprises contacting said hybridization complex with a proofreading polymerase and at least one dNTP, wherein said first ligation probe comprises a 3' extension block.

In one aspect the invention provides a method of identifying a detection nucleotide in a primary target sequence, said primary target sequence comprising a first target domain, a second target domain and said detection nucleotide, said method comprising: (a) providing a first ligation probe comprising: in order, (i) an address sequence, (ii) a first priming domain and (iii) a first hybridization domain that is complementary to said first target domain; (b) providing a second ligation probe comprising: in order, (i) a second hybridization domain that is complementary to said second target domain and (ii) a second priming domain, wherein one of said first and second ligation probes comprises a cleavage site; (c) hybridizing, in a first vessel, said first and second ligation probes to said first and second target domains, respectively, to form a hybridization complex; (d) contacting said hybridization complex with a first dNTP and a polymerase such that said first or second ligation probe is extended to comprise an interrogation nucleotide if said first dNTP is perfectly complementary to said detection nucleotide; (e) subjecting said hybridization complex to conditions whereby ligation occurs to form a ligated probe; (f) forming a circularized probe from said ligated probe; (g) cleaving said circularized probe at said cleavage site to form a secondary target sequence comprising, in order, said second priming domain, said first address sequence and said first priming domain; (h) amplifying said first address sequence using said priming domains on said secondary target sequence to form a tertiary target sequence; and (i) detecting the presence of said tertiary target sequence to identify said detection nucleotide, wherein said first and second ligation probes are different molecules.

In some embodiments, said circularized probe is formed by circularizing said ligated probe.

In some embodiments, the method further comprises repeating the method, wherein the second hybridizing step occurs in a second vessel, and said contacting step comprises contacting said hybridization complex with a second dNTP and a polymerase such that said first or second ligation probe is extended to comprise an interrogation nucleotide if said second dNTP is perfectly complementary to said detection nucleotide, wherein said second dNTP is different from said first dNTP.

In some embodiments, said first and second target domains are separated only by said detection nucleotide.

In some embodiments, said cleavage site comprises one or more moieties selected from the group consisting of inosine, a ribonucleotide, an abasic site, a photocleavable group, and a restriction enzyme cleavage sequence.

In some embodiments, said amplifying step comprises performing PCR.

In some embodiments, said tertiary target sequence comprises a label.

In some embodiments, said detecting step comprises forming a signaling complex comprising said tertiary target sequence, a capture probe and a solid support.

In some embodiments, said signaling complex further comprises a signal probe.

In some embodiments, said second priming domain comprises a priming domain extension.

In some embodiments, at least one of said address sequences comprises an adapter sequence.

In some embodiments, at least one of said address sequences comprises a sample index sequence.

In some embodiments, at least one of said address sequences comprises a locus index sequence.

In some embodiments, said locus index sequence comprises a sequence corresponding to a fragment of said primary target sequence.

In some embodiments, said locus index sequence does not comprise a sequence corresponding to a fragment of said primary target sequence.

In some embodiments, a priming domain or an address sequence does not comprise a sequence corresponding to a fragment of said primary target sequence.

In some embodiments, the step of amplifying said template address sequence is performed under conditions such that no hybridization domain is amplified.

In some embodiments, said primary target sequence is DNA of a fetus.

In some embodiments, said DNA is inherited by said fetus from the father of said fetus.

In some embodiments, said detecting step comprises sequencing said tertiary target sequence.

In some embodiments, said primary target sequence is obtained from maternal blood, maternal serum, maternal plasma, or maternal urine.

In some embodiments, said primary target sequence is DNA of a fetus and the number of primary target sequences is counted in said maternal blood, maternal serum or maternal plasma in order to detect trisomy in said fetus.

In one aspect, the invention provides a method of identifying a fetal cell in a blood sample from a mother, said method comprising: (a) determining the genotype of a plurality of genes in DNA from a mother to identify a plurality of query genes that are homozygotic in said DNA from said mother; (b) determining the genotype of one or more of said plurality of query genes in the DNA of a cell in said blood sample from said mother to identify at least one heterozygotic gene, the genotyping step comprising performing a method of the invention, thereby identifying said fetal cell.

In some embodiments, the method further comprises enriching said blood sample with fetal cells.

In some embodiments, the method further comprises amplifying DNA from said fetal cell to obtain amplified DNA and using said amplified DNA in a comparative genomic hybridization assay to detect a genetic abnormality.

In some embodiments, said genetic abnormality is trisomy.

A circularized probe produced by a method of the invention is disclosed herein.

A hybridization complex produced by a method of the invention is disclosed herein.

A secondary target sequence produced by a method of the invention is disclosed herein.

### BRIEF DESCRIPTION OF THE DRAWINGS

Figure 1 shows an embodiment of the compositions and methods of the present invention.
Figure 2 shows an embodiment of the compositions and methods of the present invention.
Figure 3 shows an embodiment of the compositions and methods of the present invention.
Figure 4 shows an embodiment of the compositions and methods of the present invention.
Figure 5 shows an embodiment of the compositions and methods of the present invention.
Figure 6 shows an embodiment of the compositions and methods of the present invention.
Figure 7 shows an embodiment of the compositions and methods of the present invention.
Figure 8 shows an embodiment of the compositions and methods of the present invention.
Figure 9 shows an embodiment of the compositions and methods of the present invention.
Figure 10 shows an embodiment of the compositions and methods of the present invention.
Figure 11 shows an embodiment of the compositions and methods of the present invention.
Figure 12 shows an embodiment of the compositions and methods of the present invention.
Figure 13 shows an embodiment of the compositions and methods of the present invention.
Figure 14 shows an embodiment of the compositions and methods of the present invention.
Figure 15 shows an embodiment of the compositions and methods of the present invention.
Figure 16 shows an embodiment of the compositions and methods of the present invention.
Figures 17A, 17B, and 17C show exemplary nucleotide sequences that can be used to construct the probes and primers for detecting a number of different SNPs.
Figure 18 shows an exemplary linear oligonucleotide that was designed to mimic a ligated probe of the present invention.
Figure 19 sets forth data showing circularization, relinearization, and amplification of products formed by carrying out the methods of the present invention.
Figures 20A and 20B set forth data showing PCR amplification and detection of an exemplary ligated probe and secondary target sequence of the present invention.
Figures 21A and 21B set forth data showing PCR amplification and detection of an exemplary ligated probe and secondary target sequence of the present invention.
Figure 22 sets forth data showing PCR amplification and detection of an exemplary ligated probe and secondary target sequence of the present invention.

### DETAILED DESCRIPTION OF THE INVENTION

The invention provides methods for detecting (or, in some cases, discovering) single nucleotide polymorphisms (SNPs) in a primary target sequence. In exemplary embodiments, the primary target sequence is not amplified. Instead, the methods utilize amplification of secondary target sequences that differ from the primary target sequence in order to genotype the primary target sequence for the presence of particular SNPs. Accordingly, the invention provides, in exemplary embodiments, for the detection of nucleic acid variations in a primary target without amplification of the primary target itself, generally by aligning the amplification primers to prevent primary target amplification. The secondary target sequence is generally an "address sequence", which identifies the nucleotide at the SNP detection position; that is, a different secondary target sequence is used for each nucleotide at the SNP detection position.

The methods of the invention can be performed in a variety of ways. As generally described herein, the base at the SNP position is called a "detection position" and the base of the ligation probe that hybridizes to the detection position is called the "interrogation base" at the "interrogation position". In one aspect, a plurality of different first ligation probes, each comprising a different address sequence and an interrogation nucleotide corresponding to a different allele of a SNP, is used, thereby allowing the practitioner to distinguish between possible gene variations at a detection nucleotide in the primary target sequence. The different first ligation probes along with second ligation probes are allowed to hybridize (either adjacently or with a gap, as further described below) to the primary target sequence in the same reaction vessel. Only the first ligation probe that contains an interrogation nucleotide that is perfectly complementary to a SNP at the detection nucleotide will be ligated to the second ligation probe. The ligated probe is circularized and cleaved, resulting in a secondary target sequence comprising an address sequence for further amplification and detection. The secondary target sequence is flanked by primer sequences for a polymerase chain reaction (PCR), thus resulting in the amplification of only the secondary target sequence to form amplicons.

Disclosed herein for reference, the first and second ligation probes may actually be distinct ends of a single probe, frequently referred to as "pre-circle" or "open circle" probes. An additional circularization step after ligation is therefore not necessary, but any unligated probe generally is removed for example, by degradation using an exonuclease before performing any amplification step.

In one aspect, the above methods are performed with an additional step in which the first and second ligation probes hybridize along a target sequence with one or more nucleotides between them, e.g. the ligation probes form a "gap" between them when hybridized. In this embodiment, one of the ligation probes is extended by one or more dNTPs using a polymerase before ligation. This results in added specificity, as a polymerase will not generally add a base to a mismatched terminus; that is, if the interrogation base is incorrect, the polymerase will not extend the gap and the ligase will be unable to ligate the two probes (or the end of an open circle probe) together. Thus requiring that a polymerase successfully extend a ligation probe before ligation can result in more accurate detection of the allele of a SNP.

In one aspect, detecting a SNP in a primary target sequence comprises using ligation probes that do not comprise an interrogation nucleotide corresponding to any particular SNP and hybridize to the complementary target sequence on either side of the detection (SNP) position, with a single nucleotide gap. Here, first and second ligation probes are allowed to hybridize with primary target sequences in at least two different reaction vessels, to each of which are added different dNTPs. In the vessel where an added dNTP is perfectly complementary to a SNP at a detection nucleotide, one of the ligation probes will be extended by a polymerase. The ligation probes are then ligated, circularized and cleaved, resulting in a secondary target sequence comprising an address sequence for further amplification and detection. As described herein, this can also be done with open circle probes, where the "gap" between the ends of the open circle probe is the detection position.

In one aspect, detecting a SNP in a primary target sequence comprises using (i) a first ligation probe comprising (a) an address cassette comprising, in order, a first priming domain, an address sequence and a second priming domain, and (b) a first target specific sequence that is complementary to a first target domain of the target sequence (sometimes referred to herein as a "first hybridization domain") and contains an interrogation base at the interrogation position, and (ii) a second ligation probe comprising (a) a second target specific sequence that is complementary to a second target domain of the target sequence (sometimes referred to herein as a "second hybridization domain") and (b) a binding label that is part of a binding pair. As is further described below, what is important is that the address and interrogation position are on one probe and the binding label is on the other, such that only if the interrogation position matches the detection position will ligation occur and the binding label is then associated with the address.

In one aspect, the above methods are performed with an additional step in which a ligation probe is extended by one or more dNTPs using a polymerase before ligation. The added specificity in requiring that a polymerase successfully extend a ligation probe before ligation will result in more accurate detection of a SNP.

Secondary target sequences can be amplified and detected using a variety of methods known in the art or further disclosed herein, including, but not limited to, sequencing and traditional array detection methods as described below.

The SNP detection methods described herein find general use in a variety of applications in which the identity of various nucleotides in a nucleic acid target is to be determined. These methods, however, are especially useful in contexts where primary target sequence amplification is undesirable. One application is the sorting of cells in a blood sample from a pregnant woman. Using the methods disclosed herein, both fetal DNA and fetal cells, which are normally present in very low numbers in maternal blood, can be detected for the general purpose of fetal diagnostic testing. Generally, a number of genes in a DNA sample known to be maternal are genotyped to identify a set of SNPs within a set of genes for which the mother is homozygous. This set is then genotyped in nucleic acids from cell-free nucleic acid or cells obtained from a maternal blood sample until at least one of the set is identified as heterozygous. Cells, or cell-free nucleic acids, bearing DNA in which a heterozygous genotype is found for a gene that is normally homozygous in the mother can thus be identified as fetal, since the heterozygosity must be inherited from the father, e.g. is paternal in origin. As is generally described in International Publication No. WO 2010/075459, identifying fetal cells in the maternal bloodstream allows minimally invasive fetal diagnostic testing. In general, the methods of the invention are used to identify non-maternal alleles, e.g. paternal alleles, by choosing SNPs for which the mother is homozygotic; thus, the appearance of the "other" allele identifies a cell that contains paternal DNA and thus is a fetal cell.

DNA from a population of fetal cells can then be pooled for a wide variety of further analyses. In one application, the pooled DNA is amplified, labeled and analyzed in a comparative genomic hybridization assay. This test can be used to detect chromosome copy number variations, e.g., trisomy or other genetic diseases. Similarly, cells that can now be identified as fetal can be cloned, and any number of genetic tests, including whole genome sequencing, additional SNP analysis, etc., can be done. Generally, any assay for obtaining fetal genetic information can be applied to the DNA collected through the present methods.

### Target sequence

Provided herein are compositions and methods for determining the identity of one or more nucleotides in a primary target sequence. A "primary target sequence" or a "primary target nucleic acid" refers to an oligonucleotide comprising one or more nucleotides whose identity is to be determined. In some instances, the presence or absence of one or more nucleotides is determined. A primary target sequence may be directly detected or, in specific embodiments, may be indirectly detected by way of a secondary target sequence, a tertiary target sequence and so on. These "higher-order" or "derivative" target sequences are different from the primary target sequence but nevertheless may be detected as a means to detect the primary target sequence. In some embodiments, a derivative target sequence is a product of a reaction such as an extended probe from a PCR reaction, a PCR amplification product ("amplicon") etc.

A target sequence may take many forms. For example, it may be contained within a larger nucleic acid sequence, i.e. all or part of a gene or mRNA, a restriction fragment of a plasmid or genomic DNA, among others. Thus, the target sequence may be a portion of a gene, a regulatory sequence, genomic DNA, cDNA, RNA including mRNA and rRNA, or others, including any complement thereof. Where reference is made to a "target sequence", it should be understood that the complement of target sequence is equally contemplated. Accordingly, in some embodiments, detecting a target sequence refers to detecting the complement of the target sequence. In exemplary embodiments, the target sequence (e.g. primary target sequence) refers to genomic DNA, for example, from a mammal, particularly from a human.

The primary target sequences are obtained from samples. In the case of fetal diagnostics, samples obtained non-invasively are preferred, such as maternal blood samples. The blood sample can be whole blood, blood cells (preferably white blood cells), serum or plasma. When fully maternal samples are desired (e.g. to do the original homozygosity tests), other samples can be used, such as buccal swabs, saliva, urine, etc. In some embodiments, the sample used in the methods of the present invention comprises an admixture of maternal and fetal material (i.e., cell-free nucleic acids and/or cells). In other embodiments, the sample used in the methods of the present invention is substantially purified for fetal or maternal material (i.e., cell-free nucleic acids and/or cells).

As is outlined more fully below, probes are made to hybridize to target sequences to determine the presence or absence of the target sequence (e.g., one allele vs. another) in a sample. The target sequence may comprise one or more target domains, which are smaller subsequences of the target sequence. A target domain generally serves as a template for constructing complementary sequences that hybridize to the target domain. For example, as outlined below, a first target domain of a target sequence may hybridize to a first hybridization domain of a first ligation probe and a second target domain of a target sequence may hybridize to a second hybridization of a second ligation probe. Ordinal terms such as "first" and "second" are not meant to confer an orientation of the domains with respect to the 5' and 3' ends of the target sequence. For example, a first target domain may be located either 5' to the second domain or 3' to the second domain.

Target domains may be adjacent (i.e. contiguous) or separated. In particular embodiments, a primary target sequence comprises a first and a second target domain. In the case where the target domains are adjacent, one or the other of the target domains contains the detection (SNP) position. In general, the detection position is located at the "end" (e.g. 5' or 3' terminus) of the target domain (referred to herein as the "zero position"), although in some embodiments the detection position is located within 1, 2, 3,4, 5, or 6 nucleotides of the terminus of the domain. What is important is that the detection position be close enough to the ligation junction to allow for discrimination between mismatches and perfect matches; that is, ligases generally will not ligate mismatches that are contained within 0-6 bases of the ligation junction, with bases at the ligation junction (e.g. position zero) providing the highest specificity for ligase activity.

In an alternative embodiment, the target domains are separated by one or more nucleotides, e.g. there is a "gap" between the ligation probes when they hybridize to the target. In this embodiment, the detection position is still within 0-6 nucleotides of one of the probe termini, but there are additional nucleotides between the two probes. Alternatively, the "gap" is the detection position, as described herein. Thus, in some embodiments, a first target domain and a second target domain are separated only by a detection nucleotide.

A detection nucleotide of a target sequence is a nucleotide whose presence or identity is to be ascertained. The detection nucleotide usually is one of a plurality of polymorphisms for a gene. In exemplary embodiments, the identity of one detection nucleotide is detected. In some embodiments, the identity of a plurality of detection nucleotides, either contiguous or separated by one or more nucleotides, is detected. Any number of polymorphisms or SNPs of any number of nucleic acid sequences can be detected using the methods and compositions herein.

A detection nucleotide can be identified by means of a "readout" or "interrogation nucleotide". An interrogation nucleotide is the part of an oligonucleotide, such as a probe, that is positioned to form a complementary base pair with the detection nucleotide. The probe in many instances contains a portion that hybridizes to the target sequence close to the detection nucleotide. In some embodiments, an interrogation nucleotide forms a complementary base pair with a detection nucleotide, and in some embodiments, an interrogation nucleotide does not form a complementary base pair with a detection nucleotide. A ligation probe, such a first ligation probe, can comprise an interrogation nucleotide, and in some cases can be extended to comprise an interrogation nucleotide.

The term "nucleic acid", "oligonucleotide" or "polynucleotide" herein means at least two nucleotides covalently linked together. A nucleic acid of the present invention will generally contain one or more phosphodiester bonds, although in some cases, as outlined below (for example in the construction of primers and probes such as label probes), nucleic acid analogs are included that may have alternate backbones, comprising, for example, phosphoramide (Beaucage et al., Tetrahedron 49(10): 1925 (1993) and references therein; Letsinger, J. Org. Chem. 35: 3800 (1970); Sprinzl et al., Eur. J. Biochem. 81: 579 (1977); Letsinger et al., Nucl. Acids Res. 14: 3487 (1986); Sawai et al, Chem. Lett. 805 (1984), Letsinger et al., J. Am. Chem. Soc. 110:4470 (1988); and Pauwels et al., Chemica Scripta 26:141 91986)), phosphorothioate (Mag et al., Nucleic Acids Res. 19:1437 (1991); and U.S. Pat. No. 5,644,048), phosphorodithioate (Briu et al., J. Am. Chem. Soc. 111:2321 (1989), O-methylphosphoroamidite linkages (see Eckstein, Oligonucleotides and Analogues: A Practical Approach, Oxford University Press), and peptide nucleic acid (also referred to herein as "PNA") backbones and linkages (see Egholm, J. Am. Chem. Soc. 114:1895 (1992); Meier et al., Chem. Int. Ed. Engl. 31:1008 (1992); Nielsen, Nature, 365:566 (1993); Carlsson et al., Nature 380:207 (1996)). Other analog nucleic acids include those with bicyclic structures including locked nucleic acids (also referred to herein as "LNA"), Koshkin et al., J. Am. Chem. Soc. 120:13252 3 (1998); positive backbones (Denpcy et al., Proc. Natl. Acad. Sci. USA 92:6097 (1995); non-ionic backbones (U.S. Patent. Nos. 5,386,023, 5,637,684, 5,602,240, 5,216,141 and 4,469,863; Kiedrowshi et al., Angew. Chem. Intl. Ed. English 30:423 (1991); Letsinger et al., J. Am. Chem. Soc. 110:4470 (1988); Letsinger et al., Nucleoside & Nucleotide 13:1597 (1994); Chapters 2 and 3, ASC Symposium Series 580, "Carbohydrate Modifications in Antisense Research", Ed. Y. S. Sanghui and P. Dan Cook; Mesmaeker et al., Bioorganic & Medicinal Chem. Lett. 4:395 (1994); Jeffs et al., J. Biomolecular NMR 34:17 (1994); Tetrahedron Lett. 37:743 (1996)) and non-ribose backbones, including those described in U.S. Pat. Nos. 5,235,033 and 5,034,506, and Chapters 6 and 7, ASC Symposium Series 580, "Carbohydrate Modifications in Antisense Research", Ed. Y. S. Sanghui and P. Dan Cook. Nucleic acids containing one or more carbocyclic sugars are also included within the definition of nucleic acids (see Jenkins et al., Chem. Soc. Rev. (1995) pp 169-176). Backbone sugar moieties other than ribose include mannose, arabinose, glucopyranose, galactopyranose, 4'-thioribose and the like. Several nucleic acid analogs are described in Rawls, C & E News Jun. 2, 1997 page 35. "Locked nucleic acids" (LNA™) are also included within the definition of nucleic acid analogs. LNAs are a class of nucleic acid analogues in which the ribose ring is "locked" by a methylene bridge connecting the 2'-O atom with the 4'-C atom. These modifications of the ribose-phosphate backbone may be done to increase the stability and half-life of such molecules in physiological environments. For example, PNA:DNA and LNA-DNA hybrids can exhibit higher stability and thus may be used in some embodiments. See also U.S. Patent Application Publication No. 2010/0105052.

In some embodiments, terminal blocking moieties, or "extension blocks", are used to prevent one or more of the enzymes in the reaction(s) to alter a probe. For example, 3' extension blocks can be used on one of the probes to avoid extension or ligation. In some embodiments, a nucleic acid comprises a modification at the 2' carbon of the ribofuranosyl ring (see U.S. Patent Application Publication No. 2009/0198047) or any other sugar modification that increases the stability of the molecule, in particular by conferring resistance to nucleases. An exemplary modification of the 2' carbon includes substitution with a group selected from H, halogen, -R, -OR, -SH, -SR, -NH₂, -NHR, -NR₂ and -CN, wherein R is alkyl. The term "alkyl" refers to a straight or branched chain, cyclic hydrocarbon radical or combination thereof, which may be fully saturated, mono- or polyunsaturated and can include di- and multivalent radicals. In some embodiments, R is an alkyl selected from C₁, C₂, C₃, C₄, C₅ and C₆ alkyl. In exemplary embodiments, the 2' carbon is substituted by a halogen. In exemplary embodiments, the 2' carbon is substituted by -OR, wherein R is alkyl, particularly wherein R is an alkyl selected from C₁, C₂, C₃, C₄, C₅ and C₆ alkyl. In some embodiments, a nucleic acid comprises a modification at the 3' carbon of the ribofuranosyl ring. In particular embodiments, the modification at the 3' carbon of the ribofuranosyl ring is a 3' extension block. A 3' extension block refers to a moiety at the 3' carbon that prevents addition of a nucleotide by a polymerase. These embodiments find particular use in proofreading assays described below and as known in the art. In some embodiments, the 3' extension block is - R-M wherein R is alkyl and M is a polar group. In particular embodiments, R is an alkyl selected from C₁, C₂, C₃, C₄, C₅, C₆, C₇, C₈, C₉, C₁₀ alkyl. In some embodiments, M is selected from -OR, -SH, -SR, -NH₂, -NHR, -NR₂ and -CN, wherein R is alkyl, which in some embodiments is an alkyl selected from C₁, C₂, C₃, C₄, C₅ and C₆ alkyl. In some embodiments, the 3' extension block is selected from -(CH₂)₃SH, -(CH₂)₃NH₂, -(CH₂)₃OH and inorganic phosphate. In particular embodiments, the 3' extension block is -(CH₂)₃OH. In some embodiments, the 3' extension block is selected from inorganic phosphate and -R-M, wherein R is alkyl and M is a polar group. In some embodiments, the alkyl is C₁-C₁₀ alkyl and the polar group is selected from -NH₂, -SH and -OH.

In some embodiments, a nucleotide comprises a base other than adenine, guanine, cytosine, thymine or uracil. For example, a nucleotide can be xanthine, inosine, queuosine or other base known in the art. Any type of base can be modified by the replacement or addition of one or more atoms or groups. Accordingly, a base can be alkylated, halogenated, thiolated, aminated, amidated, or acetylated in any combination. Specific examples of modified bases include, for example, 5-propynyluridine, 5-propynylcytidine, 6-methyladenine, 6-methylguanine, N,N,-dimethyladenine, 2-propyladenine, 2-propylguanine, 2-aminoadenine, 1-methylinosine, 3-methyluridine, 5-methylcytidine, 5-methyluridine and other bases having a modification at the 5 position, 5-(2-amino)propyl uridine, 5-halocytidine, 5-halouridine, 4-acetylcytidine, 1-methyladenosine, 2-methyladenosine, 3-methylcytidine, 6-methyluridine, 2-methylguanosine, 7-methylguanosine, 2,2-dimethylguanosine, 5-methylaminoethyluridine, 5-methyloxyuridine, deazanucleotides such as 7-deaza-adenosine, 6-azouridine, 6-azocytidine, 6-azothymidine, 5-methyl-2-thiouridine, other thio bases such as 2-thiouridine and 4-thiouridine and 2-thiocytidine, dihydrouridine, pseudouridine, queuosine, archaeosine, naphthyl and substituted naphthyl groups, any O- and N-alkylated purines and pyrimidines such as N6-methyladenosine, 5-methylcarbonylmethyluridine, uridine 5-oxyacetic acid, pyridine-4-one, pyridine-2-one, phenyl and modified phenyl groups such as aminophenol or 2,4,6-trimethoxy benzene, modified cytosines that act as G-clamp nucleotides, 8-substituted adenines and guanines, 5-substituted uracils and thymines, azapyrimidines, carboxyhydroxyalkyl nucleotides, carboxyalkylaminoalkyl nucleotides, and alkylcarbonylalkylated nucleotides.

The term "nucleotide" also includes what are known in the art as universal bases. By way of example, universal bases include but are not limited to 3-nitropyrrole, 5-nitroindole, or nebularine. The term "nucleotide" is also meant to include the N3' to P5' phosphoramidate, resulting from the substitution of a ribosyl 3'-oxygen with an amine group.

### Ligation probes

The methods described herein generally require the use of one or more ligation probes. A ligation probe may be either an allele specific probe or locus specific probe. An "allele specific" probe is one that either hybridizes to a target sequence (e.g. the interrogation position is contained within the probe) and discriminates between alleles. A "locus specific" probe is one that hybridizes to a target sequence at a particular locus, but does not necessarily discriminate between alleles (e.g. where the "gap" is the interrogation position).

In exemplary embodiments, a ligation probe comprises a hybridization domain complementary to a target domain of the target sequence. In general, a probe disclosed herein is designed to be complementary to a domain of a target sequence (either a primary target sequence or a derivative target sequence), such that hybridization of the target sequence and the probe occurs. In exemplary embodiments, a hybridization domain and a target domain are perfectly complementary to each other. In some embodiments, the complementarity is not perfect; there may be any number of base pair mismatches between the target sequence and the probe. If the number of mismatches is so great that no hybridization can occur under even the least stringent of hybridization conditions, the sequence is not a complementary target sequence. Thus, "substantially complementary" refers to sequences that are sufficiently complementary such that they hybridize under selected reaction conditions, for example, stringent hybridization conditions as are known in the art.

In exemplary embodiments, a ligation probe comprises a priming domain. In some embodiments, a ligation probe comprises two priming domains. A "priming domain" refers to a nucleotide sequence that is complimentary to an oligonucleotide (i.e. primer) that is used to generate a copy of some part of the molecule to which the oligonucleotide hybridizes (in general, the address sequence). For example, a primer can be hybridized to the priming domain to form a complex that is then subjected to PCR conditions or some other art-known conditions that allow a copy to be synthesized.

In some embodiments, a priming domain further comprises additional sequences, sometimes referred to herein as "extension" sequences. Priming domain extensions can be useful for adding nucleotides to serve various purposes, such as to add adapter(s), index sequence(s), additional address sequence(s), a label or any combination thereof. For example, when one priming domain comprises a first address sequence, the extension domain of the second priming domain may include additional address sequence such that upon formation of circularized probe, the address sequence is now longer, containing both the original address sequence and the extension address sequence. In general, the extension domain of a priming domain does not hybridize/is not complementary to a primer.

In some embodiments, a first priming domain of a first ligation probe comprises a first label. In some embodiments, a first priming domain of a first ligation probe comprises a first label and a first priming domain of a third ligation probe comprises a second label. In some embodiments, said first label and said second label are different.

In exemplary embodiments, where a first ligation probe and a second ligation probe are used, the first ligation probe comprises a first priming domain and the second ligation probe comprises a second priming domain, in configurations that upon circularization, flank the address sequence as is generally depicted in the figures to allow for amplification of the address sequence. In some aspects, a single ligation probe comprises a first priming domain and a second priming domain (again, flanking the address sequence upon circularization). In some embodiments, when two primers are used, their complementarity to the two respective priming domains may be different. That is, one PCR primer will directly hybridize to one priming domain, while the other PCR primer will hybridize to the complement of the other priming domain. That is, pairs of primers hybridize to opposite strands when PCR techniques are used. That is, if the probe is considered a "Crick" strand, the primers that hybridize to the priming domains will include a "Watson" primer, that hybridizes directly to the "Crick" priming domain, and a "Crick" primer, that hybridizes to the complement of the second priming domain.

In exemplary embodiments, a first and a second primer are chosen such that only the primers and an address sequence are amplified. In other words, in exemplary embodiments, a first and a second primer are chosen such that no portion of a primary target sequence is amplified. In exemplary embodiments, a primer is not the same as or complementary (perfectly, substantially or otherwise) to a primary target sequence (such as genomic DNA).

In exemplary embodiments, a ligation probe comprises an address sequence. Alternatively known in the art as a "zip code", "bar code" or "tag", an address sequence serves to distinguish the molecule in which the address sequence is found from a different molecule in which a different address sequence is found. As described herein, the "address sequences" can be distinguishable from each other on the basis of size (for example for capillary electrophoresis readouts) and/ or sequence (for example when the different amplicons are hybridized to an array containing capture probes that will hybridize to the different address sequences).

In some embodiments, an address sequence comprises one or more shorter sequences that themselves could be referred to as an address sequence. For example, a "template address sequence" could comprise one or more adapters, locus or allele index sequences and sample index sequences or any combination thereof. As discussed further below, a "locus or allele index sequence" and a "sample index sequence" serve to associate a larger sequence with a specific locus or allele or with a type of sample, respectively.

In some embodiments, an address sequence comprises an adapter. The address sequence may have additional appended nucleotides that function as an adapter. Alternatively, the address sequence may itself be an adapter. An "adapter" is a sequence that can be used to bind, covalently or noncovalently, a first molecule to a second molecule or structure.

Adapters can be useful for making a molecule compatible with or useful in various systems, such as a nucleic acid sequencing system, in which adapters can be used, for example, during a sample preparation phase. In some embodiments, an address sequence functions as a sequencing adapter. Adapter oligonucleotides can be compatible with, for example, HiSeq (Illumina), SOLiD (Applied Biosystems), 454 sequencing technologies (Roche), Ion Torrent (Life Technologies), HeliScope (Helicos Biosciences), SMRT (Pacific Biosciences), CGA Service (Complete Genomics) or any other sequencing or array technologies and services. Compatible adapter oligonucleotides are well known in the art (see, e.g., Ansorge W., (2009) New Biotechnology. 25:195-203). In some embodiments, adapters used in sequencing systems can have additional functionalities. For example, an adapter can be used as a primer or a priming domain for target amplification.

In exemplary embodiments, the difference between a first sequence comprising a first address sequence and a second sequence comprising a second address sequence is that the nucleotide at corresponding interrogation positions is different. An address sequence contained within a probe can facilitate immobilization of the probe to a universal array, which is an array (either a solid phase or liquid phase array) containing capture probes that are not specific to a primary target, but rather, specific to individual, particularly artificial sequences, e,g. the address sequences. In exemplary embodiments, an address sequence is not the same as or complementary (perfectly, substantially or otherwise) to a primary target sequence (such as genomic DNA). In exemplary embodiments, an address sequence is allele specific. That is, an allele specific address sequence serves to identify a particular allele. For example, an allele specific address sequence can be part of a probe, such as a first ligation probe, comprising a first interrogation nucleotide, and is distinguishable from another allele specific address sequence that is part of another probe, such as a different ligation probe (i.e., a third ligation probe) comprising a second interrogation nucleotide. The first and second interrogation nucleotides are different, hence making the address sequences allele specific.

In some embodiments, a first address sequence has a size different from that of a second address sequence. Changing the size of the address sequences between two probes provides an added dimension for differentiating the two probes. This can be achieved, for example, by shifting or "walking" a priming domain along a probe to obtain probes and hence amplicons whose address sequences differ by sequence length. Molecules containing differently sized address sequences can be detected in numerous ways, such as capillary or standard electrophoresis or any other method known for detecting molecules based on size. In other embodiments, ligation probes can be selected based on size to enhance, for example, circularization of ligation probes.

Size differentiation could optionally be combined with other components to provide multiple dimensions for detecting different probes and the amplicons that the probes can be used to generate. For example, a label (e.g. a fluorescent label) can be incorporated into an amplicon by labeling a primer or dNTP that is used to synthesize the amplicon as is well known in the art. Thus, in some embodiments, a primer comprises a label. Additionally, a label can be part of a separate labeling probe that hybridizes to some part of the amplicon (e.g. in "sandwich assays"). Molecules comprising both different size and optical labels can be detected in a variety of ways, such as through electrophoresis (e.g., capillary electrophoresis). The labels can be two or more labels that emit light at different frequencies. Other types of labels, such as electronically detected, radioactive or other labels described herein, can be used in addition to or instead of an optical label.

In some embodiments, an address sequence is complementary to a capture probe on a surface of an array. In general, sets of address sequences and the corresponding capture probes on arrays are developed to minimize cross-hybridization with both each other and other components of the reaction mixtures, including nucleic acid sequences outside of the target sequences (e.g. to sequences within genomic DNA). Other forms of address sequences act as mass tags that can be separated using mass spectroscopy, electrophoretic tags that can be separated based on electrophoretic mobility, etc. Some address sequences are outlined in U.S. Patent Application Publication No. 2003/0096239. In exemplary embodiments, an address sequence or its complement is not found in a genome, particularly a human genome, and lacks a hairpin loop.

In some embodiments, address sequences are used with a "universal" surface, that is, one standard array comprising a set of capture probes that can be used in any application. The end-user can customize the array by designing different soluble capture probes, which, as will be appreciated by those in the art, is generally simple and cost-effective. For example, a soluble capture probe can comprise a first domain that hybridizes to the capture probe and a second domain that hybridizes to the address sequence. It can be appreciated that multiple soluble capture probes can be used to create a chain that indirectly binds an address sequence to a capture probe. In exemplary embodiments, an array of different and usually artificial capture probes is used; that is, the capture probes do not have complementarity to a naturally occurring primary target sequence. The address sequences can then be incorporated in the capture probes.

The length of the address sequences can vary, depending on the desired strength of binding and the number of different address sequences desired. In exemplary embodiments, an address sequence range is about 6 to about 500 nucleotides in length, in more particular embodiments, from about 8 to about 100, and in more particular embodiments, from about 10 to about 25.

In exemplary embodiments, a ligation probe comprises an interrogation nucleotide. As stated previously, an interrogation nucleotide is used to distinguish the possible detection nucleotides in a primary target sequence. An interrogation nucleotide can be in any position of a ligation probe. In exemplary embodiments, one or more nucleotides flanking an interrogation nucleotide in a ligation probe is complementary to a targeting domain of a primary target sequence. In exemplary embodiments, the 3' terminal nucleotide of a ligation probe is an interrogation nucleotide (although as outlined herein the interrogation position may be slightly removed from the 3' terminus). In some embodiments, the 5' terminal nucleotide of a ligation probe is an interrogation nucleotide (although as outlined herein the interrogation position may be slightly removed from the 5' terminus). Multiple ligation probes, each comprising a different interrogation nucleotide, can be used simultaneously. In exemplary embodiments, a set of two, three or four ligation probes is used, each probe comprising an interrogation nucleotide different from each other interrogation nucleotide of each other ligation probe in the set. The number of probes is dependent on the alleles of the SNP position; while most SNPs comprise two alleles, some comprise three or four. Such a set generally comprises allele specific probes that can be used to genotype a gene. Multiple sets of ligation probes can be used to genotype multiple genes. It will be understood by one of skill in the art that the screening or genotyping methods in any of the embodiments described herein may be performed to detect at least one allele of at least one genetic locus.

In general, a unique address sequence is used for each allele specific ligation probe. An address sequence is unique if it can be differentiated from another address sequence in some way. For example, the address sequences can be differentiated by sequence, size, chemical label or the like, in any combination.

In some aspects, a ligation probe does not comprise an interrogation nucleotide. Such ligation probes can be used in free base assays described below in which the same ligation probe but different free bases are added to different reaction vessels comprising a primary target sequence. The ligation probe forms an open circle in a hybridization complex with the primary target sequence and is extended (or "filled in") in the presence of free base that is complementary to the detection nucleotide in the gap between the ends of the open circle. Thus, a ligation probe can be extended or filled in to comprise an interrogation nucleotide.

In one embodiment, all nucleotides outside of one or more interrogation nucleotides in two or more ligation probes are the same as between the ligation probes; that is, in some embodiments the probes used in the methods of the present invention have all components other than the interrogation position (e.g. both the length of the probes as well as the non-interrogation bases) identical to allow good discrimination.

In some embodiments, a ligation probe comprises a label sequence, i.e. a sequence that can be used to bind a label or signal probe. The label sequence can be substantially or perfectly complementary to a label probe. Systems utilizing such a label probe are sometimes referred to in the art as "sandwich" assays. That is, by incorporating a label sequence into a ligation probe, at least part of which is then amplified and present in the amplicons, a label probe comprising primary (or secondary) detection labels can be added to the mixture, either before addition to an array or after. This allows the use of high concentrations of label probes for efficient hybridization. In one embodiment, it is possible to use the same label sequence and label probe for all ligation probes on an array; alternatively, different ligation probes can have a different label sequence.

### Extension Blocks

In some embodiments, a ligation probe comprises a nucleic acid analog at the 3' terminus. In some embodiments, a ligation probe comprises a nucleic acid analog at a plurality of positions including the 3' terminus. A nucleic acid analog at one or more positions including the 3' terminus would be particularly useful in embodiments where resistance to nucleotide excision by an enzyme is desired. For example, in addition to being able to extend a priming sequence hybridized to a template sequence, certain nucleic acid polymerases, such as T4 DNA polymerase, are capable of "proofreading", 3'-5' exonuclease activity that results in the removal of a mismatch at the 3' end of a strand undergoing extension. In order to ensure that a ligation probe comprising a mismatch is not extended following proofreading activity that would excise the mismatch, the interrogation nucleotide at the 3' end of a ligation probe could be attached to the rest of the ligation probe via a linkage other than a phosphodiester bond. For example, a ligation probe can comprise at the 3' terminus of the probe a nucleic acid analog comprising a phosphorothioate linkage. In other words, the nucleoside at the 3' terminus is coupled to the remainder of the probe by a phosphorothioate linkage. In some embodiments, it may be necessary to incorporate more than one phosphorothioate linkages between a plurality of nucleic acids including the 3' terminus. For example, in order to increase nuclease resistance, it may be necessary to include a second phosphorothioate linkage 5' to a first phosphorothioate linkage between a 3' terminal nucleic acid and the remainder of the molecule. Thus, in some embodiments, at least one ligation probe comprises a nucleotide analog in at least the 3' terminus. In some embodiments, a nucleotide analog is selected from the group consisting of phosphorothioate and a locked nucleotide.

In some embodiments, a ligation probe comprises a 3' extension block. In particular embodiments, a ligation probe comprises an interrogation nucleotide which comprises a 3' extension block. Ligation probes comprising a 3' extension block are useful in "proofreading assays" as known in the art. See Bi and Stambrook, Nucleic Acids Research, 1998, 26(12): 3073-3075. In certain examples of these assays, a ligation probe is allowed to hybridize with a target sequence to form a hybridization complex, wherein the ligation probe comprises an interrogation nucleotide that is a mismatch with a detection nucleotide of the target sequence. The mismatch is corrected by contacting the hybridization complex with at least one dNTP and a polymerase having proofreading capabilities (i.e., a proofreading polymerase, which optionally lacks strand displacement activity), which replaces the mismatch with the correct nucleotide for Watson-Crick pairing. In some embodiments, the correct nucleotide can then be ligated to a second ligation probe. In some embodiments, the correct nucleotide can then be extended (e.g. by a polymerase in contact with at least one dNTP) and then ligated to a second ligation probe. In some embodiments, the ligation probe with the corrected nucleotide is extended and detected or extended and amplified with detection of the amplification products. Thus, any of the embodiments described herein can be adapted to utilize a proofreading assay in which a probe containing a mismatch with a detection nucleotide of a target sequence is used to detect a SNP of a gene.

### Cleavage Sites

At least one of the first and second ligation probes comprises a cleavage site. In some embodiments, a first ligation probe comprises a first cleavage site and a second ligation probe comprises a second cleavage site, and the first and second cleavage sites can be the same or different. The term "cleavage site" refers to a portion of an oligonucleotide comprising one or more covalent bonds that are broken when subjected to conditions known in the art to result in bond breakage. These conditions include high energy conditions, such as high heat or ionizing radiation (e.g., UV light); pH conditions conducive to bond breakage, such as acidic or alkali solutions; and enzymes that catalyze bond breakage, such as an endonuclease or other enzyme that hydrolyzes specific types of bonds within an oligonucleotide. Typically, only one or more bonds within the cleavage site are broken when subjected to the appropriate conditions, with the remainder of the oligonucleotide lacking other cleavage sites and thus resisting any additional bond breakage.

The cleavage site in a ligation probe is a single stranded sequence of nucleotides, that is, DNA, RNA, a nucleic acid analog or a combination of these. In some embodiments, it may be necessary to hybridize an additional sequence to the cleavage site before actual cleavage is to occur. In those embodiments, the double stranded cleavage site comprises a hybrid of two single stranded DNA, a hybrid of two single stranded RNA, a mixed hybrid of single stranded DNA and single stranded RNA or analogs thereof. Double stranded cleavage sites can have two strands of nucleic acid that are perfectly complementary or that are perfectly complementary at every position except for one or more mismatches. The number of strands at a cleavage site may differ from the number of strands found in the remainder of the oligonucleotide containing the cleavage site. For example, a cleavage site might be double stranded whereas portions outside of the cleavage site (for example, in a circularized probe as described below) are single stranded. One or more nucleotides in a cleavage site may also contain sugar moieties differing from those in the rest of the oligonucleotide. For example, a strand of DNA could comprise a cleavage site that comprises one or more RNAs. Thus, single stranded oligonucleotides herein can comprise a mixture of DNA, RNA and analogs thereof. One or more backbone sugar moieties in a cleavage site might lack a base; such moieties are sometimes referred to as being abasic, AP, apurinic or apyrimidinic sites.

In exemplary embodiments, a cleavage site comprises an inosine. "Inosine" refers to a nucleotide in which hypoxanthine is attached to ribofuranose via a β-N9-glycosidic bond. In some embodiments, inosine refers to deoxyinosine. An oligonucleotide incorporating inosine in its sequence can be cleaved close to the inosine site when contacted with a nuclease, such as an endonuclease. In exemplary embodiments, the endonuclease is endonuclease V. Endonuclease V, also known as deoxyinosine 3'-endonuclease, is an enzyme that cleaves the second phosphodiester bond 3' from an inosine in either double or single stranded DNA. Additionally, endonuclease V can also cleave DNA containing abasic sites, urea, uracil, base mismatches, insertion/deletion mismatches, hairpin or unpaired loops, flaps and pseudo-Y structures. Thus, in some embodiments, a cleavage site comprises one or more nucleotides or sites that are recognized by endonuclease V.

In some embodiments, a cleavage site comprises an abasic site. An abasic site is a portion of an oligonucleotide in which a sugar backbone lacks a base. Terms used in the art to refer to an abasic site include apurinic, apyrimidinic and AP. Cleavage of an abasic site can catalyzed by any of a number of AP-cleaving enzymes known in the art, including Escherichia coli exonuclease III (gene xthA), Streptococcus pneumoniae and Bacillus subtilis exonuclease A (gene exoA), Mammalian AP endonuclease 1 (AP1), Drosophila recombination repair protein 1 (gene Rrp1), Arabidopsis thaliana apurinic endonuclease-redox protein (gene arp), Dictyostelium DNA-(apurinic or apyrimidinic site) lyase (gene apeA), Bacterial endonuclease IV (gene nfo), Fungal and Caenorhabditis elegans apurinic endonuclase APN1, Dictyostelium endonuclease 4 homolog, Archaeal probable endonuclease 4 homologs and Mimivirus putative endonuclease 4.

Additionally, an abasic site can be cleaved through heat or chemical-treatment. For example, an oligonucleotide comprising an abasic site could be subjected to 100°C for 30 min. See Sugiyama et al., Chem. Res. Toxicol., 1994, 7: 673-683. An abasic site can also be cleaved chemically by using reagents such as morpholine, piperidine, piperazine, 1,2-ethylenediamine and N,N'-dimethylethylenediamine. Alkali treatments, such as raising sample pH using sodium hydroxide, can also be used to cleave an oligonucleotide at an abasic site.

In some embodiments, as described below, an abasic site is created in an oligonucleotide and then cleaved by treatment with an AP-cleaving enzyme, heat or chemical treatment or other method known in the art.

In some embodiments, a cleavage site comprises a ribonucleotide. Certain enzymes, in particular RNases, are known to cleave RNA. Certain RNases such as RNase A and RNase T₁ act upon single stranded RNA. Other RNases act on double stranded nucleic acids. Examples include RNase III, whose substrate is double stranded RNA, and RNase H, which cleaves RNA found in an RNA:DNA hybrid. If needed, a cleavage site comprising RNA can be cleaved by RNase III, RNase H and the like by introduction of an additional DNA or RNA strand that will form a double stranded site sufficient for recognition and cleavage by these enzymes.

In some embodiments, a cleavage site comprises uracil. In exemplary embodiments, a cleavage site comprises deoxyuracil. Various glycosylases recognize the presence of uracil within a DNA sequence. These glycosylases excise the uracil, leaving an abasic site that can be cleaved in various ways as described herein and known in the art. For example, uracil DNA glycosylase (UDG) removes uracil from single or double stranded DNA, and the resulting abasic site can be cleaved, for example, by heat treatment or by contact with an AP-cleaving enzyme, such as endonuclease VIII. Accordingly, the activities of a glycosylase and an AP-cleaving enzyme can be combined to cleave one or more strands of DNA containing uracil.

In some embodiments, a cleavage site comprises a base pair mismatch. As is known in the art, complementary base pairs form between adenine (A) and thymine (T), between adenine and uracil (U) and between guanine (G) and cytosine (C) in double stranded nucleic acids. A "base pair mismatch" or "mismatch" is a base pair that deviates from these possibilities. Repair enzymes play a role in various organisms to remove mismatches from nucleic acids. For example, MutY glycosylase recognizes a G:A mismatch, from which it removes adenine to leave an abasic site. An AP-cleaving enzyme, such as endonuclease IV, subsequently cleaves the strand containing the abasic site. Accordingly, the activities of a glycosylase and an AP-cleaving enzyme can be combined to cleave one or more strands of a hybridized nucleic acid containing a mismatch.

In some embodiments, a cleavage site comprises a restriction enzyme cleavage site. A restriction enzyme cleavage site is a nucleic acid sequence, typically though not always 4-8 base pairs in length, that is cleaved by any one of a number of restriction enzymes. Encompassing a diverse group of catalytic proteins, restriction enzymes that are useful for experimental purposes include Type II, Type III and Type IV enzymes. Certain Type II enzymes, including HhaI, HindIII and NotI, cleave nucleic acids within their recognition site, which can be continuous or discontinuous, symmetric or asymmetric. Other Type II enzymes cleave outside of their recognition site. TypeIIS endonucleases recognize asymmetric double stranded DNA sequences 4-7 bp long, and cleave both strands at specific locations up to 20 bases away from their recognition site. TypeIIS endonucleases, such as Fokl and AlwI, contain two distinct domains for DNA recognition binding and cleaving. In some cases, TypeIIS enzymes are more effective at DNA cleavage after having interacted with more than one recognition sequence. TypeIIG enzymes are another type of endonuclease that cleaves outside of a restriction enzyme recognition site. Accordingly, in some embodiments, a nucleic acid comprises a restriction enzyme recognition site apart from a cleavage site, such as a restriction enzyme cleavage site. In some embodiments, it may be necessary to hybridize an additional nucleic acid strand to the cleavage site in order to ensure proper recognition and cleavage by a restriction enzyme.

In some embodiments, a cleavage site comprises a 3'-*S*-phosphorothiolate bond. It has been shown that DNA containing a 3'-*S*-phosphorothiolate bond can be cleaved by contacting the DNA with an aqueous solution containing iodine and pyrimidine. See Vyle et al., Biochemistry, 1992, 31: 3012. Thus, it may be useful to have in some embodiments a cleavage site comprising a linkage that is an analog to the phosphodiester linkages normally found in a nucleic acid.

In other embodiments, a cleavage site comprises a photocleavable group. Photocleavable groups are known in the art. (See e.g., U.S. Patent No.. 5,919,917.) A photocleavable group is cleaved photochemically following irradiation with a light at an appropriate wavelength for a predetermined period of time, thus cutting the predetermined bond in the polynucleotide containing the photocleavable group. Accordingly, photoclevage permits a cyclic oligonucleotide to be a linear oligonucleotide. For example, a photosensitive texaphyrin or texaphyrin-conjugate having catalytic activity for photolytic cleavage of DNA is contacted with the oligonucleotide, and the texaphyrin is then exposed to light for a time sufficient to cleave the oligonucleotide. (See, e.g., U.S. Patent No. 5,607,924.)

### Probe Configurations

The components described herein can be combined in various ways. In some embodiments, a first ligation probe, but not a second ligation probe, comprises a cleavage site. In some embodiments, a second ligation probe, but not a first ligation probe comprises a cleavage site. In some embodiments, a first ligation probe comprises a first cleavage site and a second ligation probe comprises a second cleavage site, and the first and second cleavage sites can be the same or different. Use of a first and a second cleavage site is especially useful when a first target domain is 5' to a second target domain; this can be done to ensure that only a primer pair and an address sequence are amplified. Accordingly, in some embodiments, a first ligation probe comprises, in order, (i) an address sequence, (ii) a first priming domain, (iii) a first hybridization domain complementary to a first target domain and (iv) an interrogation nucleotide; and a second ligation probe comprises, in order, (i) a second hybridization domain complementary to a second target domain, (ii) a cleavage site and (iii) a second priming domain. In some embodiments, a first ligation probe comprises, in order, (i) an address sequence, (ii) a first priming domain, (iii) a cleavage site, (iv) a first hybridization domain complementary to a first target domain and (v) an interrogation nucleotide; and a second ligation probe comprises, in order, (i) a second hybridization domain complementary to a second target domain and (ii) a second priming domain. In some embodiments, a first ligation probe comprises, in order, (i) an address sequence, (ii) a first priming domain, (iii) a first cleavage site, (iv) a first hybridization domain complementary to a first target domain and (v) an interrogation nucleotide; and a second ligation probe comprises, in order, (i) a second hybridization domain complementary to a second target domain, (ii) a second cleavage site and (iii) a second priming domain. "In order" refers to either a 5' to 3' direction or a 3' to 5' direction, and does not preclude additional elements that do not change the order specified.

In some embodiments, an additional ligation probe is used, which is identical to a first ligation probe except for having a different address sequence and a different interrogation nucleotide. An additional ligation probe may or may not comprise a cleavage site. Additional ligation probes would be useful in assays in which multiple probes comprising an interrogation nucleotide are present in a sample and compete to bind to the same site. Accordingly, in exemplary embodiments, a third ligation probe comprises, in order, (i) a second address sequence, (ii) a first priming domain, (iii) a first hybridization domain complementary to a first target domain and (iv) a second interrogation nucleotide. In exemplary embodiments, a third ligation probe comprises, in order, (i) a second address sequence, (ii) a first priming domain, (iii) a cleavage site, (iv) a first hybridization domain complementary to a first target domain and (v) a second interrogation nucleotide. In exemplary embodiments, a fourth ligation probe comprises in order, (i) a third address sequence, (ii) a first priming domain, (iii) a first hybridization domain complementary to a first target domain and (iv) a third interrogation nucleotide. In exemplary embodiments, a fourth ligation probe comprises, in order, (i) a third address sequence, (ii) a first priming domain, (iii) a cleavage site, (iv) a first hybridization domain complementary to a first target domain and (v) a third interrogation nucleotide. In exemplary embodiments, a fifth ligation probe comprises in order, (i) a fourth address sequence, (ii) a first priming domain, (iii) a first hybridization domain complementary to a first target domain and (iv) a fourth interrogation nucleotide. In exemplary embodiments, a fifth ligation probe comprises, in order, (i) a fourth address sequence, (ii) a first priming domain, (iii) a cleavage site, (iv) a first hybridization domain complementary to a first target domain and (v) a fourth interrogation nucleotide. In exemplary embodiments, the interrogation nucleotide and address sequence of each of a third, fourth or fifth ligation probe differ from the interrogation nucleotide and address sequence of the others and of a first ligation probe. It will be understood by those of skill in the art that in some embodiments herein, the terms "a first priming domain" and "a first hybridization domain complementary to a first target domain" may refer, respectively, to a copy of a first priming domain and a copy of a first hybridization domain complementary to a first target domain.

In some aspects, a first ligation probe and a second ligation probe are joined between the address sequence of the first ligation probe and the second priming domain of the second ligation probe. The resulting "open circle" or "pre-circle" probe can then be hybridized to a primary target sequence. A cleavage site in an open circle probe can be between (i) a first priming domain and a first hybridization domain complementary to a first target domain, (ii) a second priming domain and a second hybridization domain complementary to a second target domain or (iii) both. Thus, in some aspects different ligation probes each containing a unique pair of address sequence and interrogation nucleotide are each joined to a second ligation probe, resulting in a plurality of open circle probes comprising a characteristic address sequence and interrogation nucleotide. Accordingly, in some aspects, a ligation probe comprises, in order, (i) an interrogation nucleotide, (ii) a first hybridization domain complementary to a first target domain, (iii) a first priming domain, (iv) a first address sequence, (v) a second priming domain, (vi) a cleavage site and (vii) a second hybridization domain complementary to a second target domain. In some aspects, a ligation probe comprises, in order, (i) an interrogation nucleotide, (ii) a first hybridization domain complementary to a first target domain, (iii) a cleavage site, (iv) a first priming domain, (v) a first address sequence, (vi) a second priming domain and (vii) a second hybridization domain complementary to a second target domain. In some aspects, a ligation probe comprises, in order, (i) an interrogation nucleotide, (ii) a first hybridization domain complementary to a first target domain, (iii) a first cleavage site, (iv) a first priming domain, (v) a first address sequence, (vi) a second priming domain, (vii) a second cleavage site and (viii) a second hybridization domain complementary to a second target domain.

In some embodiments, a ligation probe does not comprise an interrogation nucleotide. In particular, a first ligation probe can be used with a second ligation probe disclosed herein wherein neither comprises an interrogation nucleotide. These probes may be useful in free base assays described herein. Accordingly, in some embodiments, a first ligation probe comprises, in order, (i) an address sequence, (ii) a first priming domain and (iii) a first hybridization domain complementary to a first target domain; and a second ligation probe comprises, in order, (i) a second hybridization domain complementary to a second target domain, (ii) a cleavage site and (iii) a second priming domain. In some embodiments, a first ligation probe comprises, in order, (i) an address sequence, (ii) a first priming domain, (iii) a cleavage site and (iv) a first hybridization domain complementary to a first target domain; and a second ligation probe comprises, in order, (i) a second hybridization domain complementary to a second target domain and (ii) a second priming domain. In some embodiments, a first ligation probe comprises, in order, (i) an address sequence, (ii) a first priming domain, (iii) a first cleavage site and (iv) a first hybridization domain complementary to a first target domain; and a second ligation probe comprises, in order, (i) a second hybridization domain complementary to a second target domain, (ii) a second cleavage site and (iii) a second priming domain.

Similarly, in some aspects, an open circle probe does not comprise an interrogation nucleotide. Accordingly, in some aspects, a ligation probe comprises, in order, (i) a first hybridization domain complementary to a first target domain, (ii) a first priming domain, (iii) an address sequence, (iv) a second priming domain, (v) a cleavage site and (vi) a second hybridization domain complementary to a second target domain. In some aspects, a ligation probe comprises, in order, (i) a first hybridization domain complementary to a first target domain, (ii) a cleavage site, (iii) a first priming domain, (iv) an address sequence, (v) a second priming domain and (vi) a second hybridization domain complementary to a second target domain. In some aspects, a ligation probe comprises, in order, (i) a first hybridization domain complementary to a first target domain, (ii) a first cleavage site, (iii) a first priming domain, (iv) an address sequence, (v) a second priming domain, (vi) a second cleavage site and (vii) a second hybridization domain complementary to a second target domain.

In some embodiments, any ligation probe herein comprises a label. In exemplary embodiments, the label is a binding label. In some embodiments, a first ligation probe comprises, in order, (i) a first priming domain, (ii) an address sequence, (iii) a second priming domain, (iv) a first hybridization domain that is complementary to a first target domain and (v) an interrogation nucleotide; and a second ligation probe comprises (i) a second hybridization domain that is complementary to a second target domain and (ii) a binding label. In some embodiments, a first ligation probe comprises, in order, (i) a first priming domain, (ii) an address sequence, (iii) a second priming domain and (iv) a first hybridization domain that is complementary to a first target domain; and a second ligation probe comprises (i) a second hybridization domain that is complementary to a second target domain and (ii) a binding label. The term "address cassette" may be used herein to refer to, in order, a first priming domain, an address sequence and a second priming domain. Additional components can be part of the address cassette and can be added between the first and second priming domain.

### Hybridization

A ligation probe described herein is designed to hybridize to a target sequence to form a hybridization complex. A "hybridization complex" comprises a target sequence and one or more ligation probes and is formed when a hybridization domain of a ligation probe hybridizes to a target domain of a target sequence. The term "to hybridize" means to form a stable duplex. "Duplex" means at least two oligonucleotides that are fully or partially complementary undergoing Watson-Crick type base pairing among all or most of their nucleotides. In one embodiment, a stable duplex is a duplex structure that is not destroyed by a stringent wash, e.g. conditions including temperature of about 5 °C less that the Tₘ of a strand of the duplex and low monovalent salt concentration, e.g. less than 0.2 M, or less than 0.1 M. The term "duplex" includes pairing with nucleotide analogs, such as deoxyinosine, nucleosides with 2-aminopurine bases, PNAs, and the like. A "mismatch" in a duplex between two oligonucleotides refers to a pair of nucleotides in opposite strands of the duplex failing to undergo Watson-Crick bonding.

In exemplary embodiments, a hybridization domain is perfectly complementary to a target domain. In some embodiments, a hybridization domain is substantially complementary to a target domain. Two single stranded RNA or DNA molecules may be said to be "substantially complementary" when the nucleotides of one strand, optimally aligned and compared and with appropriate nucleotide insertions or deletions, pair with at least about 80% of the nucleotides of the other strand, usually at least about 90% to 95%, and more particularly from about 98 to 100%. In general, hybridization domains should have no more than 1 or 2 bases that are not perfectly matched to the target (aside from interrogation nucleotides). In some cases, for example, a target domain may contain more than one SNP position, and probe sets are designed to allow hybridization to query the first SNP position and the second SNP position, thus requiring a mismatch at the second or first position, respectfully.

Alternatively, substantial complementarity exists when an RNA or DNA strand will hybridize under selective hybridization conditions to its complement. Typically, selective hybridization will occur when there is at least about 65% complementary over a stretch of at least about 14 to about 25 nucleotides, particularly at least about 75%, more particularly at least about 90% complementary. See, generally, M. Kanehisa, Nucleic Acids Res., 2004, 12: 203.

In some embodiments, a hybridization complex comprises a primary target sequence and a first and a second ligation probe, wherein a terminal nucleotide of a first hybridization domain is separated from the closer terminal nucleotide of a second hybridization domain by at least one nucleotide. In some embodiments, a hybridization complex comprises a primary target sequence and a first and a second ligation probe, wherein a terminal nucleotide of a first hybridization domain is separated from a terminal nucleotide of a second hybridization domain by 1, 2, 3, 4, 5 or 6 nucleotides. In exemplary embodiments, a hybridization complex comprises a primary target sequence and a first and a second ligation probe, wherein a terminal nucleotide of a first hybridization domain is separated from a terminal nucleotide of a second hybridization domain by 2 nucleotides.

In some aspects, a hybridization complex comprises a primary target sequence and a ligation probe (for example, an open circle probe) wherein a terminal nucleotide of a first hybridization domain is separated from the closer terminal nucleotide of a second hybridization domain by at least one nucleotide. In some aspects, a hybridization complex comprises a primary target sequence and a ligation probe (for example, an open circle probe), wherein a terminal nucleotide of a first hybridization domain is separated from a terminal nucleotide of a second hybridization domain by 1, 2, 3, 4, 5 or 6 nucleotides. In exemplary aspects, a hybridization complex comprises a primary target sequence and a ligation probe (for example, an open circle probe), wherein a terminal nucleotide of a first hybridization domain is separated from a terminal nucleotide of a second hybridization domain by 2 nucleotides.

### Ligation

A hybridization complex can be subjected to conditions such that the ends of a first and a second ligation probe or the ends of the same ligation probe are ligated to form a ligated probe. That is, ligation can be intra- or intermolecular. The ligation probe or probes are typically hybridized to a target sequence before ligation. In exemplary embodiments, the ends of a first and a second ligation probe are ligated together if an interrogation nucleotide of the first ligation probe is perfectly complementary to a detection nucleotide on a target sequence. In some embodiments, the interrogation nucleotide is directly ligated to a second ligation probe. In exemplary embodiments, the interrogation nucleotide is coupled to one or more nucleotides, particularly one nucleotide, and the last coupled nucleotide is ligated to a second ligation probe. In some embodiments, the ends of a ligation probe are ligated together if an interrogation nucleotide is perfectly complementary to a detection nucleotide on a target sequence.

In embodiments where no ligation probe in a hybridization complex comprises an interrogation nucleotide, a ligation probe can be extended by a polymerase such that the ligation probe comprises an interrogation nucleotide. The interrogation nucleotide can then be ligated to a second ligation probe or further coupled to one or more nucleotides, the last of which is ligated to a second ligation probe. In another format, where the hybridization complex comprises a ligation probe, the interrogation nucleotide can then be ligated to the other end of the ligation probe.

In some embodiments, the hybridization complex is contacted with a nuclease (e.g. an exonuclease) after being subjected to conditions to extend and/or ligate the ends of one or more ligation probes. One reason for using a nuclease at such time is to degrade ligation probes that have not been successfully extended and/or ligated. Thus, in some embodiments, a method herein comprises removing or separating or degrading (or a combination thereof) an unligated ligation probe (such as a first and second ligation probe that have not ligated to each other, or a ligation probe that has not ligated to itself), for example, after an extending step, ligating step or both.

Ligation can be performed in various ways. In exemplary embodiments, a ligating enzyme, such as a ligase, is contacted with one or more ligation probes. In exemplary embodiments, the ligase is T4 DNA ligase. T4 DNA ligase catalyzes the formation of a phosphodiester bond between 5' phosphate and 3' hydroxyl termini in duplex DNA or RNA. In particular, T4 DNA ligase can seal single stranded nicks in duplex DNA, RNA or DNA/RNA hybrids. Any ligase that can perform this function can be used in the methods herein. Examples of other useful ligases include Ampligase® DNA Ligase, Chlorella virus PBCV-1 DNA ligase, human DNA ligase I and human DNA ligase III.

In some embodiments, a ligating reagent is contacted with one or more ligation probes. Typical ligating reagents are small (e.g. < 500D) molecules that are known to effect "chemical ligation" of nucleic acids. Examples of ligating reagents include cyanogen bromide (BrCN), carbodiimide, N-cyanoimidazole, 1-ethyl-3-(3-dimethylaminopropyl)-carbodiimide hydrochloride, See Dolinnaya et al., Nucleic Acids Research, 1988, 16(9): 3721-3738; and Shabarova et al., Nucleic Acids Research, 1991, 19(15): 4247-4251; Metelev et al., Nucleosides & Nucleotides (1999) 18:2711; Luebke and Dervan, J. Am. Chem. Soc. (1989) 111: 8733. Kool (U.S. Patent No. 7,033,753) describes the use of chemical ligation and fluorescence resonance energy transfer (FRET) to detect genetic polymorphisms. Other chemical ligation methods react a 5'-tosylate or 5'-iodo group with a 3'-phosphorothioate group, resulting in a DNA structure with a sulfur replacing one of the bridging phosphodiester oxygen atoms. See Gryanov, S.M., and Letsinger, R.L., Nucleic Acids Research (1993) 21:1403; Xu, Y. and Kool, E.T. Tetrahedron Letters (1997) 38:5595; and Xu, Y. and Kool, E.T., Nucleic Acids Research (1999) 27: 875. See also US 2008/0124810 for discussion of other chemical ligation techniques known in the art.

Thus, if an interrogation nucleotide is perfectly complementary to a detection nucleotide in a hybridization complex, the hybridization complex can be subjected to any ligation technique, such as those described above, in order to ligate one or more probes.

### Extension

In some embodiments, one or more ligation probes is extended by one or more nucleotides. In exemplary embodiments, a first or second ligation probe in a hybridization complex is contacted with a polymerase and one or more dNTPs (in other words, free bases or free nucleotides), thereby extending the first or second ligation probe by the one or more dNTPs to form an extended first or second ligation probe. In some aspects, a ligation probe (such as an open circle probe) is contacted with a polymerase and one or more dNTPs, thereby extending the ligation probe by the one or more dNTPs to form an extended ligation probe. By employing an extension step in addition to a ligation step, the specificity of an assay can in some instances be increased, since a successful extension due, for example, to the matching of an interrogation nucleotide to a detection site is needed in some embodiments before a ligation can occur between an extended ligation probe and itself or another ligation probe. Thus, in some embodiments, a ligation probe, such as a first or second ligation probe, is extended with one or more dNTPs before a ligation step. In some embodiments, a ligation probe, such as a first or second ligation probe, is extended by 1, 2, 3,4, 5, 6, 7, 8, 9 or 10 nucleic acids. In exemplary embodiments, a ligation probe, such as a first or second ligation probe, is extended by 1 nucleic acid. The term "filled-in" may be synonymous with "extended", particularly when a probe is extended by 1 nucleic acid.

In some embodiments, the end of a ligation probe in a hybridization complex can be extended to include one or more dNTPs In some embodiments, a ligation probe comprises an interrogation nucleotide, and one or more dNTPs are appended to the interrogation nucleotide. In exemplary embodiments, a first ligation probe comprising a terminal interrogation nucleotide is extended by binding a dNTP to the terminal interrogation nucleotide. It may be useful in these embodiments to contact a hybridization complex as described herein with one or more different dNTPs, each in a separate vessel and then to determine which dNTP(s) has or have been incorporated into the ligation probe, for example in the case where there is a single nucleotide gap at the interrogation position. The term "vessel" used herein refers to any container or receptacle for material. One example of a vessel is a well of a microarray as understood in the art.

A "polymerase", as used herein, refers to any protein that is useful for covalently binding a free nucleotide to an oligonucleotide. Many polymerases are known in the art and can be chosen according to the activities or lack of activities possessed by the polymerase. For example, a polymerase can be chosen according to whether it possesses or lacks 5'-3' exonuclease activity, 3'-5' exonuclease activity, strand displacement activity or heat activation/inactivation ability, among others. In some embodiments, the polymerase lacks strand displacement activity, lacks exonuclease activity or both. In exemplary embodiments, the polymerase has 3'-5' exonuclease activity, lacks 5'-3' exonuclease activity and lacks strand displacement activity. An exemplary polymerase for use in extension, for example before ligation, is T4 polymerase. As described herein, a polymerase used for extension in some embodiments can be different from a polymerase used for amplification of a nucleic acid product, such as a secondary target sequence. For example, T4 polymerase could be used for extension while Taq DNA polymerase could be used for amplification.

### Circularization

A first and a second ligation probe that have been joined together to form a linear molecule ("a ligated probe") can be joined together again to form a circularized probe. In exemplary embodiments, a ligated probe is separated from a target sequence, such as a primary target sequence, before circularization. In exemplary embodiments, a ligated probe is contacted with a circularizing enzyme. One particularly useful circularizing enzyme is CircLigase™ (Epicentre Biotechnologies). CircLigase™ catalyzes intramolecular ligation (i.e. circularization) of ssDNA templates having a 5'-phosphate and a 3'-hydroxyl group or single stranded oligonucleotides having a 3'-hydroxyl ribonucleotide and a 5'-phosphorylated ribonucleotide or deoxyribonucleotide. Generally, any circularizing enzyme that ligates the 5' and 3' terminal nucleotides of an oligonucleotide, particularly of a single stranded molecule, such as ssDNA, may be used.

In some aspects, a circularization step is not required or performed.

### Cleavage

A probe comprising a cleavage site can be cleaved following methods known in the art or described herein. Cleaving a probe, such as a circularized probe, at a cleavage site comprises subjecting the probe to any condition that is conducive to forming a "secondary target sequence" by bond breakage. In exemplary embodiments, a secondary target sequence comprises, in order, a first priming domain, an address sequence and a second priming domain, with optional sequences on either end, such as hybridization domains and with optional sequences between the address sequence and either priming domain. Cleaving a probe can comprise, for example, contacting the probe with an enzyme; exposing the probe to ionizing radiation, elevated temperature, basic or acidic conditions, or one or more chemical reagents that cause the breakage of bonds at the cleavage site; or any combination of these and other methods described herein. As described above, it may be necessary to hybridize an additional sequence to the cleavage site to form a double stranded site to serve as a substrate for recognition and/or cleavage. In exemplary embodiments, a circularized probe is contacted with an endonuclease, such as endonuclease V. Cleaving a circularized probe at a cleavage site will result in a linear molecule (for example, a secondary target sequence), and this step is sometimes referred to as "linearization" or "relinearization".

### Capture

In some embodiments, capturing a molecule by a capture ligand attached to a solid support may be useful. A wide number of suitable supports can be used, depending on the complexity of the assay; that is, how many different addresses/SNPs are being detected. A capture step can be performed at any point in an assay and the captured molecule can be any molecule generated as a method is being performed. After capture, the support is washed and the various captured species are further subjected to additional steps, for example, amplification and/or detection. The molecule captured by the capture ligand typically comprises a binding label that binds to the capture ligand. In some embodiments, one or more ligation probes, such as a first or second ligation probe, comprise a binding label. In exemplary embodiments, a ligation probe, such as a second ligation probe, comprises a hybridization domain (such as a second hybridization domain that is complementary to a second target domain) and a binding label, and in particular embodiments does not comprise an address sequence or address cassette before the ligation probe is subjected to a ligation step.

In some embodiments, a second ligation probe comprising (i) a binding label and (ii) a second hybridization domain that is complementary to a second target domain is ligated to a first ligation probe comprising (i) an address cassette and (ii) a first hybridization domain that is complementary to a first target domain to provide a secondary target sequence. Often, ligation of first and second ligation probes that are hybridized to a primary target sequence to form a secondary target sequence will not occur unless a nucleotide forms a complementary base pair to a detection nucleotide in the primary target sequence. The secondary target sequence is then captured by a capture ligand attached to a solid support and then detected. Optionally, the secondary target sequence is amplified before detection. Here and in other cases, it would be useful to wash the solid support in order to eliminate species that comprise an address sequence or cassette but that have not successfully ligated to a probe comprising a binding label. Thus, these embodiments exemplify how capture and wash steps may be used to determine whether two probes have been successfully ligated.

### Amplification

A secondary target sequence formed by a method described herein can be directly detected. However, it may be desirable in some cases to amplify a portion of the secondary target sequence in order to, for example, generate multiple copies (i.e. "tertiary target sequences", also referred to as "amplicons") for improved detection. Thus, in some embodiments, an address sequence is amplified. A priming domain pair generally should be oriented with respect to an address sequence on a secondary target sequence such that only the priming domains, any sequence between the primer domain pair and any complement thereof can be amplified. In addition, it may be necessary to choose a primer pair in order to ensure that only the priming domain, any sequence between the primer domain pair, and any complement thereof can be amplified. In exemplary embodiments, a primer hybridizing to a priming domain that is 5' to an address site (i.e., a 5' address primer) has the same 5'-3' sequence as the priming domain. In exemplary embodiments, a primer hybridizing to a priming domain that is 3' to an address site (i.e., a 3' address primer) is the reverse complement of the priming domain; in other words, the 5'-3' sequence of a 3' address primer is the complement of the 3'-5' sequence of the priming domain. In exemplary embodiments, a sequence comprising a portion of a primary target sequence (e.g., a hybridization domain) or of the complement of the primary target sequence is not amplified. In other words, in exemplary embodiments, a tertiary target sequence is not complementary (perfectly, substantially or otherwise) to a primary target sequence (such as genomic DNA) or its complement.

Amplifying a portion of the secondary target sequence (such as an address sequence) to obtain one or more tertiary target sequences can be accomplished through any art known method, such as polymerase chain reaction (PCR). A polymerase as described herein or known in the art can be used. In exemplary embodiments, the polymerase possesses 5'-3' exonuclease activity. In exemplary embodiments, the polymerase lacks strand displacement activity. In exemplary embodiments, the polymerase possesses 5'-3' exonuclease activity and lacks strand displacement activity. Such a polymerase may be used to ensure that circularized probes that have failed to linearize (for example, for lack of a cleavage site) will not amplify. A particularly useful polymerase is Taq DNA polymerase.

In some embodiments, the secondary target is amplified using emulsion PCR. Emulsion PCR isolates polynucleotide molecules along with primer-coated beads in aqueous droplets within an oil phase. Polymerase chain reaction (PCR) then coats each bead with clonal copies of the polynucleotide molecule followed by immobilization for subsequent analysis by, for example, sequencing. Any method of emulsion PCR available to one of skill in the art may be used in the methods of the present invention (see, e.g., Hori et al. (2007) Biochem Biophys Res Commun. 352:323-8; Xu et al. (2010) Biotechniques. 48:409-12; Williams et al. (2006) Nat Methods. 3:545-50; Nakano et al. (2003) J Biotechnol. 102:117-24.)

### Detection

A target sequence, in particular a derivative target sequence such as a tertiary target sequence, can be detected or measured through a variety of assays, methods and detection systems known to one of skill in the art. The term "measuring," "detecting," or "taking a measurement" refers to a quantitative or qualitative determination of a property or characteristic of an entity, e.g., quantifying the amount or the activity level of a molecule or determining the absence or presence of a molecule.

Various methods include but are not limited to ultra-violet (UV) spectroscopy, near-infrared (near-IR) spectroscopy, infrared (IR) spectroscopy, visible spectroscopy, refractive index (RI) spectroscopy, fluorescence spectroscopy, colorimetry, nuclear magnetic resonance spectroscopy (NMR), dispersive Raman spectroscopy, light scattering analysis (LS), mass spectrometry (MS) (such as matrix-assisted laser desorption ionization-time of flight MS (MALDI-TOF MS), pyrolysis MS, ion spray MS), gas chromatography (optionally combined with mass spectrometry), liquid chromatography (optionally combined with mass spectrometry), electrophoresis (such as capillary or gel electrophoresis), radiochemical analysis, and surface plasmon resonance (such as according to systems provided by Biacore Life Sciences) and nephelometry.

Sequences that are newly discovered or known in the art, such as those deposited in a sequence database or provided commercially, can be used to construct probes and primers for various detection methods. Thus, provided herein are probe sets comprising or consisting of a plurality of probes for detecting a target sequence. Also provided herein are primer sets comprising or consisting of a plurality of probes for detecting a target sequence.

As further defined below, a ligand that "specifically binds" or "selectively binds" or is "selective for" a species means that the ligand binds the species with specificity sufficient to differentiate between the species and other components or contaminants of the sample.

In some embodiments, an assay for detecting a target sequence is done in a solution format. In some embodiments, end-point or real time PCR formats are used, as are well known in the art. These assays can be done either as a panel, in individual tubes or wells, or as multiplex assays, using sets of primers and different labels within a single tube or well. qPCR techniques relying on 5' nuclease assays using FRET probes or intercalating dyes such as SYBR Green can also be used. In addition to PCR-based solution formats, other formats can be utilized, including, for example, ligation based assays utilizing FRET dye pairs. In this embodiment, only upon ligation of two (or more) probes that are hybridized to the target sequence is a signal generated.

In exemplary embodiments, an assay for detecting a target sequence is done utilizing an array. Arrays can be divided into two broad classes of surface-based multiplex platforms: those using fixed planar surfaces (fixed arrays, sometimes called chips or biochips) and those that are particle-based (liquid arrays). In a fixed array platform, assays are performed on a shared, typically two-dimensional matrix in which assays are identified by location. In a liquid array platform, each assay is performed on a separate support, such as a bead, which can be identified by some characteristic other than location, such as frequency of fluorescence. In either case, the assay system comprises a substrate or a solid support comprising a capture ligand (also called an adsorbent, affinity reagent or binding ligand, or when nucleic acid is measured, a capture probe).

The term "solid support" or "substrate" refers to any material appropriate for the attachment or association of a capture binding ligand. Suitable substrates include metal surfaces such as gold, electrodes, glass and modified or functionalized glass, plastics (including acrylics, polystyrene and copolymers of styrene and other materials, polypropylene, polyethylene, polybutylene, polycarbonate, polyurethanes, Teflon, derivatives thereof, etc.), polysaccharides, nylon or nitrocellulose, resins, mica, silica or silica-based materials including silicon and modified silicon, carbon, metals, inorganic glasses, fiberglass, ceramics, GETEK (a blend of polypropylene oxide and fiberglass) and a variety of other polymers.

In some embodiments, a solid support is modified to contain discrete individual sites. The surface of a biochip thus can comprise a plurality of addressable locations, each of which comprises a capture binding ligand. An "array location," "addressable location," "pad" or "site" means a location on the substrate that comprises a covalently attached capture ligand. An array can comprise a plurality of capture ligands in a regular, ordered format, such as a matrix. An array can also include controls, replicates of the markers and the like. In some embodiments, compositions comprising a single capture ligand may be made as well. In addition, in some arrays, multiple substrates may be used, either of different or identical compositions. Thus, for example, a large array may comprise a plurality of smaller substrates.

A number of different biochip array platforms as known in the art may be used. For example, the compositions and methods of the present invention can be implemented with array platforms such as GeneChip (Affymetrix), CodeLink Bioarray (Amersham), Expression Array System (Applied Biosystems), SurePrint microarrays (Agilent), Sentrix LD BeadChip or Sentrix Array Matrix (Illumina) and Verigene (Nanosphere). In exemplary embodiments, detecting the presence of target sequence comprising using a liquid array, for example, xMAP (Luminex Corp.) or an Arrayable Liquid Array platform, based on Encoded Sortable Particle (ESP) technology, such as provided by Arrayomics.

A capture probe or capture binding ligand can be covalently attached to a surface, for example, via functional groups, such as amino, hydroxy or thiol groups, that are attached to surfaces such as silanized glass. Alternatively, noncovalent attachment, such as electrostatic, hydrophobic/hydrophilic adhesion can be utilized. As appreciated by those in the art, a large number of attachments are possible on a wide variety of surfaces. For examples of platforms and methods useful for measuring nucleic acids, see U.S. Patent Application Publication Nos. 2006/0275782 and 2005/0064469.

By "binding ligand," "capture binding ligand," "capture binding species," "capture probe" or "capture ligand" herein is meant a compound that will bind to a target analyte, target species or target sequence (all used interchangeably) in order to detect the presence of or to quantify, relatively or absolutely the target analyte, target species or target sequence. In exemplary embodiments, the capture binding ligand or capture probe allows the attachment of a target species or target sequence to a solid support for the purposes of detection as further described herein. Attachment of the target species to the capture binding ligand may be direct or indirect. In exemplary embodiments, the target species is a target sequence, such as a secondary, tertiary or other derivative target sequence. As will be appreciated by those in the art, the composition of the binding ligand will depend on the composition of the target sequence. Binding ligands for a wide variety of species are known or can be readily found using known techniques. Binding ligands include proteins (particularly including antibodies or fragments thereof (FAbs, etc.) as discussed further below) or small molecules. The binding ligand may also have cross-reactivity with proteins of other species. Antigen-antibody pairs, receptor-ligands, and carbohydrates and their binding partners are also suitable analyte-binding ligand pairs. In exemplary embodiments, the binding ligand may be nucleic acid. As is generally described in U.S. Patent Nos. 5,270,163; 5,475,096; 5,567,588; 5,595,877; 5,637,459; 5,683,867; 5,705,337 and related patents, nucleic acid "aptamers" can be developed for binding to virtually any target. There is a wide body of literature relating to the development of binding partners based on combinatorial chemistry methods. See, for example, International Publication No. WO 1998/020162.

A capture probe can bind directly to a target sequence, which can optionally be bound to one or more additional components such as a label or signal probe. In some embodiments of such "sandwich" assays, a capture probe binds to a first domain of the target sequence and the label probe binds to a second domain of the target sequence. Alternatively, a capture probe can bind indirectly to a target sequence through the use of additional probes, such as a soluble binding ligand or soluble capture ligand. For example, a capture probe might bind a first domain of the soluble binding ligand and the target sequence might bind a second domain of the soluble binding ligand.

Often, a "label" or "reporter" is detected in order to determine the presence of the target sequence in a sample. A label is an atom or molecule that enables the detection of the compound. In some embodiments, the label is directly bound (e.g., via a covalent, ionic or hydrogen bond, or van der Waals interaction) to the target sequence. In some embodiments, the label is indirectly bound (e.g., via a molecule) to the target sequence. In some embodiments, the label is bound directly or indirectly with one or more molecules that indicate the presence of the target sequence. A signal generated by a "signaling complex" comprising a label and one or more molecules can be detected to provide an indication as to the presence of a molecule of interest, such as a primary target sequence. In general, labels fall into four classes: a) isotopic labels, which may be radioactive or heavy isotopes; b) magnetic, electrical, thermal; c) luminescent dyes; and d) enzymes; although labels include particles such as magnetic particles as well. The dyes may be chromophores or phosphors but in specific embodiments are fluorescent dyes, which because of their strong signals provide a good signal-to-noise ratio for decoding. Suitable dyes for use in the invention include, but are not limited to, fluorescent lanthanide complexes, including those of europium and terbium, fluorescein, rhodamine, tetramethylrhodamine, eosin, erythrosin, coumarin, methyl-coumarins, pyrene, Malacite green, stilbene, Lucifer Yellow, Cascade Blue, Texas Red, Alexa dyes and others described in Molecular Probes Handbook (6th ed.) by Richard P. Haugland. Additional labels include nanocrystals or Q-dots as described in U.S. Patent No. 6,544,732.

A label can be referred to as either a primary or secondary label. A primary label produces a detectable signal that can be directly detected. For example, a label on a primer or a dNTP incorporated during amplification can be a primary label such as a fluorophore. Alternatively, a label may be a secondary label, such as biotin or an enzyme. A secondary label requires additional reagents that lead to the production of a detectable signal. A secondary label is one that is indirectly detected; for example, a secondary label can bind or react with a primary label for detection, can act on an additional reagent to generate a primary label, or may allow the separation of a compound comprising the secondary label from unlabeled materials, etc. Secondary labels include, but are not limited to, one of a binding partner pair, such as biotin; a binding partner pair; chemically modifiable moieties; nuclease inhibitors; enzymes such as horseradish peroxidase; alkaline phosphatases; luciferases, etc.

In some embodiments, a signaling complex is in a sandwich format wherein a target is unlabeled. In these embodiments, a signaling complex comprises (i) a capture ligand attached to a support, (ii) a target bound to the capture ligand and (iii) a "signal probe" or "label probe" bound independently to the target and either directly or indirectly comprising one or more labels. In these embodiments, the label probe can comprise either a primary (e.g. a fluorophore) or a secondary (biotin or enzyme) label. In some cases, a label probe comprises biotin, which is then bound to a streptavidin-enzyme (e.g. comprising horseradish peroxidase) conjugate and forms a colored precipitate with the addition of a precipitating agent, such as 3,3',5,5'-tetramethylbenzidine (TMB), o-dianisidine (3,3'-dimethoxybenzidine (dihydrochloride), Fast Blue B) or the like. This embodiment has a particular benefit in that the optics for detection do not require the use of a fluorometer or other detector, which can add to the expense of carrying out the methods. In some embodiments, an enzyme such as horseradish peroxidase is directly conjugated to a label probe.

In some embodiments, a signaling complex comprises a binding partner pair. Suitable binding partner pairs include, but are not limited to: antigens (such as a polypeptide) and antibodies (including fragments thereof (FAbs, etc.)); other polypeptides and small molecules, including biotin/streptavidin; enzymes and substrates or inhibitors; other protein-protein interacting pairs; receptor-ligands; and carbohydrates and their binding partners. Nucleic acid-nucleic acid binding proteins pairs are also useful. In certain embodiments, binding partner pairs include, but are not limited to, biotin (or imino-biotin) and streptavidin, digeoxinin and Abs, and Prolinx™ reagents.

In some embodiments, a ligation probe comprises a secondary label, such as biotin. In exemplary embodiments, a ligation probe comprising a secondary label such as biotin is contacted with a solid support comprising a binding partner to the secondary label, such as streptavidin. The complex thus formed can be washed and the biotin-containing moiety can be used in further assays.

As used herein, the term "fluorescent signal generating moiety" or "fluorophore" refers to a molecule or part of a molecule that absorbs energy at one wavelength and re-emits energy at another wavelength. Fluorescent properties that can be measured include fluorescence intensity, fluorescence lifetime, emission spectrum characteristics, energy transfer, and the like. Signals from single molecules can be generated and detected by a number of detection systems, including, but not limited to, scanning electron microscopy, near field scanning optical microscopy (NSOM), total internal reflection fluorescence microscopy (TIRFM), and the like. Abundant guidance is found in the literature for applying such techniques for analyzing and detecting nanoscale structures on surfaces, as evidenced by the following references Reimer et al, editors, Scanning Electron Microscopy: Physics of Image Formation and Microanalysis, 2nd Edition (Springer, 1998); Nie et al, Anal. Chem., 78: 1528-1534 (2006); Hecht et al, Journal Chemical Physics, 112: 7761-7774 (2000); Zhu et al, editors, Near-Field Optics: Principles and Applications (World Scientific Publishing, Singapore, 1999); Drmanac, WO 2004/076683; Lehr et al, Anal. Chem.., 75: 2414-2420 (2003); Neuschafer et al, Biosensors & Bioelectronics, 18: 489-497 (2003); Neuschafer et al, U.S. Patent No. 6,289,144; and the like. Thus, a detection system for fluorophores includes any device that can be used to measure fluorescent properties as discussed above. In various embodiments, the detection system comprises an excitation source, a fluorophore, a wavelength filter to isolate emission photons from excitation photons and a detector that registers emission photons and produces a recordable output, in some embodiments as an electrical signal or a photographic image. Examples of detection devices include without limitation spectrofluorometers and microplate readers, fluorescence microscopes, fluorescence scanners (including e.g. microarray readers) and flow cytometers.

In some embodiments, a molecule can be considered labeled if it is characterized by a detectable difference. For example, a "size label" is a molecule that differs in size from another molecule. Size labels can be distinguished by detecting or measuring the difference in size, such as through mass spectrometry or electrophoresis. Any of a number of analytical systems known in the art may be used for this purpose. For example, LabChip GX/GXII (Caliper Life Sciences), 3130 Genetic Analyzer and other Analyzer systems (Applied Biosystems), PA 800 *plus* (Beckman Coulter), MegaBACE (GE Healthcare), and 2100 Bioanalyzer (Agilent) have electrophoretic capabilities that are useful for analyte separation and genetic analysis.

Capture binding ligands that are useful in the present invention may be "selective" for, "specifically bind" or "selectively bind" their target. A capture binding ligand is selective for a target if the capture binding ligand binds to the target with a higher binding constant or lower dissociation constant compared to a different capture binding ligand. Typically, specific or selective binding can be distinguished from non-specific or non-selective binding when the dissociation constant (K_{D}) is less than about 10⁻⁵ M, less than about 10⁻⁶ M, less than about 10⁻⁷ M, less than about 10⁻⁸ M, less than about 10⁻⁹ M , less than about 10⁻¹⁰ M, less than about, less than about 10⁻¹¹ M, less than about 10⁻¹² M, less than about 10⁻¹³ M, less than about 10⁻¹⁴ M, or less than about 10⁻¹⁵ M. Specific binding can be detected, for example, by ELISA, immunoprecipitation, coprecipitation, with or without chemical crosslinking, two-hybrid assays and the like. Appropriate controls can be used to distinguish between specific and nonspecific binding.

Capture probes that "selectively bind" (i.e., are "complementary" or "substantially complementary") to or are "selective for" a target nucleic acid find use in the present invention. "Complementary" or "substantially complementary" refers to the hybridization or base pairing or the formation of a duplex between nucleotides or nucleic acids, such as, for instance, between the two strands of a double stranded DNA molecule or between an oligonucleotide primer and a priming domain on a single stranded nucleic acid; these terms are defined above.

### Kits

Disclosed herein are kits for performing any of the methods disclosed herein. Kits may comprise a portable carrier, such as a box, carton, tube or the like, having in close confinement therein one or more containers, such as vials, tubes, ampoules, bottles, pouches, envelopes and the like. In various aspects, a kit comprises one or more components selected from one or more media or media ingredients and reagents for the performing the genotyping methods disclosed herein. For example, kits of the disclosure may also comprise, in the same or different containers, in any combination, one or more enzymes (e.g. a polymerase, a nuclease, a ligase and the like in any combination), one or more primers, one or more probes, one or more binding ligands, one or more buffers, one or more nucleotides (such as deoxynucleoside triphosphates (dNTPs) and labeled dNTPs), one or more detectable labels and markers and one or more solid supports, any of which is described herein. The components may be contained within the same container, or may be in separate containers to be admixed prior to use. A kit may also comprise one or more instructions or protocols for carrying out the methods disclosed herein. A kit may comprise a detector for detecting a signal generated through use of the components of the disclosure in conjunction with a sample. A kit may also comprise a computer or a component of a computer, such as a computer-readable storage medium or device. Examples of storage media include, without limitation, optical disks such as CD, DVD and Blu-ray Discs (BD); magneto-optical disks; magnetic media such as magnetic tape and internal hard disks and removable disks; semi-conductor memory devices such as EPROM, EEPROM and flash memory; and RAM. Any storage medium can be transformed (for example, electromagnetically) to comprise software that may be interpreted by a computer to aid in analyzing the signals produced by the various species described herein. Generally, any of the methods disclosed herein can comprise using any of the kits (comprising primers, probes, labels, ligands, reagents and solid supports in any combination) disclosed herein.

### Assay Formats

### Competitive Ligation

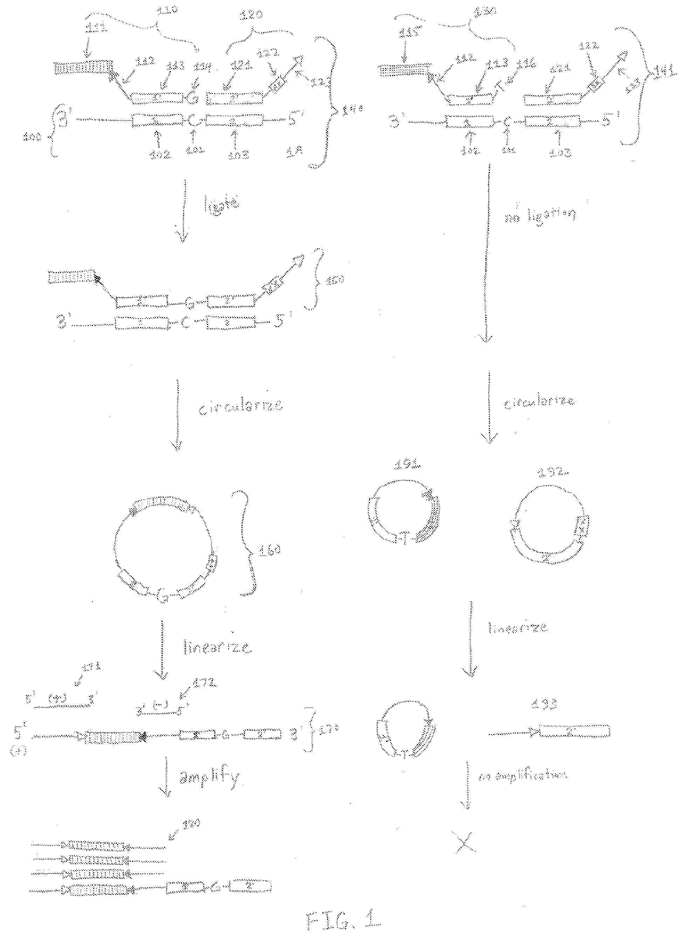

The components described herein can be used in various combinations to detect a primary target sequence in a number of different ways. Figs. 1, 2, 5 and 6 show examples of how a "competitive ligation" assay is performed. A plurality of different ligation probes comprising different interrogation nucleotides compete to bind a given target. When a ligation probe comprises an interrogation nucleotide that forms a complementary base pair with a detection nucleotide in a hybridization complex, ligation occurs between the ends of two ligation probes or the end of the same ligation probe (for example, with pre-circle probes). A competitive ligation assay can be performed in the same vessel using multiple first ligation probes differing by interrogation nucleotide.

In Fig. 1, a primary target sequence 100 comprises a detection nucleotide 101, a first target domain 102 and a second target domain 103. A first ligation probe 110 comprises an address sequence 111, a first priming domain 112, a first hybridization domain 113 and an interrogation nucleotide 114. A second ligation probe 120 comprises a second hybridization domain 121, a cleavage site 122 and a second priming domain 123. The first hybridization domain 113 is complementary and thus hybridizes to the first target domain 102. The second hybridization domain 121 is complementary and thus hybridizes to the second target domain 103. A hybridization complex 140 is thus formed. Because of the complementary base pairing between the interrogation nucleotide 114 and the detection nucleotide 101, ligation can occur between the interrogation nucleotide 114 to the proximate end of the second ligation probe 120. Following ligation, the ends of the ligated probe 150 are joined to form a circularized probe 160. The circularized probe 160 is then cleaved at the cleavage site to result in a secondary target sequence 170. A portion of the secondary target sequence 170 is amplified using a 5' primer 171 and a 3' primer 172 to generate tertiary target sequences 180, which can be labeled and detected as described herein.

In the hybridization complex 141, a first ligation probe 130 comprises an address sequence 115 (different from address sequence 111) and an interrogation nucleotide 116 that does not form a complementary base pair with detection nucleotide 101. No ligation therefore occurs. Circular species 191 does not linearize and is not amplified if, for example, a polymerase having 5'-3' exonuclease activity and lacking strand displacement activity is used. Circular species 192 is linearized to species 193, which is not amplified because the 5' primer used for amplification has the same sequence as the second priming domain and hence will not bind. The address sequence 115 is not amplified.

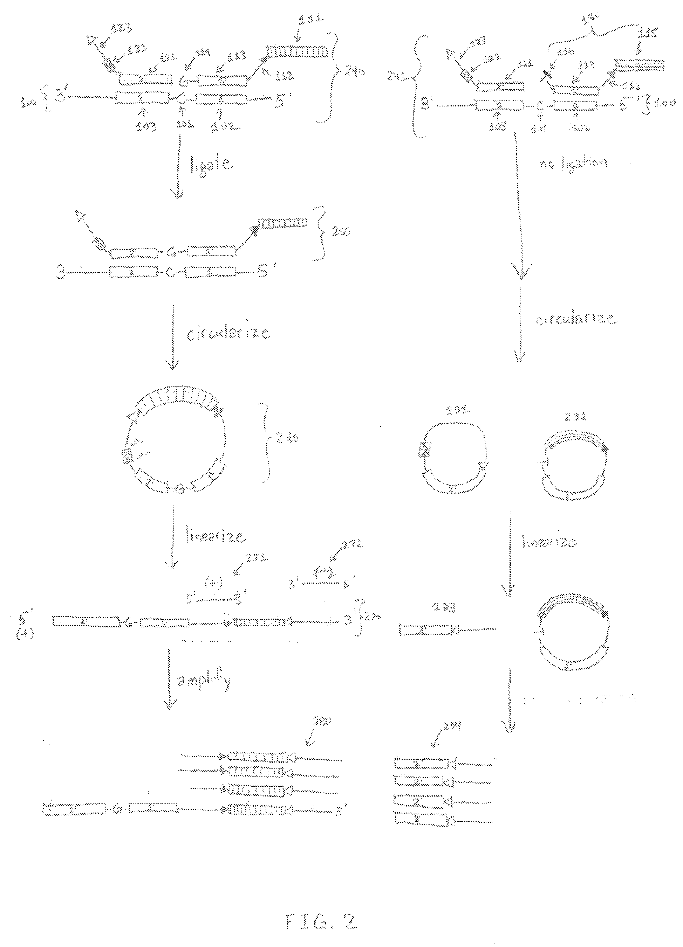

Fig. 2 shows the same general assay as that shown in Fig. 1 except that the first target domain 102 of the primary target sequence 100 is 5' to the second target domain 103. A hybridization complex 240 is formed by hybridization of the first and second ligation probes to the primary target sequence. Because of the complementary base pairing between the interrogation nucleotide 114 and the detection nucleotide 101, ligation can occur to form ligated probe 250. Following ligation, the ends of the ligated probe 250 are joined to form a circularized probe 260. The circularized probe 260 is then cleaved at the cleavage site to result in a secondary target sequence 270. A portion of the secondary target sequence 270 is amplified using a 5' primer 271 and a 3' primer 272 to generate tertiary target sequences 280, which can be labeled and detected as described herein. Tertiary target sequences 280 will form the majority of the amplified product.

In the hybridization complex 241, a first ligation probe 130 comprises an address sequence 115 (different from address sequence 111) and an interrogation nucleotide 116 that does not form a complementary base pair with detection nucleotide 101. No ligation therefore occurs. Circular species 292 does not linearize and is not amplified if, for example, a polymerase having 5'-3' exonuclease activity and lacking strand displacement activity is used. Circular species 291 is linearized to species 293 and amplifies, but amplicons 294 lack an address sequence, and in exemplary embodiments will not be detected.

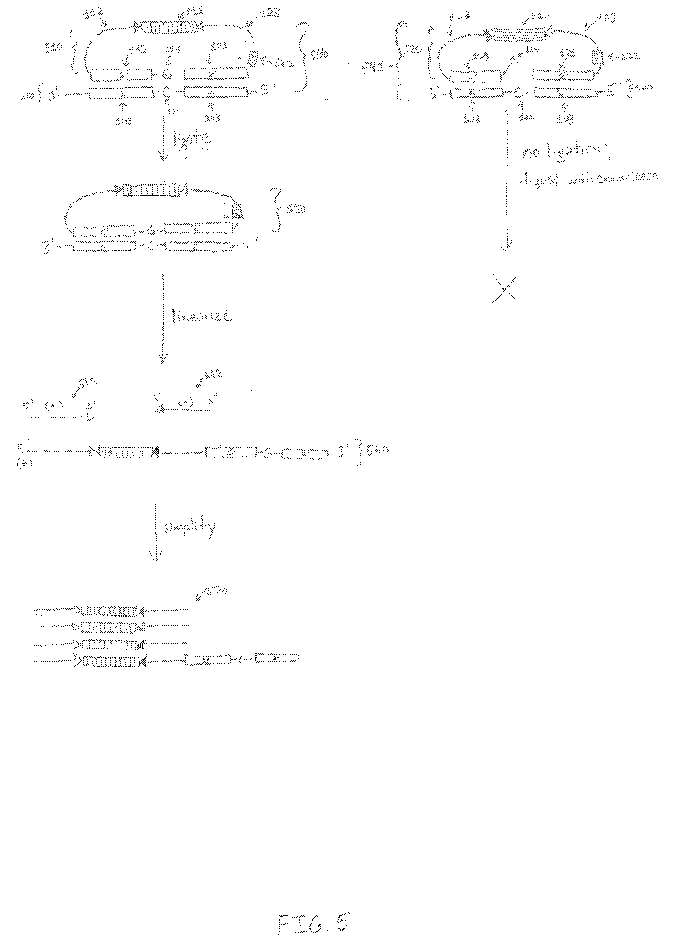

Fig. 5 shows a competitive ligation assay in which an open circle probe 510 is hybridized to the primary target sequence 100. Because of the complementary base pairing between the interrogation nucleotide 114 and the detection nucleotide 101 in hybridization complex 540, the interrogation nucleotide 114 can be ligated to the other end of the open circle probe 510, thus forming circle probe 550. Circle probe 550 is then cleaved at the cleavage site to result in a secondary target sequence 560. A portion of the secondary target sequence 560 is amplified using a 5' primer 561 and a 3' primer 562 to generate tertiary target sequences 570, which can be labeled and detected as described herein.

In the hybridization complex 541, the open circle probe 520 comprises an address sequence 115 (different from address sequence 111) and an interrogation nucleotide 116 that does not form a complementary base pair with detection nucleotide 101. No ligation therefore occurs. The open circle probe 511 is thus removed from a sample, for example, by degradation via an exonuclease. The address sequence 115 is thus not amplified.

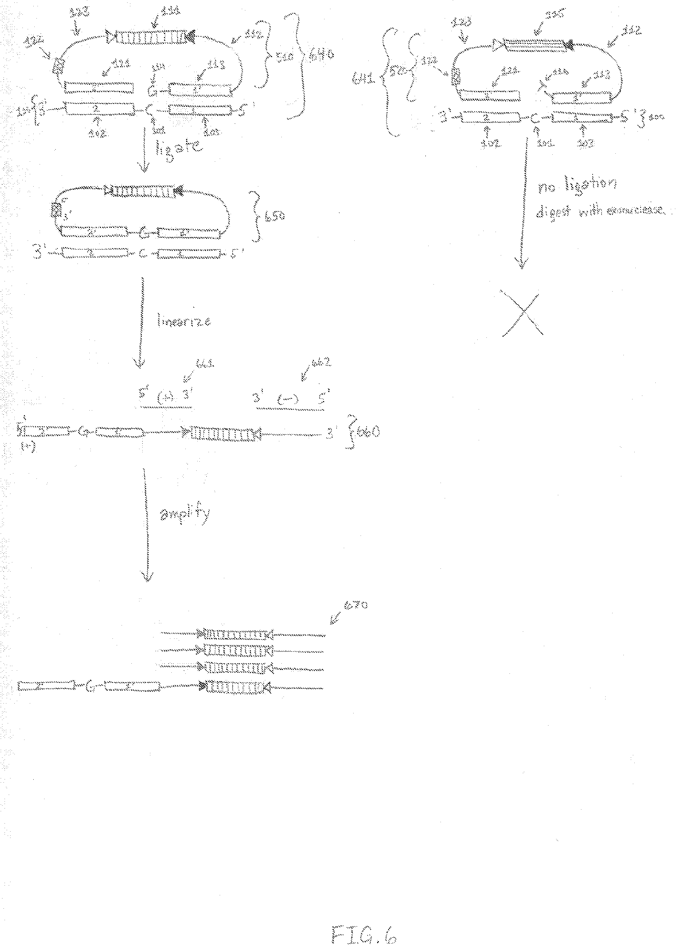

Fig. 6 shows the same general assay as that shown in Fig. 5 except that the first target domain 102 of the target sequence 100 is 5' to second target domain 103. A hybridization complex 640 is formed by hybridization of circle probe 510 to the primary target sequence 100. Because of the complementary base pairing between the interrogation nucleotide 114 and the detection nucleotide 101, ligation can occur to form circle probe 650. Circle probe 650 is then cleaved at the cleavage site to result in a secondary target sequence 660. A portion of the secondary target sequence 660 is amplified using a 5' primer 661 and a 3' primer 662 to generate tertiary target sequences 670, which can be labeled and detected as described herein. Tertiary target sequences 670 will form the majority of the amplified product.

In the hybridization complex 641, the open circle probe 520 comprises an address sequence 115 (different from address sequence 111) and an interrogation nucleotide 116 that does not form a complementary base pair with detection nucleotide 101. No ligation therefore occurs. The open circle probe 520 is thus removed from a sample, for example, by degradation via an exonuclease. The address sequence 115 is thus not amplified.

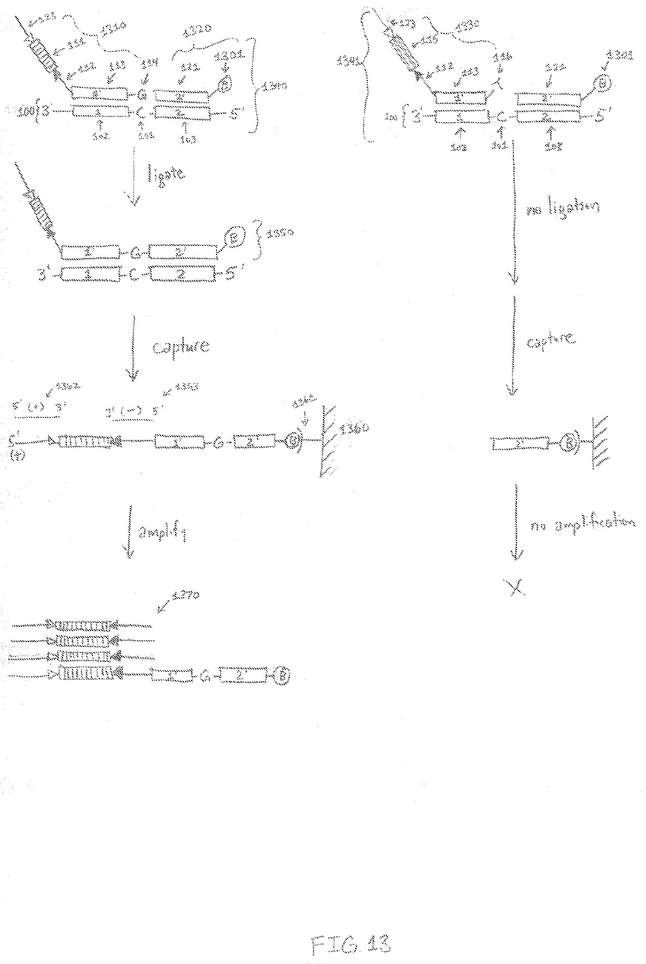

Fig. 13 shows a type of competitive ligation assay in which one probe comprises an address cassette and another probe comprises a binding label. This embodiment is described further below.

### Competitive Extension and Ligation

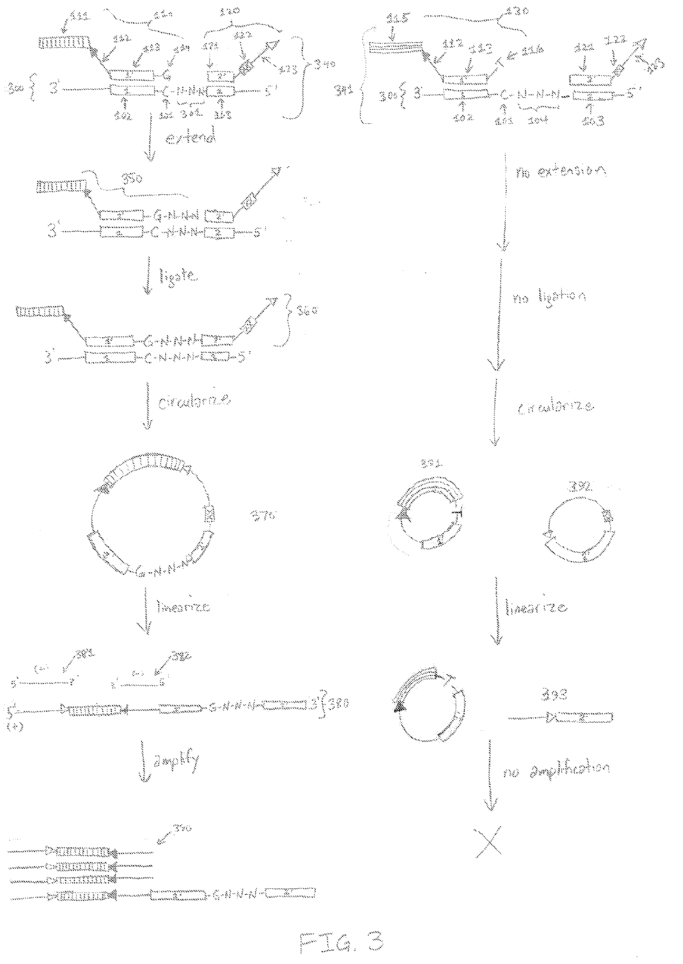

Figs. 3,4, 7 and 8 show examples of how a competitive extension and ligation assay is performed. A plurality of different ligation probes comprising different interrogation nucleotides compete to bind a given target. In some embodiments, the interrogation nucleotide is extended by one or more nucleotides (for example by a polymerase). In some embodiments, a ligation probe, for example at the end of a hybridization domain, is extended by one or more nucleotides. In either case, when a ligation probe comprises an interrogation nucleotide that forms a complementary base pair with a detection nucleotide in a hybridization complex, ligation occurs between the ends of two ligation probes or the ends of the same ligation probe. A competitive extension and ligation assay can be performed in the same vessel using multiple ligation probes differing by interrogation nucleotide.

In Fig. 3, a primary target sequence 300 comprises a detection nucleotide 101, a first target domain 102, a second target domain 103 and a target extension domain 301. A first ligation probe 110 comprises an address sequence 111, a first priming domain 112, a first hybridization domain 113 and an interrogation nucleotide 114. A second ligation probe 120 comprises a second hybridization domain 121, a cleavage site 122 and a second priming domain 123. The first hybridization domain 113 is complementary and thus hybridizes to the first target domain 102. The second hybridization domain 121 is complementary and thus hybridizes to the second target domain 103. A hybridization complex 340 is thus formed. Because of the complementary base pairing between the interrogation nucleotide 114 and the detection nucleotide 101, a polymerase can extend the first ligation probe 110 with base pairs complementary to a target extension domain 301, forming an extended first ligation probe 350. The terminal nucleotide of the extended first ligation probe 350 is ligated to the proximate end of the second ligation probe to form a ligated probe 360. Following ligation, a circularizing enzyme joins the ends of the ligated probe 360 to form a circularized probe 370. The circularized probe 370 is then cleaved at the cleavage site to result in a secondary target sequence 380. A portion of the secondary target sequence 380 is amplified using a 5' primer 381 and a 3' primer 382 to generate tertiary target sequences 390, which can be labeled and detected as described herein.

In the hybridization complex 341, a first ligation probe 130 comprises an address sequence 115 (different from address sequence 111) and an interrogation nucleotide 116 that does not form a complementary base pair with detection nucleotide 101. No extension and no ligation therefore occur. Circular species 391 does not linearize and is not amplified if, for example, a polymerase having 5'-3' exonuclease activity and lacking strand displacement activity is used. Circular species 392 is linearized to species 393, which is not amplified because the 5' primer used for amplification has the same sequence as the second priming domain and hence will not bind. The address sequence 115 is not amplified.

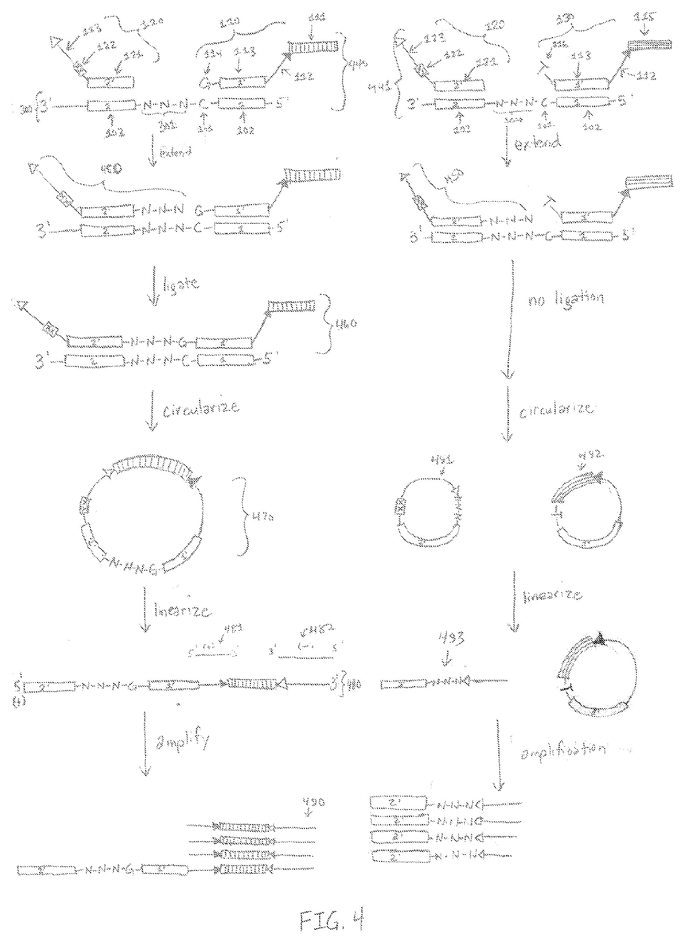

Fig. 4 shows the same general assay as that shown in Fig. 3 except that the first target domain 102 of the primary target sequence 300 is 5' to the second target domain 103. A polymerase can extend the second ligation probe 120 with base pairs complementary to a target extension domain 301, forming an extended second ligation probe 450. Because of the complementary base pairing between the interrogation nucleotide 114 and the detection nucleotide 101, the end of extended second ligation probe 450 is ligated to the proximate end of the first ligation probe, forming ligated probe 460. Following ligation, a circularizing enzyme joins the ends of the ligated probe 460 to form a circularized probe 470. The circularized probe 470 is then cleaved at the cleavage site to result in a secondary target sequence 480. A portion of the secondary target sequence 480 is amplified using a 5' primer 481 and a 3' primer 482 to generate tertiary target sequences 490, which can be labeled and detected as described herein. Tertiary target sequences 490 will form the majority of the amplified product.

In the hybridization complex 441, a second ligation probe 120 is extended with base pairs complementary to a target extension domain 301, forming an extended second ligation probe 450. The first ligation probe 130 comprises an address probe 115 (different from address sequence 111) and an interrogation nucleotide 116 that does not form a complementary base pair with detection nucleotide 101. No ligation therefore occurs. Circular species 492 does not linearize and is not amplified if, for example, a polymerase having 5'-3' exonuclease activity and lacking strand displacement activity is used. Circular species 491 is linearized to species 493 and amplifies, but amplicons 494 lack an address sequence, and in exemplary embodiments will not be detected.

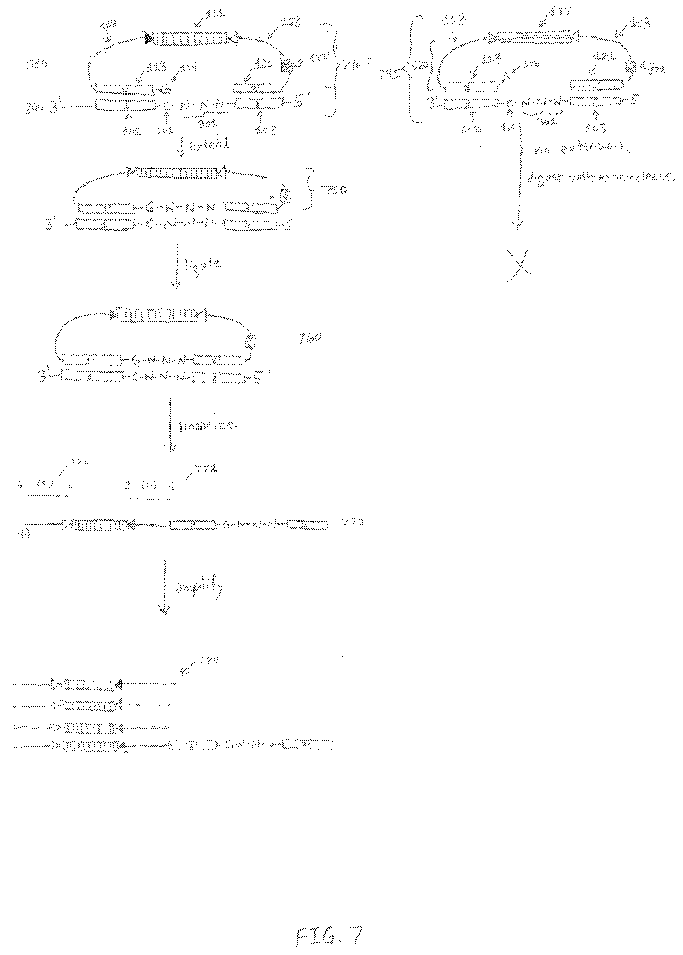

Fig. 7 shows a competitive extension and ligation assay in which an open circle probe 510 is hybridized to the primary target sequence 300. Because of complementary base pairing between the interrogation nucleotide 114 and the detection nucleotide 101 in hybridization complex 740, a polymerase can extend the open circle probe 510 from the interrogation nucleotide 114 with complementary base pairings to a target extension domain 104, forming an extended open circle probe 750. The end of extended open circle probe 750 can be ligated to the other end of the probe, forming circle probe 760. The circle probe 760 is then cleaved at the cleavage site to result in a secondary target sequence 770. A portion of the secondary target sequence 770 is amplified using a 5' primer 771 and a 3' primer 772 to generate tertiary target sequences 780, which can be labeled and detected as described herein.

In the hybridization complex 741, the open circle probe 520 comprises an address probe 115 (different from address sequence 111) and an interrogation nucleotide 116 that does not form a complementary base pair with detection nucleotide 101. No extension and ligation therefore occur. The open circle probe 520 is removed from a sample, for example, by degradation via an exonuclease. The address sequence 115 is thus not amplified.

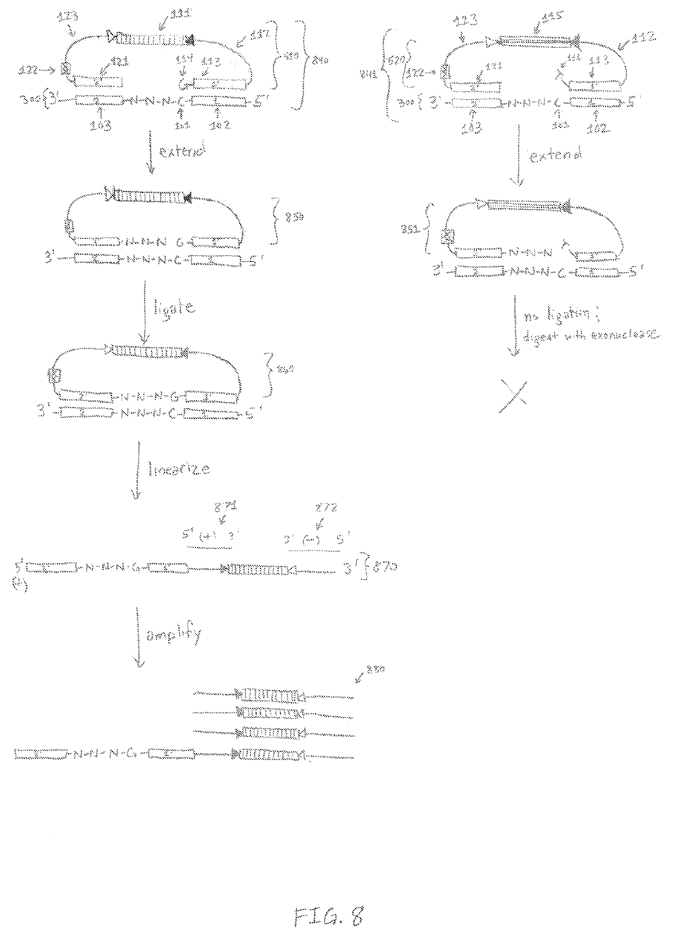

Fig. 8 shows the same general assay as that shown in Fig. 7 except that the first target domain 102 of the target sequence 100 is 5' to second target domain 103. A hybridization complex 840 is formed by hybridization of open circle probe 510 to the primary target sequence 300. A polymerase can extend the open circle probe 510, forming an extended open circle probe 850. Because of the complementary base pairing between the interrogation nucleotide 114 and the detection nucleotide 101, ligation can occur to form circle probe 860. Circle probe 860 is then cleaved at the cleavage site to result in a secondary target sequence 870. A portion of the secondary target sequence 870 is amplified using a 5' primer 871 and a 3' primer 872 to generate tertiary target sequences 880, which can be labeled and detected as described herein. Tertiary target sequences 880 will form the majority of the amplified product.

In the hybridization complex 841, the open circle probe 520 is extended with base pairs complementary to a target extension domain 301, forming an extended open circle probe 851. The open circle probe 520 comprises an address sequence 115 (different from address sequence 111) and an interrogation nucleotide 116 that does not form a complementary base pair with detection nucleotide 101. No ligation therefore occurs. The open circle probe 520 is thus removed from a sample, for example, by degradation via an exonuclease. The address sequence 115 is thus not amplified.

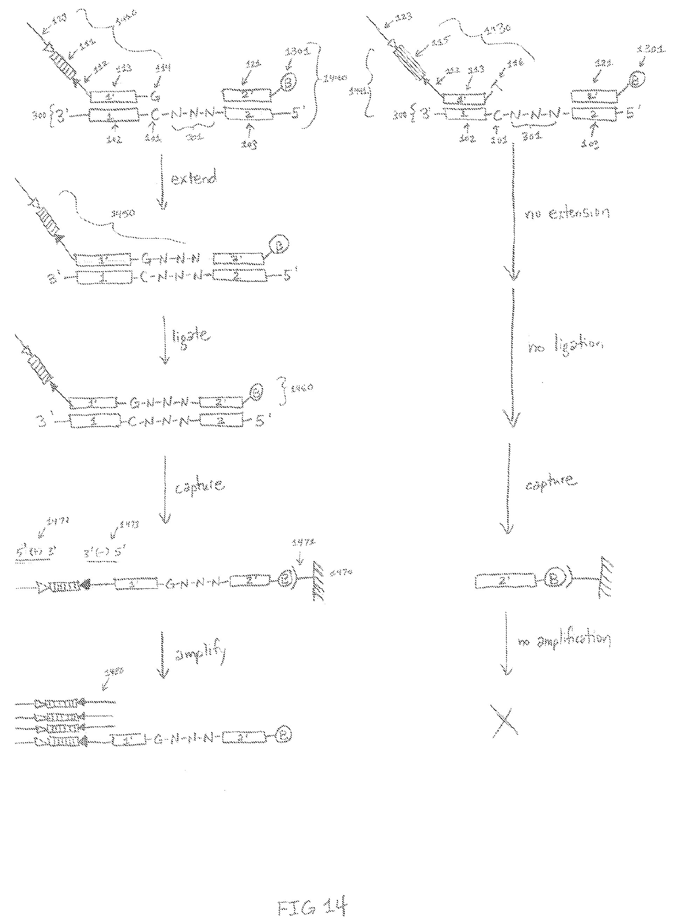

Fig. 14 shows a type of competitive extension and ligation assay in which one probe comprises an address cassette and another probe comprises a binding label. This embodiment is described further below.

### Free Base Assay

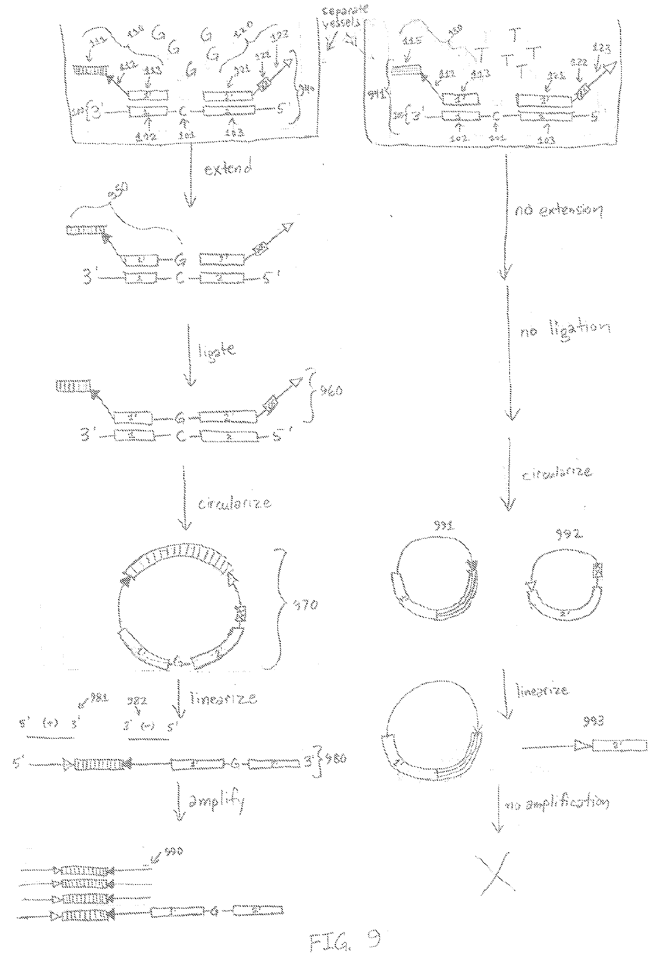

Figs. 9, 10, 11 and 12 show examples of how a "free base" or "free nucleotide" assay is performed. In this embodiment, no ligation probe initially comprises an interrogation nucleotide. The assay is performed with the addition of the same ligation probe(s) but different free nucleotides to separate reaction vessels. The ligation probes optionally differ in the address sequence. When a free nucleotide forms a complementary base pair with a detection nucleotide, extension and ligation can occur between the ends of two ligation probes or of the same ligation probe.

In Fig. 9, a primary target sequence 100 comprises a detection nucleotide 101, a first target domain 102 and a second target domain 103. A first ligation probe 110 comprises an address sequence 111, a first priming domain 112 and a first hybridization domain 113. A second ligation probe 120 comprises a second hybridization domain 121, a cleavage site 122 and a second priming domain 123. The first hybridization domain 113 is complementary and thus hybridizes to the first target domain 102. The second hybridization domain 121 is complementary and thus hybridizes to the second target domain 103. A hybridization complex 940 is thus formed. Because of the complementary base pairing between a free nucleotide G and the detection nucleotide 101, a polymerase can extend the first ligation probe 110 with the free nucleotide G. The terminal nucleotide of the extended first ligation probe 950 is then ligated to the proximate end of the second ligation probe. Following ligation, a circularizing enzyme joins the ends of the ligated probe 960 to form a circularized probe 970. The circularized probe 970 is then cleaved at the cleavage site to result in a secondary target sequence 980. A portion of the secondary target sequence 980 is amplified using a 5' primer 981 and a 3' primer 982 to generate tertiary target sequences 990, which can be labeled and detected as described herein.

In a separate vessel, hybridization complex 941 comprises a first ligation probe 130 that comprises address sequence 115 that is different from address sequence 111. In some embodiments, the same first ligation probe having the same address sequence is used across vessels. The free nucleotide T does not form a complementary base pair with the detection nucleotide 101. Extension and ligation therefore do not occur. The probes are then circularized. Circular species 991 does not linearize and is not amplified if, for example, a polymerase having 5'-3' exonuclease activity and lacking strand displacement activity is used. Circular species 992 is linearized to species 993, which is not amplified because the 5' primer used for amplification has the same sequence as the second priming domain and hence will not bind. The address sequence 115 is thus not amplified.

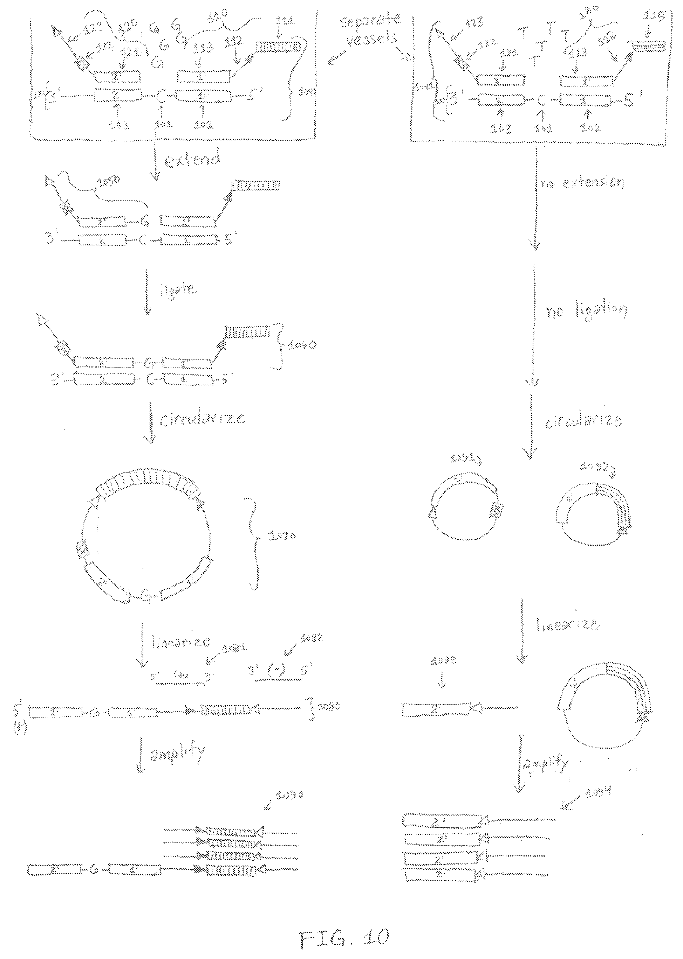

Fig. 10 shows the same general assay as that shown in Fig. 9 except that the first target domain 102 of the target sequence 100 is 5' to the second target domain 103. Complementary base pairing between free nucleotide G and detection nucleotide 101 allows formation of an extended second ligation probe 1050. Ligation between the extended second ligation probe 1050 and first ligation probe results in ligated probe 1060, which is circularized to form circularized probe 1070. Circularized probe 1070 is then cleaved at the cleavage site to result in a secondary target sequence 1080. A portion of the secondary target sequence 1080 is amplified using a 5' primer 1081 and a 3' primer 1082 to generate tertiary target sequences 1090, which can be labeled and detected as described herein. Tertiary target sequences 1090 will form the majority of the amplified product.

In a separate vessel, hybridization complex 1041 comprises a first ligation probe 130 that comprises address sequence 115 that is different from address sequence 111. In some embodiments, the same first ligation probe having the same address sequence is used across vessels. The free nucleotide T does not form a complementary base pair with the detection nucleotide 101. Extension and ligation therefore do not occur. The probes are then circularized. Circular species 1092 does not linearize and is not amplified if, for example, a polymerase having 5'-3' exonuclease activity and lacking strand displacement activity is used. Circular species 1091 is linearized to species 1093 and amplifies, but amplicons 1094 lack an address sequence, and in exemplary embodiments will not be detected.

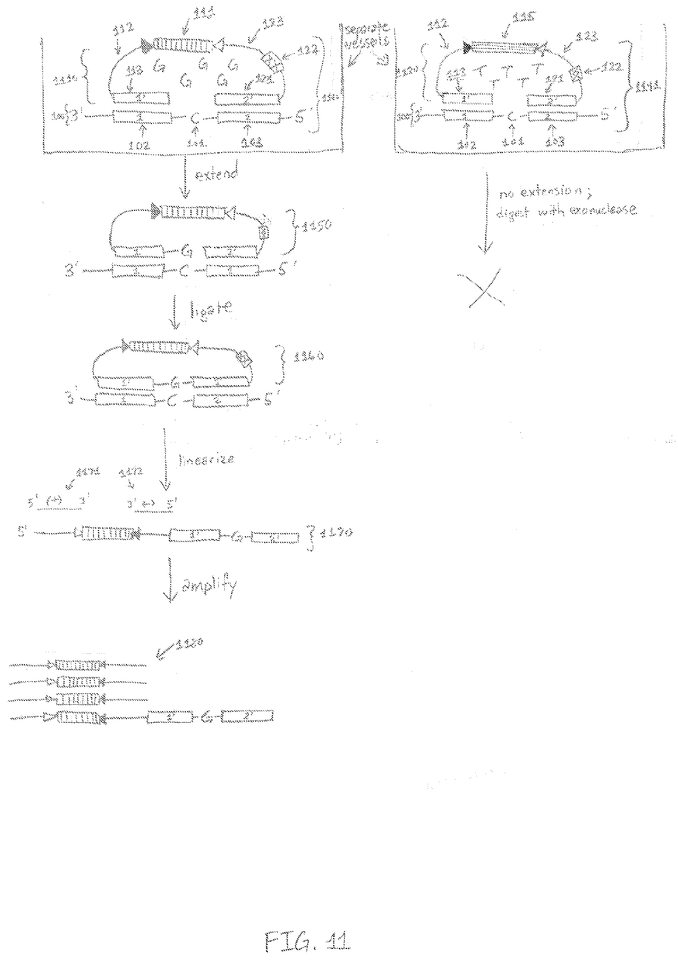

Fig. 11 shows a free base assay in which an open circle probe 1110 is hybridized to the primary target sequence 100. Because of the complementary base pairing between a free nucleotide G and the detection nucleotide 101, a polymerase can extend the open circle probe 1110 with the free nucleotide G. The terminal nucleotide of the extended open circle probe 1150 can be ligated to the other end of the probe, thus forming circle probe 1160. Circle probe 1160 is then cleaved at the cleavage site to result in a secondary target sequence 1170. A portion of the secondary target sequence 1170 is amplified using a 5' primer 1171 and a 3' primer 1172 to generate tertiary target sequences 1180, which can be labeled and detected as described herein.

In a separate vessel, the hybridization complex 1141 comprises the open circle probe 1120 that comprises address sequence 115 that is different from address sequence 111. In some aspects, the same open circle probe having the same address sequence is used across vessels. The free nucleotide T does not form a complementary base pair with the detection nucleotide 101. Extension and ligation therefore do not occur. The open circle probe 1120 is removed from a sample, for example, by degradation via an exonuclease. The address sequence 115 is thus not amplified.

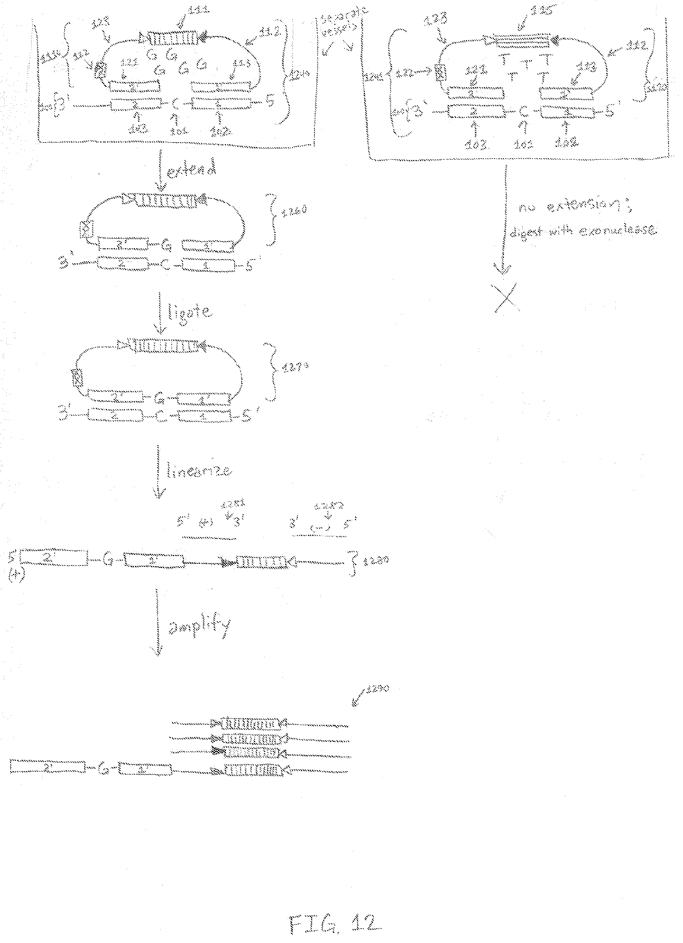

Fig. 12 shows the same general assay as that shown in Fig. 11 except that the first target domain 102 of the target sequence 100 is 5' to second target domain 103. Complementary base pairing between free nucleotide G and detection nucleotide 101 allows formation of an extended open circle probe 1260. Ligation of the ends of the extended open circle probe 1260 results in ligated probe 1270. The ligated probe 1270 is then cleaved at the cleavage site to result in a secondary target sequence 1280. A portion of the secondary target sequence 1280 is amplified using a 5' primer 1281 and a 3' primer 1282 to generate tertiary target sequences 1290, which can be labeled and detected as described herein. Tertiary target sequences 1290 will form the majority of the amplified product.

In a separate vessel, hybridization complex 1241 comprises a first ligation probe 130 that comprises address sequence 115 that is different from address sequence 111. In some embodiments, the same first ligation probe having the same address sequence is used across vessels. The free nucleotide T does not form a complementary base pair with the detection nucleotide 101. Extension and ligation therefore do not occur. The open circle probe 1120 is removed from a sample, for example, by degradation via an exonuclease. The address sequence 115 is thus not amplified.

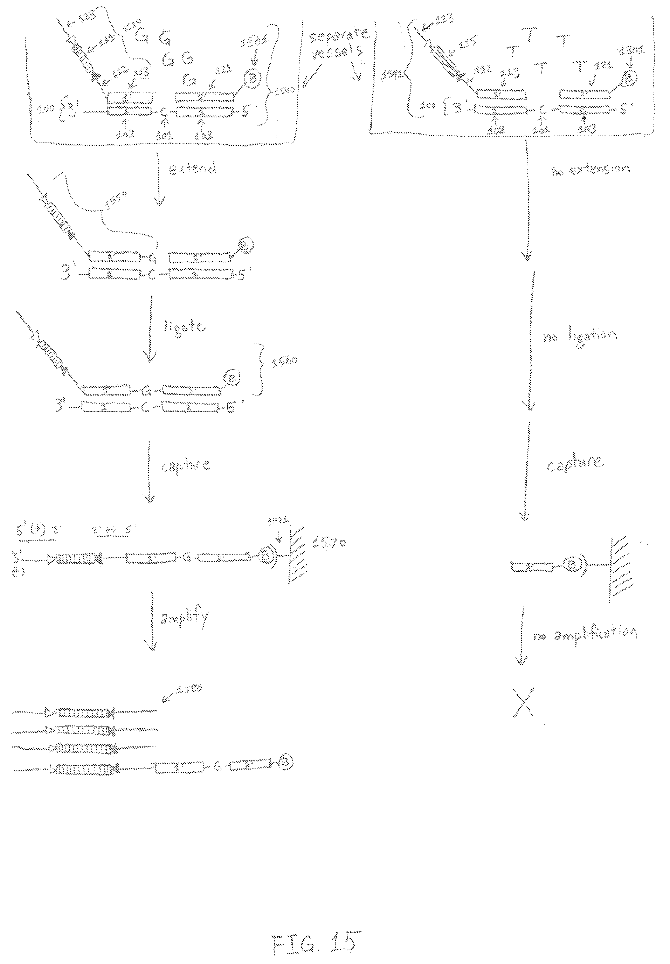

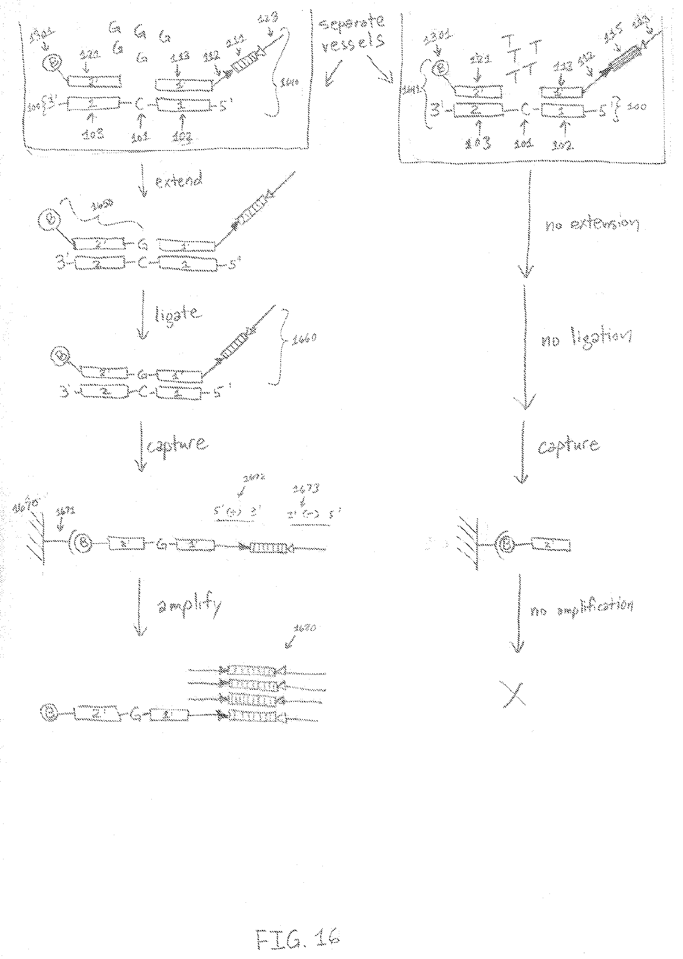

Figs. 15 and 16 show a type of free base assay in which one probe comprises an address cassette and another probe comprises a binding label. This embodiment is described further below.

### Capture Assay

Figs. 13-16 show examples of a capture assay, which can be done in a competitive or free base format and can comprise a simple ligation or both a ligation and an extension. In this embodiment, a first ligation probe comprises an address cassette and a second ligation probe comprises a binding label. A component of the assay can be captured by a solid support and then can be further analyzed to determine whether ligation has occurred.

In Fig. 13, a first ligation probe 1310 comprises an address cassette (comprising a second priming domain 123, a first address sequence 111 and a first priming domain 112), a first hybridization domain 113 and an interrogation nucleotide 114. A second ligation probe 1320 comprises a second hybridization domain 121 and a binding label (for example, biotin) 1301. The first hybridization domain 113 is complementary and thus hybridizes to the first target domain 102. The second hybridization domain 121 is complementary and thus hybridizes to the second target domain 103. A hybridization complex 1340 is thus formed. Because of the complementary base pairing between the interrogation nucleotide 114 and the detection nucleotide 101, ligation can occur between the interrogation nucleotide 114 to the proximate end of the second ligation probe. The ligated probe 1350 is then captured by a capture ligand 1361 attached to a solid support 1360, which can then be washed. A portion of the ligated probe, such as the address cassette, is amplified using a 5' primer 1362 and a 3' primer 1363 to generate derivative (secondary) target sequences 1370, which can be labeled and detected as described herein.

In hybridization complex 1341, a first ligation probe 1330 comprises an address sequence 115 and an interrogation nucleotide 116 that does not form a complementary base pair with detection nucleotide 101. No ligation therefore occurs. The second ligation probe is then captured. The second ligation probe would also not be amplified using the 5' primer and 3' primer since it contains no priming site for those primers, and hence no amplified product would be detected.

Fig. 14 shows the same assay as that shown in Fig. 13 except that an additional extension step is performed. Because of the complementary base pairing between the interrogation nucleotide 114 and the detection nucleotide 101, a polymerase can extend the first ligation probe 1410 with base pairs complementary to a target extension domain 301, forming an extended first ligation probe 1450. The terminal nucleotide of the extended first ligation probe 1450 is ligated to the proximate end of the second ligation probe to form a ligated probe 1460. The ligated probe 1460 is then captured by a capture ligand 1471 attached to a solid support 1470, which can then be washed. A portion of the ligated probe, such as the address cassette, is amplified using a 5' primer 1472 and a 3' primer 1473 to generate derivative (secondary) target sequences 1480, which can be labeled and detected as described herein.

In hybridization complex 1441, a first ligation probe 1430 comprises an address sequence 115 and an interrogation nucleotide 116 that does not form a complementary base pair with detection nucleotide 101. No extension and no ligation therefore occur. The second ligation probe is then captured. The second ligation probe would also not be amplified using the 5' primer and 3' primer since it contains no priming site for those primers, and hence no amplified product would be detected.

Similar to the assays shown in Figs. 2, 4, 6, 8, 10 and 12, the assays shown in Figs. 13 and 14 can be performed on a primary target sequence in which a first target domain is 5' to the second target domain.

Assays using a first ligation probe comprising an address cassette and a second ligation probe comprising a binding label can be performed in free base format in separate vessels.

In Fig. 15, a first ligation probe 1510 comprises an address cassette (comprising a second priming domain 123, a first address sequence 111 and a first priming domain 112) and a first hybridization domain 113. A second ligation probe 1320 comprises a second hybridization domain 121 and a binding label (for example, biotin) 1301. The first hybridization domain 113 is complementary and thus hybridizes to the first target domain 102. The second hybridization domain 121 is complementary and thus hybridizes to the second target domain 103. A hybridization complex 1540 is thus formed. Because of the complementary base pairing between a free nucleotide G and the detection nucleotide 101, a polymerase can extend the first ligation probe 110 with the free nucleotide G. The terminal nucleotide of the extended first ligation probe 1550 is then ligated to the proximate end of the second ligation probe to form a ligated probe 1560. The ligated probe 1560 is then captured by a capture ligand 1561 attached to a solid support 1560, which can then be washed. A portion of the ligated probe, such as the address cassette, is amplified using a 5' primer 1562 and a 3' primer 1563 to generate derivative (secondary) target sequences 1570, which can be labeled and detected as described herein.

In a separate vessel, hybridization complex 1541 comprises a first ligation probe 130 that comprises address sequence 115 that is different from address sequence 111. In some embodiments, the same first ligation probe having the same address sequence is used across vessels. The free nucleotide T does not form a complementary base pair with the detection nucleotide 101. Extension and ligation therefore do not occur. The second ligation probe is then captured. The second ligation probe would also not be amplified using the 5' primer and 3' primer since it contains no priming site for those primers, and hence no amplified product would be detected.

Fig. 16 shows the same general assay shown in Fig. 15 except that the first target domain 102 of the target sequence 100 is 5' to the second target domain 103. Complementary base pairing between free nucleotide G allows formation of an extended second ligation probe 1650. Ligation between extended second ligation probe 1650 and the first ligation probe results in ligated probe 1660. The ligated probe 1660 is then captured by a capture ligand 1671 attached to a solid support 1670, which can then be washed. A portion of the ligated probe, such as the address cassette, is amplified using a 5' primer 1672 and a 3' primer 1673 to generate derivative (secondary) target sequences 1680, which can be labeled and detected as described herein. Tertiary target sequences 1680 will form the majority of the amplified product.

In a separate vessel, hybridization complex 1641 comprises a first ligation probe 130 that comprises address sequence 115 that is different from address sequence 111. In some embodiments, the same first ligation probe having the same address sequence is used across vessels. The free nucleotide T does not form a complementary base pair with the detection nucleotide 101. Extension and ligation therefore do not occur. The second ligation probe is then captured. The second ligation probe would also not be amplified using the 5' primer and 3' primer since it contains no priming site for those primers, and hence no amplified product would be detected.

In any of the figures, it can be appreciated that while the second ligation probe is shown to comprise a cleavage site, the first ligation probe can comprise a cleavage site instead of or in addition to the second ligation probe. For example, any of the first ligation probes shown in the figures or described herein can comprise a cleavage site between the first priming domain and the first hybridization domain.

Furthermore, a binding label such as biotin can be incorporated at various parts of any of the components shown in the figures. For example, in Figs. 1-12, a biotin or other binding label can be incorporated between a second hybridization domain and the cleavage site. Use of a binding label in this way can facilitate or improve the detection of the various species shown. For example, incorporating a binding label in the probes shown in the figures can allow binding of the probes to a solid support, which can be washed so that only the species of interest is retained.

### Applications

The compositions and methods described herein can be employed in a wide variety of applications. In some embodiments, the screening or genotyping methods of the present invention may be used to detect at least one allele of at least one genetic locus. In one particularly useful embodiment, the genotyping methods herein are used to detect alleles found in a fetal genome. Currently known prenatal diagnostic methods typically involve invasive techniques such as amniocentesis, the removal chorionic villi and the removal of fetal blood or tissue biopsies. Non-invasive methods based on enriching maternal blood samples for fetal cells and analyzing the population of cells in the sample to identify fetal cells have been described. See International Publication Nos. WO 2008/048931 and WO 2010/075459. Lo, U.S. Patent No. 6,258,540 discloses a method of performing a prenatal diagnosis on a maternal blood sample, which method comprises obtaining a non-cellular fraction of the blood sample, amplifying a paternally inherited nucleic acid from the non-cellular fraction and performing nucleic acid analysis on the amplified nucleic acid to detect paternally inherited fetal nucleic acid. The genotyping methods described herein, however, need not and in particular embodiments do not amplify a primary target sequence, such as the fetal genomic DNA inherited from a father or mother.

The present genotyping methods can be combined with methods known in the art for determining fetal gene variants. For example, Oliphant, International Publication No. WO 2010/075459, describes, among others, a method in which a maternal sample is genotyped, a mixture of maternal and fetal cells is obtained, and the sample is concentrated for fetal cells and divided into subsamples. A panel of at least one target locus at which the maternal sample is homozygous is selected for screening or genotyping of the subsamples. Each of the subsamples is individually screened or genotyped at at least one of these loci, with detection of a heterozygous genotype indicating the presence of a non-maternal allele in the subsample. Alternatively, a panel of at least one target locus at which the maternal sample is heterozygous is selected for screening or genotyping of the subsamples. Each of the subsamples is individually screened or genotyped at at least one of these loci, with detection of a homozygous genotype indicating the presence of a non-maternal allele in the subsample. The methods for genotyping described herein are particularly suited for use in that and other methods described in Oliphant, and provide a way of detecting fetal DNA without amplification and/or detection of the fetal DNA, particularly by amplification and/or detection of artificial, non-genomic sequences.

As will be appreciated in the art, a wide number of different SNPs or other types of polymorphisms can be tested to determine at which alleles the maternal sample is homozygotic. For example, Oliphant, International Publication No. WO 2010/075459, discloses methods for designing a SNP panel useful for identifying fetal genetic material in a sample. Any number of common SNP/alleles can be used. In general, a set of SNPs are initially evaluated to determine at which alleles the maternal sample is homozygotic. This set can range from 1 to 200, from 10 to 150 being useful, and from 50-100 being particularly useful. In some embodiments, the set of SNPs that are tested for maternal homozygosity are selected from any combination of SNPs disclosed in dbSNP accession records rs1424506, rs207509, rs1004044, rs1673003, rs4789798, rs1887889, rs207509, rs4593206, rs189664, rs1006779, rs7027512, rs165924, rs6803756, rs207509, rs4684327, rs239683, rs1014803, rs598416, rs2830169, rs448887, rs220162, rs4982485, rs2825069, rs1014803, rs576762, rs10129348, rs13151776, rs2205533, rs2650957, rs2830234, rs11087884, rs1419747, rs2269355, rs12920309, rs2205533, rs35748020, rs2838818, rs1153280, rs731750, rs2242360, rs6581667, rs2268262, rs2391110, rs2010355, rs1153280, rs3788696, rs658857, rs1263416, rs2824547, rs2306612, rs1153280, rs11700636, rs6902513, rs11074601, rs7462034, rs2829694, rs354359, rs1537852, rs11700636, rs1522666, rs10758556, rs1241633, rs2832668, ra1864462, rs2268262, rs12482516, rs224065, rs3784301, rs2130643, rs2833756, rs10805396, rs879261, rs12482516, rs1039753, rs10217351, rs7589684, rs2839619, rs6955206, rs6687726, rs12626413, rs6533225, rs1111832, rs2249102, rs3787659, rs13332281, rs2205533, rs12626873, rs1841946, rs12423234, rs13406272, rs3787728, rs1830138, rs2725303, rs13049530, rs12662883, rs1572641, rs3213856, rs3787831, rs7155331, rs3818, rs13049530, rs2270433, rs1554472, rs2634421, rs3787894, rs1889819, rs9888005, rs1537852, rs224147, rs10815923, rs7311115, rs3788097, rs7585579, rs723469, rs1543754, rs1456017, rs1335788, rs1495772, rs3853054, rs6844640, rs1481065, rs1543754, rs4437273, rs6475240, rs721457, rs461853, rs13095333, rs284877, rs1921361, rs11071641, rs2182957, rs11161520, rs587085, rs220162, rs2241145, rs2000490, rs7573728, rs1923874, rs4271524, rs7279064, rs11087884, rs4144457, rs2000490, rs4753881, rs2812148, rs1385161, rs917580, rs3787728, rs9610714, rs2032652, rs11624331, rs2834708, rs324554, rs3787831, rs7875113, rs2064391, rs2182241, rs747039, rs11935170, rs3788097, rs2834924, rs2064391, rs7295630, rs8130025 and rs4368579. The dbSNP is an online public archive of records detailing genetic variations that can be accessed by way of the reference numbers provided above.

In other embodiments, by choosing SNPs for which one or the other parent is homozygotic (usually but not always the mother), the assays of the present invention can be done in a format that utilizes fewer labels than normally required. In these embodiments, rather than label all probes, only the non-homozygotic alleles are labeled. For example, assuming a first SNP that is A/G at the detection position, and one parent is homozygotic A, only the G probes are labeled; similarly, a second SNP that is T/C, with one parent being homozygotic T, only the C probes are labeled. In this way, more information is obtained using fewer labels.

### Synthetic Sequencing Libraries

The compositions and methods of the present invention can be used to generate synthetic nucleic acid libraries. Such synthetic nucleic acid libraries may be used for sequencing or in oligonucleotide arrays. In certain embodiments, the methods and compositions of the present invention are used to generate synthetic nucleic acid libraries that encode a target locus or allele. In other embodiments, multiple ligation probes, each comprising a different interrogation nucleotide, can be used simultaneously. In exemplary embodiments, multiple sets of ligation probes are used, each probe comprising an interrogation nucleotide different from each other interrogation nucleotide of each other ligation probe in the set.

In another aspect of the present embodiment, the ligation probe comprises a locus or allele index sequence and, optionally, a sample index sequence. The locus or allele index sequence can encode some or all of a locus or allele targeted by the ligation probe. The sample index sequence provides an option to multiplex. A sample index sequence can serve to identify the source of a target sequence. For example, one or more ligation probes comprising a first sample index sequence can be allowed to bind to a target sequence contained in a sample obtained from a first source, such a first subject. Separately, one or more ligation probes comprising a second sample index sequence different from the first sample index sequence can be allowed to bind to a target sequence contained in a sample obtained from a second source, such as a second subject. The ligation probes from both samples can then be pooled and analyzed in a single run. For example, the pooled sample can be contacted with a single DNA array chip or can be deposited into a single lane for electrophoresis. The provision of a locus or allele index sequence in a ligation probe of the present invention facilitates identification and counting of target loci or alleles. The methods described herein can be used to produce a library in which each component is 1:1 with a target sequence in a sample. The library components can be counted, for example by sequencing. In this way, a sequencing platform acts as a digital counter of a sequence in a sample.

In some embodiments, the length of the sequence library oligonucleotides generated by the methods and compositions of the present invention is a length preferred by a specific sequencing technology (e.g. Illumina HiSeq). In certain aspects, a synthetic library oligonucleotide is about 25-50 base pairs, about 50-100 base pairs, about 100-500 base pairs, or about 500-1000 base pairs.

The components of a synthetic sequencing library can incorporate any number of synthetic sequences that could be useful for decreasing errors or artifacts that might arise in various applications. Any adapter, index sequence or address sequence disclosed herein can be designed to optimize the sequences for their intended use. For example, it known in the art that nucleic acid amplification can be biased due to the presence of GC basepairs. Accordingly, synthetic sequences can be designed to provide uniform GC distribution in order to minimize bias.

In other embodiments, error correction codes are included in the index oligonucleotides used in the methods of the present invention. Such error correction codes are useful for compensation for sequencing errors (See, e.g., U.S. Patent Application Publication No. 2007/0042372). Examples of error correction codes include, e.g., analogs of cyclic redundancy checks. In some embodiments, a function can be used to map a known sequence to a error correction code, such as a check value. The function can then be applied to a sequencing result to produce a test value that can be compared to the error correction code. A discrepancy between the test value and the error correction code can alert the practitioner to a potential error in the sequencing result.

### EXAMPLES

The invention is further understood by reference to the following examples, which are intended to be purely exemplary of the invention. The present invention is not limited in scope by the exemplified embodiments, which are intended as illustrations of single aspects of the invention only.

Example 1: Design of Oligonucleotides for Genotyping a Single Nucleotide Polymorphism (SNP) in a Primary Target Sequence

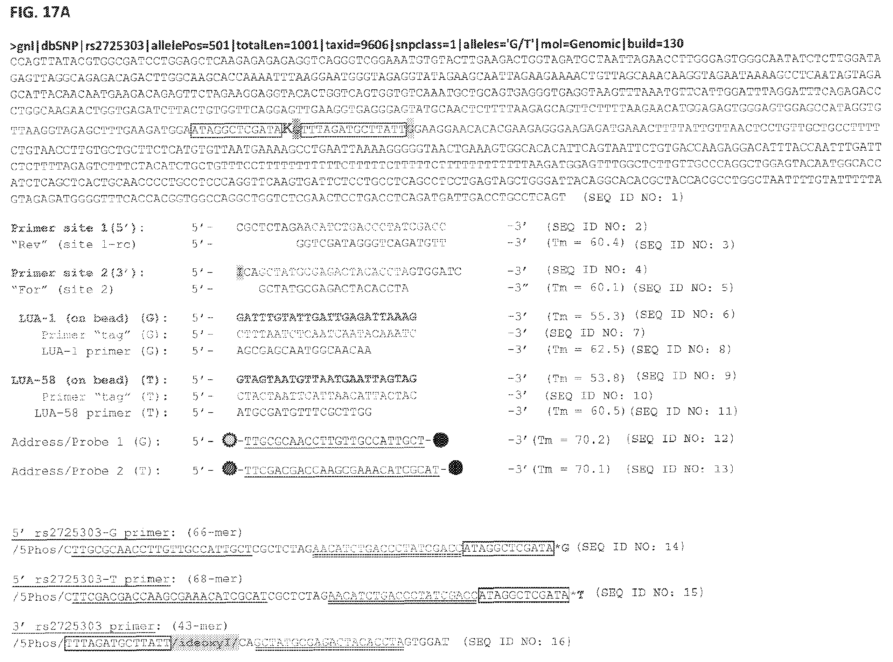

The methods described herein can be performed to genotype any gene. Particularly useful genes are those that are characterized by multiple alleles, and examples of these genes can be found in the NCBI Single Nucleotide Polymorphism database (dbSNP). Figs. 17A-17C shows oligonucleotide sequences that can be used in the methods disclosed herein for genotyping *RBPJ,* a gene relating to a recombination signal binding protein for immunoglobulin kappa J region. Information about this gene is provided by dbSNP record number rs2725303, which indicates that a G or T allele can be determined. In Fig. 17A, the relevant primary target sequence that can be genotyped is shown as SEQ ID NO: 1, in which the letter K marks the detection position. SEQ ID NO: 2 contains a first priming domain, and SEQ ID NO: 3 shows a primer that is useful for hybridizing thereto. SEQ ID NO: 4 contains a second priming domain, and SEQ ID NO: 5 is a primer that could bind to the complement of the second priming domain. The 5' end of SEQ ID NO: 4 is deoxyinosine, which is part of a cleavage site. SEQ ID NO: 6 is a sequence that can be attached to a solid support, such as a bead. SEQ ID NO: 7 is a primer tag that is the reverse complement of SEQ ID NO: 6. SEQ ID NO: 8 is a sequence that can be appended to the primer tag to result in SEQ ID NO: 22, which is useful, for example, in assays provided by Luminex Corp. In this case, SEQ ID NOS: 6-8 can be used to probe for the G allele. SEQ ID NO: 9 is another sequence that can be attached to a solid support, such as a bead. SEQ ID NO: 10 is a primer tag that is the reverse complement of SEQ ID NO: 9. SEQ ID NO: 11 is a sequence that can be appended to the primer tag to result in SEQ ID NO: 23, which is useful, for example, in assays provided by Luminex Corp. Here, SEQ ID NOS: 9-11 can be used to probe for the T allele. SEQ ID NOS: 12 and 13 are address sequences for probing the G and T alleles, respectively.

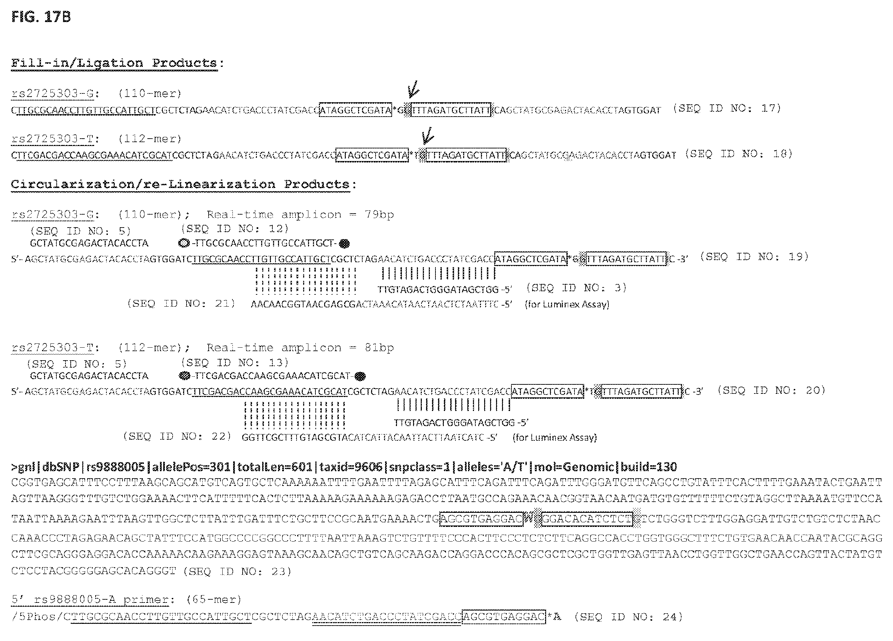

These components can be assembled to provide various ligation probes. SEQ ID NO: 14 is a first ligation probe corresponding to the G allele, and SEQ ID NO: 15 is a first ligation probe corresponding to the T allele. In these first ligation probes, the underlined sequences are different address sequences, the double underlined sequences are first priming domains and the boxed sequences are first hybridization domains. The star represents a modification, such as a phosphorothioate linkage, that confers resistance to exonuclease activity. SEQ ID NO: 16 is a second ligation probe comprising a second hybridization domain (boxed), a cleavage site including deoxyinosine, and a second priming domain (double underlined). A first ligation probe can be extended by a nucleotide and ligated to the second ligation probe to provide ligated probes according to SEQ ID NOS: 17 and 18. Arrows in Fig. 17B point to the nucleotide that has been added in an extension step.

The ligated probes given by SEQ ID NOS: 17 and 18 are circularized and relinearized by cleaving at the cleavage site, resulting in secondary target sequences shown in SEQ ID NOS: 19 and 20, respectively. The address sequences can be amplified using primers having sequences according to SEQ ID NOS: 3 and 5. SEQ ID NOS: 21 and 22 are oligonucleotides can be used, for example, in certain bead based assays.

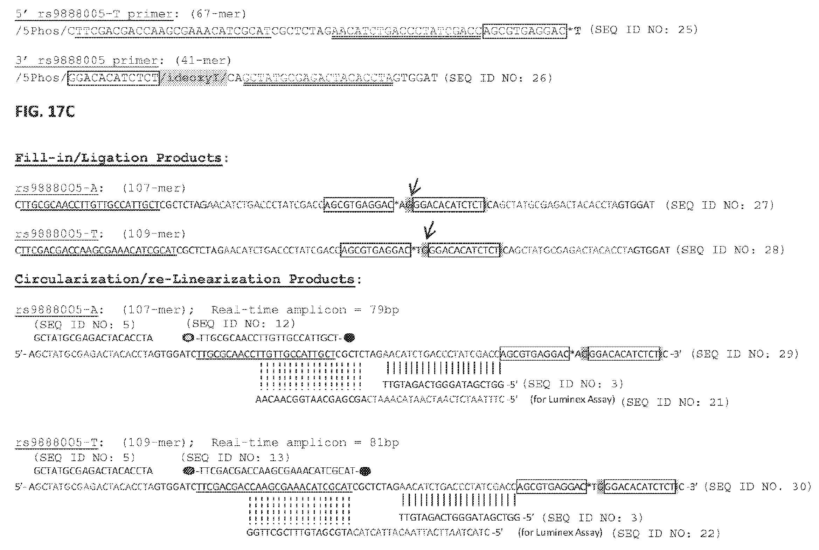

Other gene alleles that can be genotyped using probes assembled from these components are shown in dbSNP Record rs9888005. SEQ ID NO: 23 shows alleles relating to *FAM107B.* SEQ ID NOS: 24 and 25 are first ligation probes for distinguishing the A and T alleles, respectively, and SEQ ID NO: 26 is a second ligation probe. Extension of the first ligation probes and ligation to the second ligation probe result in ligated probes according to SEQ ID NOS: 27 and 28. Arrows in Fig. 17C point to the nucleotide that has been added in an extension step. Circularization and relinearization of the ligated probes result in secondary target SEQ ID NOS: 29 and 30.

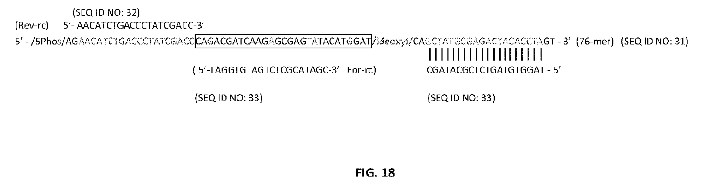

SEQ ID NO: 31 is a synthetic template for mimicking a successful fill-in/ligation product. SEQ ID NOS: 32 and 33 are primers for binding to SEQ ID NO: 31 or its complement (see Figure 18).

### Example 2: Circularization (i.e., Intramolecular Ligation) and Relinearization of Ligated Probe to Form a Secondary Target Sequence

Circularization (i.e., intramolecular ligation) and relinearization were performed on an exemplary linear oligonucleotide that was designed to mimic a ligated probe produced in the methods of the present invention. Twenty picomoles of the exemplary linear oligonucleotide (template 1, SEQ ID NO: 31 in Fig. 18) were circularized in a 20uL reaction volume using 100 units of CircLigase enzyme (Epicentre Biotechnologies; Madison, WI) in the manufacturer's supplied buffer at 1X concentration supplemented with 25uM ATP, and 0.4 units of Thermostable Inorganic Pyrophosphatase (New England Biolabs, Ipswich, MA) with and without 2.5mM MnCl₂. In addition, a "no circularization" control reaction was set up using 20 picomoles of the exemplary linear oligonucleotide as above but without the CircLigase enzyme, the Thermostable Inorganic Pyrophosphatase, and MnCl₂. These intramolecular ligation reactions were carried out in a Perkin Elmer 2400 Gene Amp PCR Thermal Cycler at 60°C for 1 hour, followed by a ligase inactivation step at 80°C for 10 minutes and a hold step at 4°C.

The resulting circularization reaction products were then individually subjected to relinearization by digesting half of each CircLigase reaction with Endonuclease V (EndoV). Specifically, 10pmol of each circularization reaction product was digested in a 20uL volume using 10 units of Endo V (New England Biolabs; Ipswich, MA) in the manufacturer's supplied buffer at 1X concentration for 2 hours at 37°C, followed by an enzyme inactivation step at 80°C for 10 minutes and a hold step at 4°C in a Perkin Elmer 2400 Gene Amp PCR Thermal Cycler.

Next, 7.5pmol of each circularization and corresponding relinearization reaction product was run on a 17% (19:1) native acrylamide gel for 65 minutes at 25 watts and stained with GelStar (Lonza; Walkersville, MD) to visualize.

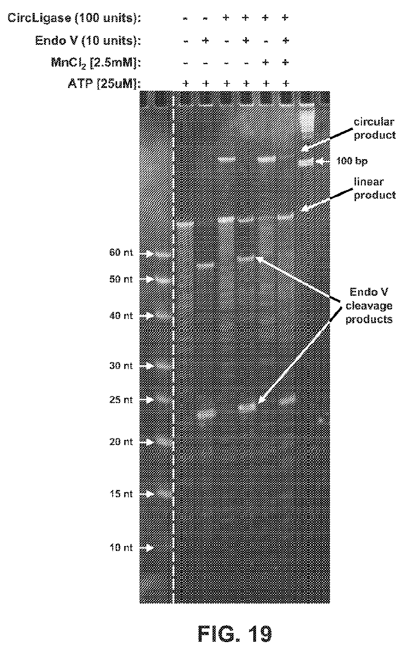

As shown in Figure 19, template 1, a linear oligonucleotide that was designed to mimic a ligated probe produced in the methods of the present invention, was successfully circularized using CircLigase (see lanes 4 and 6). Further, circularized template 1 was successfully relinearized using EndoV (see lanes 5 and 7 in Figure 19). These results showed that methods of the present invention are effective for circularizing and relinearizing an oligonucleotide template. These results suggested that the methods and compositions of the present invention would be effective for circularizing and relinearizing ligated probes produced in the methods of the present invention. These results further suggested that the methods and compositions of the present invention are useful for genotyping nucleic acids.

### Example 3: PCR Amplification and Detection of Ligated Probe and Secondary Target Sequence

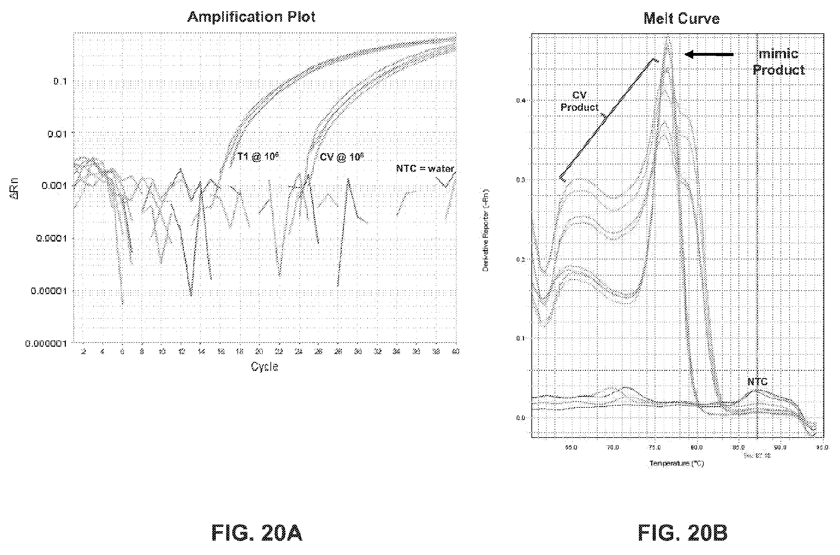

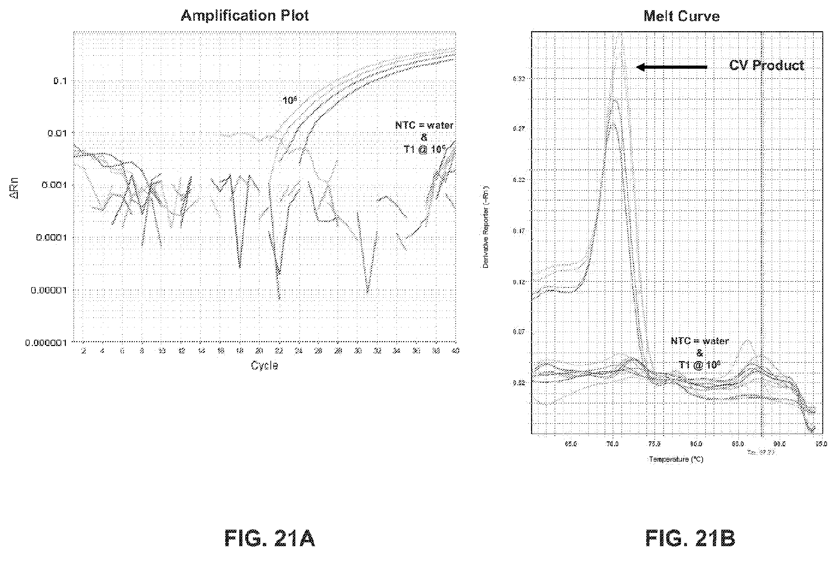

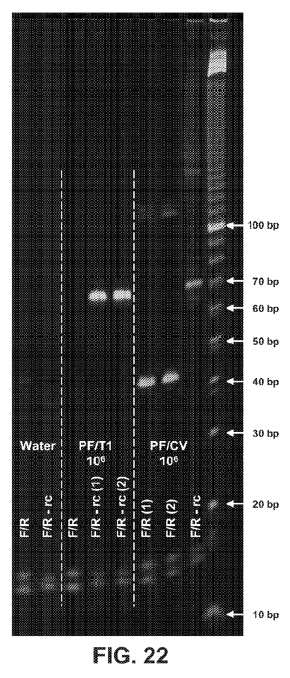

To assess sensitivity of an assay of the present invention, PCR amplification was performed on both the exemplary linear oligonucleotide (template 1, SEQ ID NO: 31 in Figure 18) that was designed to mimic a ligated probe and the exemplary secondary target sequence produced in Example 2 in the presence of either 4ng of male or female genomic DNA (i.e, background genomic DNA). PCR-based sybr green assays were run on test samples made using dilutions of both the exemplary linear oligonucleotide and the exemplary secondary target where the exemplary sequences were initially spiked into a 1ng/uL male (PM) or a 1ng/uL female (PF) genomic DNA (Promega; Madison, WI) background at concentrations of 1.32×10¹⁰ or 1.32×10⁹ exemplary molecules per uL, respectively, and then serially diluted 10-fold down to 1.32×10² for each exemplary sequence using the appropriate PM or PF genomic DNA background at a 1ng/uL concentration. Using the ABI Sybr Green Master Mix, 4ng background genomic input with spiked in exemplary sequence material, and 200nM primer concentrations, the PCR amplifications were carried out in 25uL volumes on the ABI 7500 instrument at 95°C for 10 minutes, followed by 40 cycles at 95°C for 15 seconds and 65°C for 1 minute followed by a standard melt curve analysis profile. (See Figures 20A, 20B, 21A, and 21B.) After amplification, 20uL of the reaction material was run on an 8% (19:1) native acrylamide gel for 45 minutes at 25 watts and then GelStar stained to visualize. (See Figure 22.) Primers were designed to produce PCR products of 72 bp for detection of template 1 and 44 bp for successful detection of the secondary target sequence.

As shown in Figure 22, strong bands of 72 bp were detected in samples containing template 1 spiked into the genomic DNA background. (See lanes 4 and 5 in Figure 22.) Strong bands of 44 bp were detected in samples containing an exemplary secondary target sequence spiked into the genomic DNA background. (See lanes 6 and 7 in Figure 22.) These results showed that methods of the present invention were effective for amplifying and detecting an exemplary secondary target sequence of the present invention. These results suggested that the methods and compositions of the present invention would be useful for amplifying an address sequence to form a tertiary target sequence and detecting the tertiary target sequence. These results further suggested that the methods and compositions of the present invention are useful for genotyping nucleic acids.

The articles "a," "an" and "the" as used herein do not exclude a plural number of the referent, unless context clearly dictates otherwise. The conjunction "or" is not mutually exclusive, unless context clearly dictates otherwise. The term "include" is used to refer to non-exhaustive examples.

## Claims

1. A method of identifying a detection nucleotide in a primary target sequence, said primary target sequence comprising a first target domain, a second target domain and said detection nucleotide, said method comprising:
(a) providing a first ligation probe comprising: in order, (i) a first address sequence, (ii) a first priming domain, (iii) a first hybridization domain that is complementary to said first target domain and (iv) a first interrogation nucleotide;
(b) providing a second ligation probe comprising: in order, (i) a second hybridization domain that is complementary to said second target domain and (ii) a second priming domain, wherein one of said first and second ligation probes comprises a cleavage site;
(c) hybridizing said first and second ligation probes to said first and second target domains, respectively, to form a first hybridization complex;
(d) subjecting said first hybridization complex to conditions whereby if said interrogation nucleotide is perfectly complementary to said detection nucleotide, ligation occurs to form a first ligated probe;
(e) forming a first circularized probe from said first ligated probe;
(f) cleaving said first circularized probe at said cleavage site to form a first secondary target sequence comprising, in order, said second priming domain, said first address sequence and said first priming domain;
(g) amplifying said first address sequence using said priming domains on said first secondary target sequence to form a first tertiary target sequence; and
(h) detecting the presence of said first tertiary target sequence to identify said detection nucleotide, wherein said first and second ligation probes are different molecules.

2. The method of claim 1 further comprising:
(a) providing a third ligation probe comprising: in order, (i) a second address sequence, (ii) said first priming domain, (iii) said first hybridization domain that is complementary to said first target domain and (iv) a second interrogation nucleotide,
wherein said second address sequence is different from said first address sequence,
said second interrogation nucleotide is different from said first interrogation nucleotide and
wherein one of said second or third ligation probe comprises a cleavage site;
(b) hybridizing said second and third ligation probes to said first and second target domains, respectively, to form a second hybridization complex;
(c) subjecting said second hybridization complex to conditions whereby if said interrogation nucleotide is perfectly complementary to said detection nucleotide, ligation occurs to form a second ligated probe;
(d) forming a second circularized probe from said second ligated probe;
(f) cleaving said second circularized probe at said cleavage site to form a second secondary target sequence comprising, in order, said second priming domain, said second address sequence and said first priming domain;
(e) amplifying said second address sequence using said priming domains on said second secondary target sequence to form a second tertiary target sequence; and
(f) detecting the presence of said second tertiary target sequence to identify said detection nucleotide,
wherein said first, second and third ligation probes are different molecules.

3. The method of claim 2, wherein said first address sequence has a size different from that of said second address sequence and wherein optionally said first address sequence has a nucleotide sequence different from that of said second address sequence.

4. The method of any of claims 1- 3, wherein the 3' terminus of said first ligation probe and the 5' terminus of said second ligation probe are adjacent in said hybridization complex.

5. A method of identifying a detection nucleotide in a primary target sequence, said primary target sequence comprising a first target domain, a second target domain and said detection nucleotide, said method comprising:
(a) providing a first ligation probe comprising: in order, (i) an address sequence, (ii) a first priming domain and (iii) a first hybridization domain that is complementary to said first target domain;
(b) providing a second ligation probe comprising: in order, (i) a second hybridization domain that is complementary to said second target domain and (ii) a second priming domain, wherein one of said first and second ligation probes comprises a cleavage site;
(c) hybridizing, in a first vessel, said first and second ligation probes to said first and second target domains, respectively, to form a hybridization complex;
(d) contacting said hybridization complex with a first dNTP and a polymerase such that said first or second ligation probe is extended to comprise an interrogation nucleotide if said first dNTP is perfectly complementary to said detection nucleotide;
(e) subjecting said hybridization complex to conditions whereby ligation occurs to form a ligated probe;
(f) forming a circularized probe from said ligated probe;
(g) cleaving said circularized probe at said cleavage site to form a secondary target sequence comprising, in order, said second priming domain, said first address sequence and said first priming domain;
(h) amplifying said first address sequence using said priming domains on said secondary target sequence to form a tertiary target sequence; and
(i) detecting the presence of said tertiary target sequence to identify said detection nucleotide, wherein said first and second ligation probes are different molecules.

6. The method of any preceding claim, wherein said circularized probe is formed by circularizing said ligated probe.

7. The method of any preceding claim, wherein said second ligation probe further comprises a label, and wherein prior to said amplifying step, said secondary target sequence is captured by binding said label to a capture binding ligand attached to a solid support.

8. The method of claim 5 further comprising: repeating the method, wherein the second hybridizing step occurs in a second vessel, and said contacting step comprises contacting said hybridization complex with a second dNTP and a polymerase such that said first or second ligation probe is extended to comprise an interrogation nucleotide if said second dNTP is perfectly complementary to said detection nucleotide,
wherein said second dNTP is different from said first dNTP.

9. The method of any preceding claim, wherein said cleavage site comprises one or more moieties selected from the group consisting of inosine, a ribonucleotide, an abasic site, a photocleavable group, and a restriction enzyme cleavage sequence.

10. The method of any preceding claim, wherein said tertiary target sequence comprises a label.

11. The method of any preceding claim, wherein said detecting step comprises forming a signaling complex comprising said tertiary target sequence, a capture probe and a solid support and wherein said signaling complex optionally further comprises a signal probe.

12. The method of any preceding claim, wherein said primary target sequence is DNA of a fetus
wherein optionally said DNA from said fetus is obtained from maternal blood, maternal serum, maternal plasma or maternal urine and wherein optionally the number of primary target sequences is counted in said maternal blood, maternal serum or maternal plasma in order to detect trisomy in said fetus.

13. The method of any preceding claim, wherein said detecting step comprises sequencing said tertiary target sequence.

14. A method of identifying a fetal cell in a blood sample from a mother, said method comprising:
(a) determining the genotype of a plurality of genes in DNA from a mother to identify a plurality of query genes that are homozygotic in said DNA from said mother;
(b) determining the genotype of one or more of said plurality of query genes in the DNA of a cell in said blood sample from said mother to identify at least one heterozygotic gene, the genotyping step comprising performing the method of any preceding claim, thereby identifying said fetal cell.

## Patentansprüche

1. Ein Verfahren zur Identifizierung eines Detektions-Nukleotids in einer primären Zielsequenz, die besagte primäre Zielsequenz weist dabei eine erste Zieldomäne, eine zweite Zieldomäne und das besagte Detektions-Nukleotid auf, wobei das Verfahren wiederum Folgendes aufweist:
(a) die Bereitstellung einer ersten Ligationssonde, die Folgendes aufweist: in dieser Reihenfolge, (i) eine erste Adresssequenz, (ii) eine erste Priming-Domäne, (iii) eine erste Hybridisierungsdomäne, die komplementär zur besagten ersten Zieldomäne ist, und (iv) ein erstes Abfrage-Nukleotid;
(b) die Bereitstellung einer zweiten Ligationssonde die Folgendes aufweist: in dieser Reihenfolge, (i) eine zweite Hybridisierungsdomäne, die die besagte zweite Zieldomäne ergänzt, und (ii) eine zweite Priming-Domäne, wobei entweder die erste oder zweite Ligationssonde eine Spaltstelle aufweist;
(c) die Hybridisierung der besagten ersten oder zweiten Ligationssonde in die erste bzw. zweite Zieldomäne, um einen ersten Hybridisierungskomplex zu bilden;
(d) die Unterwerfung des ersten Hybridisierungskomplexes unter Bedingungen, in denen, wenn das Abfrage-Nukleotid vollkommen komplementär zum Detektions-Nukleotid ist, eine Ligation stattfindet, um eine erste ligierte Sonde zu bilden;
(e) die Bildung einer ersten zirkularisierten Sonde aus der besagten ersten ligierten Sonde;
(f) die Spaltung der ersten zirkularisierten Sonde an der besagten Spaltstelle, um eine erste sekundäre Zielsequenz zu bilden, die in dieser Reihenfolge, Folgendes aufweist, die zweite Priming-Domäne, die erste Adresssequenz und die erste Priming-Domäne;
(g) die Erweiterung der ersten Adresssequenz unter Verwendung der Priming-Domänen auf der ersten sekundären Zielsequenz, um eine erste tertiäre Zielsequenz zu bilden; und
(h) die Erfassung des Vorhandenseins der besagten ersten tertiären Zielsequenz zur Identifizierung des Detektions-Nukleotids,
wobei die erste und zweite Ligationssonde verschiedene Moleküle sind.

2. Das Verfahren gemäß Anspruch 1, das darüberhinaus Folgendes aufweist:
(a) die Bereitstellung einer dritten Ligationssonde, die Folgendes aufweist: in dieser Reihenfolge, (i) eine zweite Adresssequenz, (ii) die erste Priming-Domäne, (iii) die erste Hybridisierungsdomäne, die komplementär zur ersten Zieldomäne ist, und (iv) ein zweites Abfrage-Nukleotid,
wobei sich die zweite Adresssequenz von der ersten Adresssequenz unterscheidet, sich das zweite Abfrage-Nukleotid vom ersten Abfrage-Nukleotid unterscheidet und
wobei entweder die zweite oder die dritte Ligationssonde eine Spaltstelle aufweist;
(b) die Hybridisierung der zweiten und dritten Ligationssonde zur ersten bzw. zweiten Zieldomäne, um einen zweiten Hybridisierungskomplex zu bilden;
(c) die Unterwerfung des zweiten Hybridisierungskomplexes unter Bedingungen, in denen, wenn das Abfrage-Nukleotid vollkommen komplementär zum Detektions-Nukleotid ist, eine Ligation auftritt, um eine zweite ligierte Sonde zu bilden;
(d) die Bildung einer zweiten zirkularisierten Sonde aus der besagten zweiten ligierten Sonde;
(f) die Spaltung der zweiten zirkularisierten Sonde an der besagten Spaltstelle, um eine zweite sekundäre Zielsequenz zu bilden, die, in dieser Reihenfolge, Folgendes aufweist, die besagte zweite Priming-Domäne, die zweite Adresssequenz und die erste Priming-Domäne;
(e) die Erweiterung der zweiten Adresssequenz unter Verwendung der Priming-Domänen auf die zweite sekundäre Zielsequenz, um eine zweite tertiäre Zielsequenz zu bilden; und
(f) die Erfassung des Vorhandenseins der zweiten tertiären Zielsequenz, um das Detektions-Nukleotid zu identifizieren,
wobei die erste, zweite und dritte Ligationssonde unterschiedliche Moleküle sind.

3. Das Verfahren gemäß Anspruch 2, wobei die erste Adresssequenz eine Größe hat, die sich von der der zweiten Adresssequenz unterscheidet und wobei optional die erste Adresssequenz eine Nukleotidsequenz hat, die sich von der der zweiten Adresssequenz unterscheidet.

4. Das Verfahren gemäß eines der Ansprüche 1- 3, wobei der 3'-Terminus der ersten Ligationssonde und der 5'-Terminus der zweiten Ligationssonde im besagten Hybridisierungskomplex nebeneinanderliegen.

5. Ein Verfahren zur Identifizierung eines Detektions-Nukleotids in einer primären Zielsequenz, die besagte primäre Zielsequenz weist dabei eine erste Zieldomäne, eine zweite Zieldomäne und das besagte Detektions-Nukleotid auf, wobei das Verfahren wiederum Folgendes aufweist:
(a) die Bereitstellung einer ersten Ligationssonde, die Folgendes aufweist: in dieser Reihenfolge, (i) eine Adresssequenz, (ii) eine erste Priming-Domäne und (iii) eine erste Hybridisierungsdomäne, die komplementär zur ersten Zieldomäne ist;
(b) die Bereitstellung einer zweiten Ligationssonde, die Folgendes aufweist: in dieser Reihenfolge, (i) eine zweite Hybridisierungsdomäne, die komplementär zur zweiten Zieldomäne ist, und (ii) eine zweite Priming-Domäne, wobei entweder die erste oder die zweite Ligationssonde eine Spaltstelle aufweist;
(c) die Hybridisierung, in einem ersten Gefäß, der ersten und zweiten Ligationssonde zur ersten bzw. zweiten Zieldomäne, um einen Hybridisierungskomplex zu bilden;
(d) die Kontaktierung des Hybridisierungskomplexes mit einer ersten dNTP und einer Polymerase, so dass die erste oder zweite Ligationssonde erweitert wird, um ein Abfrage-Nukleotid aufzuweisen, wenn die erste dNTP vollkommen komplementär zum Detektions-Nukleotid ist;
(e) die Unterwerfung des Hybridisierungskomplexes unter Bedingungen, in denen Ligation auftritt, um eine ligierte Sonde zu bilden;
(f) die Bildung einer zirkularisierten Sonde aus der besagten ligierten Sonde;
(g) die Spaltung der zirkularisierten Sonde an der Spaltstelle, um eine sekundäre Zielsequenz zu bilden, die Folgendes aufweist, in dieser Reihenfolge, die besagte zweite Priming-Domäne, die erste Adresssequenz und die erste Priming-Domäne;
(h) die Verstärkung der ersten Adresssequenz unter Verwendung der Priming-Domänen auf der sekundären Zielsequenz, um eine tertiäre Zielsequenz zu bilden; und
(i) die Erfassung des Vorhandenseins der besagten tertiären Zielsequenz, um das Detektions-Nukleotid zu identifizieren,
wobei die erste und zweite Ligationssonde unterschiedliche Moleküle sind.

6. Das Verfahren gemäß eines der vorhergehenden Ansprüche, wobei die zirkularisierte Sonde durch Zirkularisierung der ligierten Sonde gebildet wird.

7. Das Verfahren gemäß eines der vorhergehenden Ansprüche, wobei die zweite Ligationssonde darüberhinaus einen Marker aufweist, und
wobei vor dem besagten Verstärkungsschritt, die sekundäre Zielsequenz durch die Anbindung des besagten Markers an einen Anker-Bindungsliganden, der auf einer festen Auflage angebracht ist, angereichert wird.

8. Das Verfahren gemäß Anspruch 5,
das darüberhinaus Folgendes aufweist: die Wiederholung des Verfahrens, wobei der zweite Hybridisierungsschritt in einem zweiten Gefäß abläuft, und
der besagte Kontaktierungsschritt die Kontaktierung des Hybridisierungskomplexes mit einer zweiten dNTP und einer Polymerase aufweist, so dass die erste und zweite Ligationssonde erweitert wird, um ein Abfrage-Nukleotid aufzuweisen, wenn die besagte zweite dNTP vollkommen komplementär zum Detektions-Nukleotid ist,
wobei sich die zweite dNTP von der ersten dNTP unterschiedet.

9. Das Verfahren gemäß eines der vorhergehenden Ansprüche, wobei die Spaltstelle einen oder mehrere Reste aufweist, ausgewählt aus der Gruppe bestehend aus Inosin, einem Ribonukleotid, einer AP-Stelle, einer photospaltbaren Gruppe und einer Restriktionsenzym-Spaltsequenz.

10. Das Verfahren gemäß eines der vorhergehenden Ansprüche, wobei die besagte tertiäre Zielsequenz einen Marker aufweist.

11. Das Verfahren gemäß eines der vorhergehenden Ansprüche, wobei der Detektionsschritt die Bildung eines Signalkomplexes mit der besagten tertiären Zielsequenz aufweist, eine Fängersonde und eine feste Auflage und wobei der Signalkomplex optional darüberhinaus eine Signalsonde aufweist.

12. Das Verfahren gemäß eines der vorhergehenden Ansprüche, wobei die primäre Zielsequenz DNA eines Fötus ist, wobei optional die DNA von diesem Fötus aus dem mütterlichen Blut, mütterlichen Serum, mütterlichen Plasma oder dem mütterlichen Urin entnommen wird, und wobei optional die Zahl der primären Zielsequenzen in diesem mütterlichen Blut, mütterlichen Serum oder mütterlichen Plasma gezählt wird, um Trisomie in besagtem Fötus zu erkennen.

13. Das Verfahren gemäß eines der vorhergehenden Ansprüche, wobei der besagte Detektionsschritt die Sequenzierung der tertiären Zielsequenz aufweist.

14. Ein Verfahren zur Identifizierung einer Fötuszelle in einer Blutprobe der Mutter, das Verfahren weist dabei Folgendes auf:
(a) Die Bestimmung des Erbguts einer Vielzahl von Genen in der DNA einer Mutter, um eine Vielzahl von Abfragegenen zu identifizieren, die in der DNA der Mutter homozygot sind;
(b) Die Bestimmung des Erbguts einer oder mehrerer der besagten Vielzahl von Abfragegenen in der DNA einer Zelle in der Blutprobe der Mutter, um mindestens ein heterozygotes Gen zu identifizieren, der Erbgutschritt weist dabei die Ausführung des Verfahrens gemäß eines der vorhergehenden Ansprüche auf, wodurch die besagte fötale Zelle identifiziert wird.

## Revendications

1. Procédé d'identification d'un nucléotide de détection dans une séquence cible primaire, ladite séquence cible primaire comprenant un premier domaine cible, un second domaine cible et ledit nucléotide de détection, ledit procédé comprenant :
(a) la prévision d'une première sonde de ligature comprenant, dans l'ordre, (i) une première séquence d'adresse, (ii) un premier domaine d'amorçage, (iii) un premier domaine d'hybridation qui est complémentaire dudit premier domaine cible et (iv) un premier nucléotide d'interrogation ;
(b) la prévision d'une deuxième sonde de ligature comprenant, dans l'ordre, (i) un second domaine d'hybridation qui est complémentaire dudit second domaine cible (ii) un second domaine d'amorçage, dans lequel l'une des dites première et deuxième sondes de ligature comprend un site de clivage ;
(c) l'hybridation desdites première et deuxième sondes de ligature vers lesdits premier et second domaines cibles respectivement afin de former un premier complexe d'hybridation ;
(d) la soumission dudit premier complexe hybridation à des conditions dans lesquelles, si ledit nucléotide d'interrogation est parfaitement complémentaire dudit nucléotide de détection, une ligature se produit afin de former une première sonde ligaturée ;
(e) la formation d'une première sonde circularisée à partir de ladite première sonde ligaturée ;
(f) le clivage de ladite première sonde circularisée au niveau dudit site de clivage pour former une première fréquence cible secondaire comprenant, dans l'ordre, ledit second domaine d'amorçage, ladite première séquence d'adresse et ledit premier domaine d'amorçage ;
(g) l'amplification de ladite première séquence d'adresse en utilisant lesdits domaines d'amorçage sur ladite première séquence cible secondaire pour former une troisième fréquence cible tertiaire ; et
(h) la détection de la présence de ladite première séquence cible tertiaire pour identifier ledit nucléotide de détection ; et
dans lequel lesdites première et deuxième sondes de ligature sont des molécules différentes.

2. Procédé selon la revendication 1, comprenant en outre :
(a) la prévision d'une troisième sonde ligature comprenant, dans l'ordre, (i) une seconde séquence d'adresse, (ii) ledit premier domaine d'amorçage, (iii) ledit premier domaine d'hybridation qui est complémentaire dudit premier domaine cible et (iv) un second nucléotide d'interrogation,
dans lequel ladite seconde séquence d'adresse est différente de ladite première séquence d'adresse, ledit second nucléotide interrogation est différent dudit premier nucléotide d'interrogation et dans lequel l'une des dites seconde ou troisième sondes de ligature comprend un site de clivage ;
(b) l'hybridation desdites seconde et troisième sonde de ligature vers lesdits premier et second domaines cibles respectivement pour former un second complexe d'hybridation ;
(c) la soumission dudit second complexe d'hybridation à des conditions dans lesquelles, si ledit nucléotide d'interrogation est parfaitement complémentaire dudit nucléotide de détection, une ligature se produit pour former une deuxième sonde ligaturée ;
(d) la formation d'une deuxième sonde circularisée à partir de ladite deuxième sonde ligaturée ;
(f) le clivage de ladite deuxième sonde circularisée au niveau dudit site de clivage pour former une seconde séquence cible secondaire comprenant, dans l'ordre, ledit second domaine d'amorçage, ladite seconde séquence d'adresse et ledit premier domaine d'amorçage ;
(e) l'amplification de ladite seconde séquence d'adresse en utilisant lesdits domaines d'amorçage sur ladite seconde séquence cible secondaire pour former une seconde séquence cible tertiaire ; et
(f) la détection de la présence de ladite seconde cible tertiaire pour identifier ledit nucléotide de détection,
dans lequel lesdites première, seconde et troisième sondes de ligature sont des molécules différentes.

3. Procédé selon la revendication 2, dans lequel ladite première séquence d'adresse a une taille différente de celle de ladite seconde séquence d'adresse et dans lequel, en option, ladite première séquence d'adresse a une séquence nucléotide différente de celle de ladite seconde séquence d'adresse.

4. Procédé selon l'une quelconque des revendications 1- 3, dans lequel le terminus 3' de ladite première sonde de ligature et le terminus 5' de ladite deuxième sonde de ligature sont adjacents dans ledit complexe d'hybridation.

5. Procédé d'identification d'un nucléotide de détection dans une séquence cible primaire, ladite séquence cible primaire comprenant un premier domaine cible, un second domaine cible et ledit nucléotide de détection, ledit procédé comprenant :
(a) la prévision d'une première sonde de ligature comprenant, dans l'ordre, (i) une séquence d'adresse, (ii) un premier domaine d'amorçage et (iii) un premier domaine d'hybridation qui est complémentaire dudit premier domaine cible ;
(b) la prévision d'une deuxième sonde de ligature comprenant, dans l'ordre, (i) un second domaine d'hybridation qui est complémentaire dudit second domaine cible et (ii) un second domaine d'amorçage, dans lequel l'une des dites première et deuxième sondes de ligature comprend un site de clivage ;
(c) l'hybridation, dans une première cuve, desdites première et deuxième sondes de ligature vers lesdits premier et second domaines cibles respectivement pour former un complexe d'hybridation ;
(d) la mise en contact dudit complexe d'hybridation avec un premier dNTP et une polymérase de manière à ce que ladite première ou deuxième sonde de ligature soit étendue afin de comprendre un nucléotide d'interrogation si ledit premier dNTP est parfaitement complémentaire dudit nucléotide de détection ;
(e) la soumission dudit complexe d'hybridation à des conditions dans lesquelles une ligature se produit pour former une sonde ligaturée ;
(f) la formation d'une sonde circularisée à partir de ladite sonde ligaturée ;
(f) le clivage de ladite sonde circularisée au niveau dudit site de clivage pour former une seconde séquence cible secondaire comprenant, dans l'ordre, ledit second domaine d'amorçage, ladite première séquence d'adresse et ledit premier domaine d'amorçage ;
(h) l'amplification de ladite première séquence d'adresse en utilisant lesdits domaines d'amorçage sur ladite séquence cible secondaire pour former une séquence cible tertiaire ; et
(i) la détection de la présence de ladite séquence cible tertiaire pour identifier ledit nucléotide de détection,
dans lequel lesdites première et deuxième sondes de ligature sont des molécules différentes.

6. Procédé selon l'une quelconque des revendications précédentes, dans lequel ladite sonde circularisée est formée en circularisant ladite sonde ligaturée.

7. Procédé selon l'une quelconque des revendications précédentes, dans lequel ladite sonde ligaturée comprend en outre une étiquette et
dans lequel, avant ladite étape d'amplification, ladite séquence cible secondaire est capturée en liant ladite étiquette à un ligand de liaison de capture attaché à un support solide.

8. Procédé selon la revendication 5,
comprenant en outre : la répétition du procédé, dans lequel procédé la seconde étape d'hybridation se produit dans une seconde cuve et
ladite étape de mise en contact comprend la mise en contact dudit complexe d'hybridation avec un second dNTP et une polymérase de manière à ce que ladite première ou deuxième sonde de ligature soit étendue afin de comprendre un nucléotide d'interrogation si ledit second dNTP est parfaitement complémentaire dudit nucléotide de détection,
dans lequel ledit second dNTP est différent dudit premier dNTP.

9. Procédé selon l'une quelconque des revendications précédentes, dans lequel ledit site de clivage comprend une ou plusieurs fractions composées de : l'inosine, un ribonucléotide, un site abasique, un groupe photoclivable et une séquence de clivage d'enzyme de restriction.

10. Procédé selon l'une quelconque des revendications précédentes, dans lequel ladite séquence cible tertiaire comprend une étiquette.

11. Procédé selon l'une quelconque des revendications précédentes, dans lequel ladite étape de détection comprend la formation d'un complexe de signalisation comprenant ladite séquence cible tertiaire, une sonde de capture et un support solide et dans lequel ledit complexe de signalisation comprend en outre en option une sonde de signal.

12. Procédé selon l'une quelconque des revendications précédentes, dans lequel ladite séquence cible primaire est l'ADN d'un foetus, dans lequel, en option, ledit ADN dudit foetus est obtenu à partir de sang maternel, de sérum maternel, de plasma maternel ou d'urine maternelle et dans lequel, en option, le nombre de séquences cibles primaires est compté dans ledit sang maternel, sérum maternel ou plasma maternel afin de détecter la trisomie chez ledit foetus.

13. Procédé selon l'une quelconque des revendications précédentes, dans lequel ladite étape de détection comprend le séquençage de ladite séquence cible tertiaire.

14. Procédé d'identification d'une cellule foetale dans un échantillon sanguin d'une mère, ledit procédé comprenant :
(a) la détermination du génotype d'une pluralité de gènes dans l'ADN d'une mère pour identifier une pluralité de gènes de recherche qui sont homozygotes dans ledit ADN de ladite mère ;
(b) la détermination du génotype d'un ou plusieurs de ladite pluralité de gènes de recherche dans l'ADN d'une cellule dans ledit échantillon sanguin de ladite mère pour identifier au moins un gène hétérozygote, l'étape de génotypage comprenant la réalisation du procédé selon l'une quelconque des revendications précédentes en identifiant ainsi ladite cellule foetale.
